# EUROPEAN PATENT APPLICATION

(11) **EP 1 477 544 A2**
(43) Date of publication of application: **17.11.2004**
(21) Application number: 04102113.0
(22) Date of filing: 13.05.2004
(51) Int. Cl.: C09K 11/06, C07D 413/10, C07D 263/02, C07D 209/82, C08F 26/12

(54) **Blue Light-emitting Compounds, Blue Light-emitting Polymers, Processes of Preparing the Blue Light-emitting Compounds and Luminescent Element Including the Blue Light-emitting Polymers**

(30) Priority: 16.05.2003 JP 2003139677; 28.10.2003 JP 2003368157
(71) Applicant: HIROSE ENGINEERING CO., LTD., Tokyo 146-0092 (JP)
(72) Inventor: Nakaya, Tadao, Ohta-ku Tokyo 146-0092 (JP); Tobita, Michiaki, Ohta-ku Tokyo 146-0092 (JP); Saikawa, Tomoyuki, Ohta-ku Tokyo 146-0092 (JP); Ishitobi, Tatsuro, Ohta-ku Tokyo 146-0092 (JP); Ushijima, Takashi, Ohta-ku Tokyo 146-0092 (JP); Takano, Shinji, Ohta-ku Tokyo 146-0092 (JP); Tajima, Akio, Ohta-ku Tokyo 146-0092 (JP)
(74) Representative: Leonhard, Frank Reimund, Dipl.-Ing.

(57) **Abstract**

In view of the fact that luminescent compounds and polymers capable of emitting blue light for a long time and excellent in durability have not been developed, the present invention provides blue light-emitting compounds having special structures, blue light-emitting polymers having special repeating units, processes of producing the compounds, and luminescent elements including the polymers. The most characteristic blue light-emitting compound of the invention has the structure represented by formula (1): 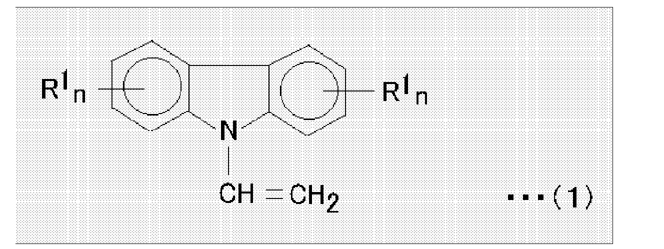 wherein R¹ is a hydrogen atom, an alkyl group having 1 to 10 carbon atoms, or a special substituent or the invention, two R¹s may be the same or different from each other, and n denotes an integer of 1 to 4.

## Description

### Technical Field

The present invention relates to blue light-emitting compounds, blue light-emitting polymers, processes of preparing the blue light-emitting compounds, and luminescent elements including the blue light-emitting polymers. More specifically, this invention rela to blue-light emitting compounds and blue light-emitting polymers capable of emitting blue light at a high luminance for a long time upon the application of electric energy, processes of producing the compounds and luminescent elements including the blue light-emitting polymers.

### Background Art

For organic electroluminescent elements, which are often abbreviated to "organic EL elements", have been proposed various luminescent compounds and polymers. However, luminescent compounds and polymers, capable of emitting blue light for a long time and excellent in durability, have not been developed.

### Summary of the Invention

The objective of this invention is to provide blue light-emitting compounds and blue light-emitting polymers capable of emitting blue light at a high luminance, and further excellent in durability. This invention also aims for providing processes for producing the blue light-emitting compounds and luminescent elements including the blue light-emitting polymers.

In order to achieve the objectives, the present invention provides a blue light-emitting compound having a chemical structure represented by formula (1): 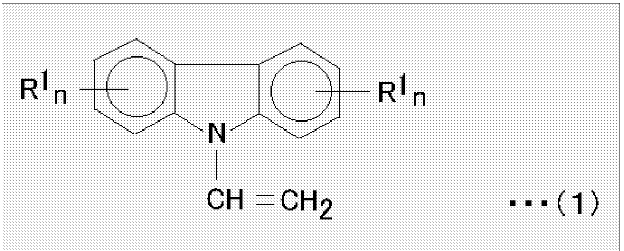 wherein R¹ is a hydrogen atom, an alkyl group having 1 to 10 carbon atoms, or a substituent represented by formula (2); two R¹s may be the same or different from each other; and n denotes an integer of 1 to 4.

The formula (2) is: 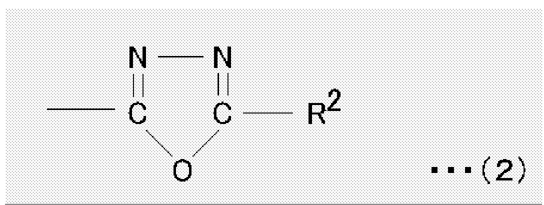 wherein R² is an aryl group represented by formulas (2-1) to (2-8).

The group represented by the formula (2-1) is: 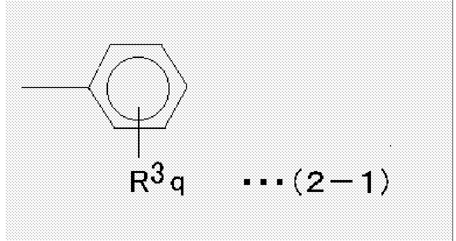 wherein R³ is a hydrogen atom or an alkyl group having 1 to 10 carbon atoms, and q denotes an integer of 1 to 5.

The group represented by the formula (2-2) is: 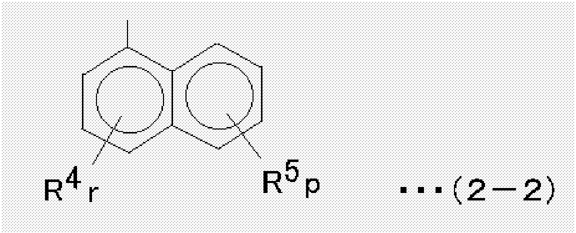 wherein each of R⁴ and R⁵ is a hydrogen atom, an alkyl group having 1 to 10 carbon atoms or an alkoxyl group having an alkyl group that has 1 to 5 carbon atoms; R⁴ and R⁵ may be the same or different from each other; p denotes an integer of 1 to 4; and r that of 1 to 3.

The group represented by the formula (2-3) is: 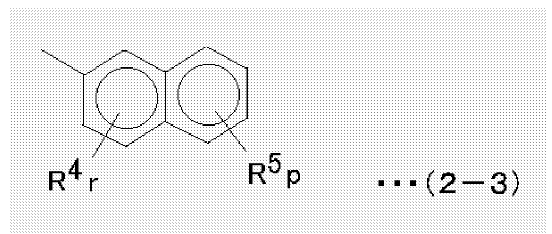 wherein R⁴, R⁵, p and r mean the same as those defined above; and R⁴ and R⁵ may be the same or different from each other.

The group represented by the formula (2-4) is: 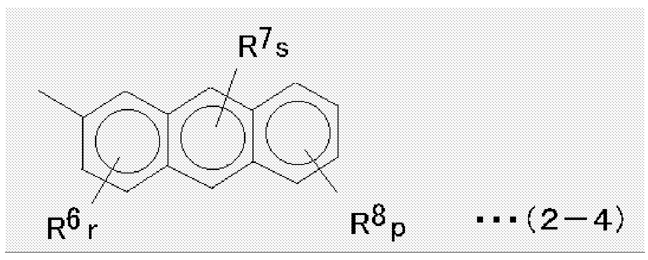 wherein each of R⁶, R⁷ and R⁸ is a hydrogen atom or an alkyl group having 1 to 5 carbon atoms; R⁶, R⁷ and R⁸ may be the same or different from each other; p and r denote the same as those defined above; and s denotes an integer of 1 or 2.

The group represented by the formula (2-5) is: 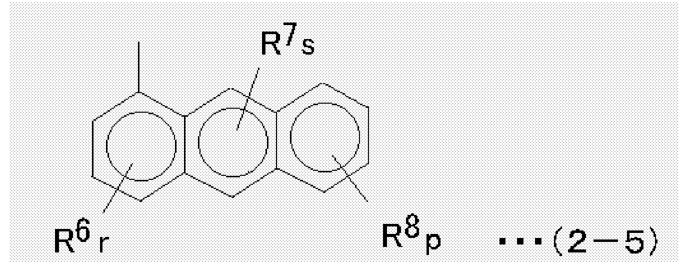 wherein R⁶, R⁷, R⁸, p, r and s mean the same as those defined above; and R⁶, R⁷ and R⁸ may be the same or different from each other.

The group represented by the formula (2-6) is: 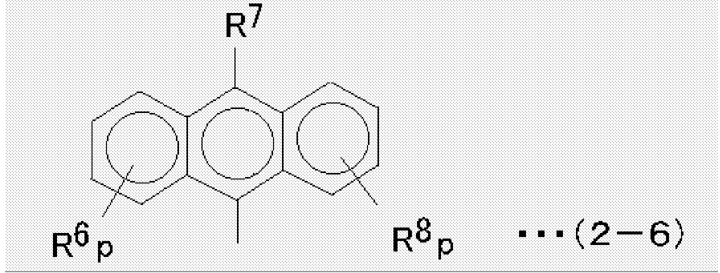 wherein R⁶, R⁷, R⁸, p, r and s mean the same as those defined above; and R⁶, R⁷ and R⁸ may be the same or different from each other.

The group represented by the formula (2-7) is: 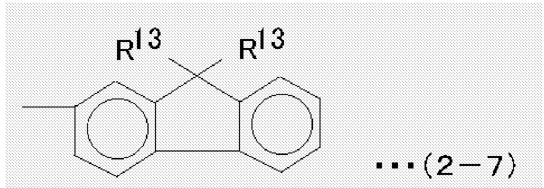 wherein R¹³ is a hydrogen atom or an alkyl group having 1 to 10 carbon atoms.

The group represented by the formula (2-8) is: 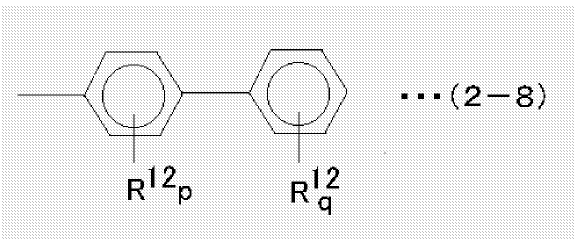 wherein R¹² is a hydrogen atom or an alkyl group having 1 to 10 carbon atoms; two R¹²s may be the same or different from each other; and p and q mean the same as those defined above.

The present invention also provides a blue light-emitting compound having a structure represented by formula (1'): 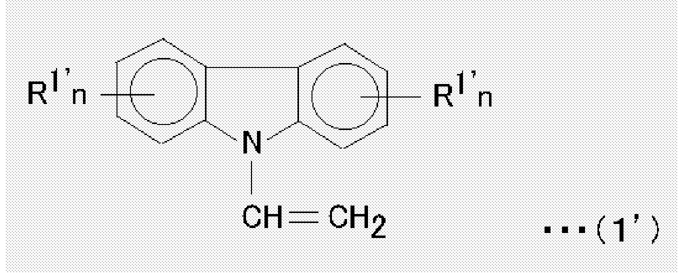 wherein R^{1'} is a hydrogen atom, an alkyl group having 1 to 10 carbon atoms, or a substituent represented by formula (2'); two R^{1'}s may be the same or different from each other; and n denotes an integer of 1 to 4.

The formula (2') is: 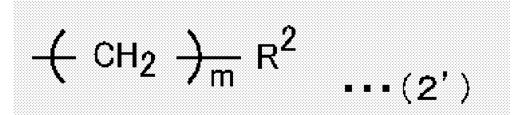 wherein R² is an aryl group represented by the formulas (2-1) to (2-8), or a substituent represented by formula (2'-1); and m denotes an integer of 0 to 3.

The substituent represented by the formula (2'-1) is: 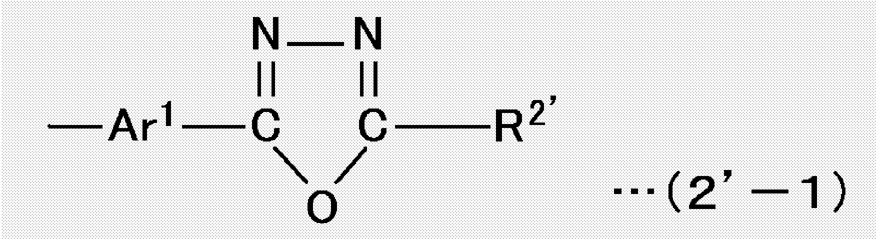 wherein Ar¹ is a phenylene group, a naphthylene group, or an anthrylene group; and R^{2'} means an aryl group represented by the formulas (2-1) to (2-8).

Furthermore, the present invention provides a process of producing the blue light-emitting compound represented by the formula (1) through the dehydration and dehydrohalogenation of the intermediate represented by formula (5), which intermediate is prepared through the reaction between a halogen compound represented by formula (4) and a hydrazide.

The formula (4) is: 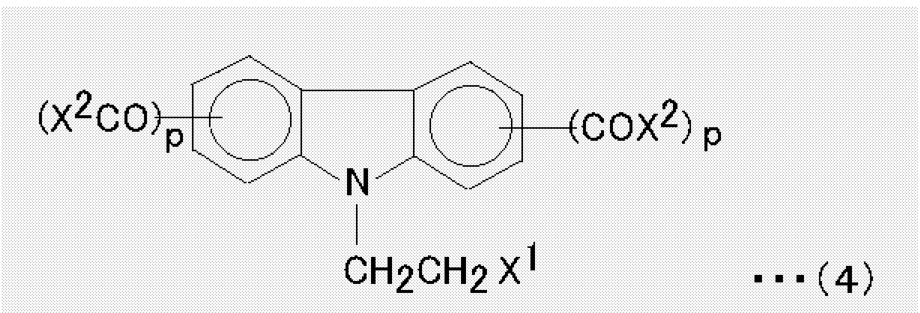 wherein X¹ and two X²s are a halogen atom, X¹ and two X²s may be the same or different from each other, and p is the same as that defined above.

The formula (5) is: 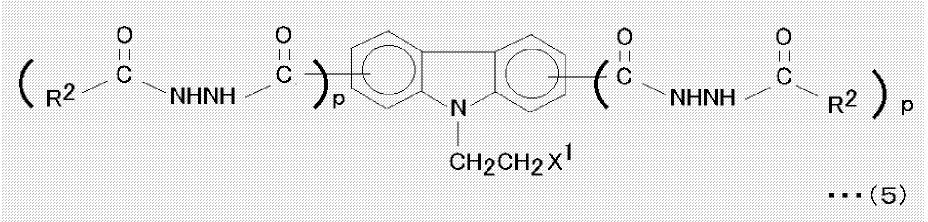 wherein R², X¹ and p are the same as those defined above, and two R²s may be the same or different from each other.

The present invention still further provides a process of producing the blue light-emitting compound that has the structure represented by the formula (1') through the dehydrohalogenation of an organic halogen compound represented by formula (5') which, in turn, is obtained through the Friedel-Crafts reaction of a halogen compound represented by formula (4').

The formula (4') is: 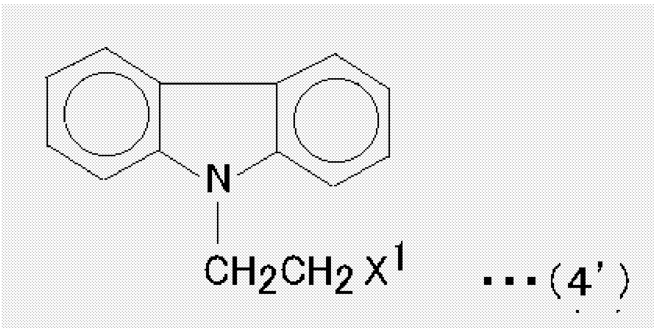 wherein X¹ is the same as that defined above.

The formula (5') is: 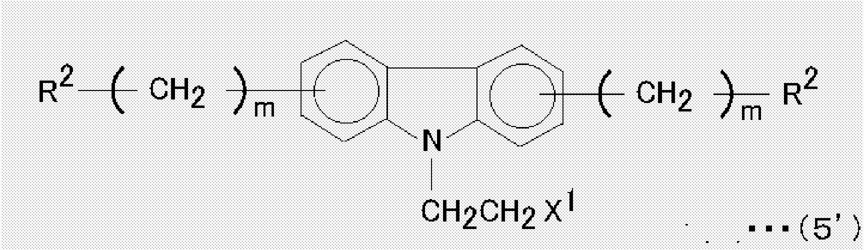 wherein R², X¹ and m are the same as those defined above.

The present invention also provides a blue light-emitting polymer having a repeating unit represented by formula (1''). 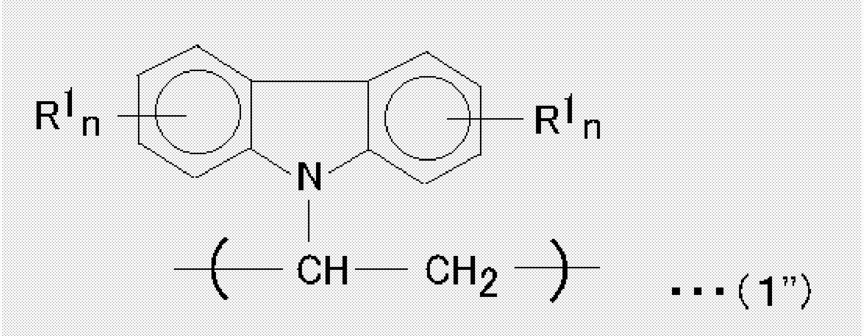 wherein R¹ and n mean the same as those defined in the formula (1).

The present invention provides another blue light-emitting polymer having a repeating unit represented by formula (1'''). 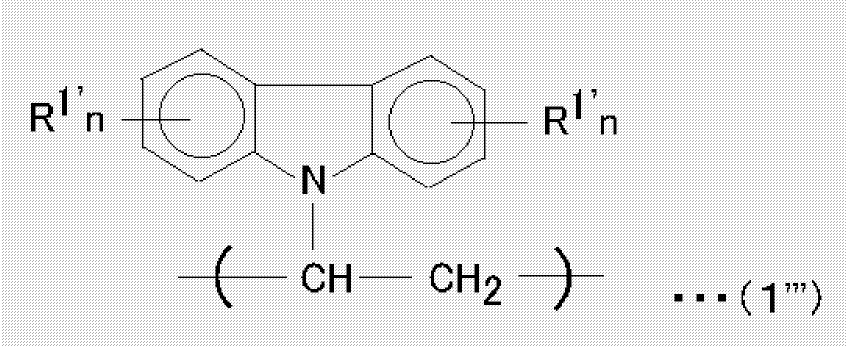 wherein R^{1'} and n mean the same as those defined in the formula (1').

Still further, the present invention provides a luminescent element which has a light-emitting layer including the blue light-emitting polymer having a repeating unit represented by the formula (1 ") or (1''') between a pair of electrodes.

The present invention can provide blue light-emitting compounds and blue light-emitting polymers capable of emitting light at a high luminance for a long time, and furthermore processes of producing the blue light-emitting compounds and luminescent elements including the blue light-emitting polymers.

### Brief Description of the Drawings

Figure 1 is an illustration showing an example of the luminescent element in accordance with the present invention.
Figure 2 is an illustration showing another example of the luminescent element in accordance with the present invention.
Figure 3 is an illustration showing a still another example of the luminescent element in accordance with the present invention.
Figure 4 is an illustration showing a further example of the luminescent element in accordance with the present invention.
Figure 5 is an NMR spectrum chart of N-2-(chloroethyl) carbazole obtained in Example 1.
Figure 6 is an NMR spectrum chart of the aldehydes compound obtained in Example 1.
Figure 7 is an NMR spectrum chart of the carboxylic acid compound obtained in Example 1.
Figure 8 is an NMR spectrum chart of the acid chloride compound obtained in Example 1.
Figure 9 is an NMR spectrum chart of the intermediate obtained in Example 1.
Figure 10 is an NMR spectrum chart of the oxadiazole compound obtained in Example 1.
Figure 11 is an IR spectrum chart of the oxadiazole compound obtained in Example 1.
Figure 12 is an NMR spectrum chart of the vinyl compound obtained in Example 1.
Figure 13 is an NMR spectrum chart of the blue light- emitting polymer produced through a reaction using a radical polymerization initiator in Example 1.
Figure 14 is an IR spectrum chart of the blue light- emitting polymer produced through a reaction using a radical polymerization initiator in Example 1.
Figure 15 is a fluorescence spectrum chart of the blue light-emitting polymer produced through a reaction using a radical polymerization initiator in Example 1.
Figure 16 is an NMR spectrum chart of the blue light- emitting polymer produced through a reaction in the presence of a transition-metal catalyst in Example 1.
Figure 17 is an IR spectrum chart of the blue light- emitting polymer produced through a reaction in the presence of a transition-metal catalyst in Example 1.
Figure 18 is a fluorescence spectrum chart of the blue light-emitting polymer produced through a reaction in the presence of a transition-metal catalyst in Example 1.
Figure 19 is an NMR spectrum chart of the intermediate obtained in Example 2.
Figure 20 is an NMR spectrum chart of the oxadiazole compound obtained in Example 2.
Figure 21 is an NMR spectrum chart of the vinyl compound obtained in Example 2.
Figure 22 is an IR spectrum chart of the vinyl compound obtained in Example 2.
Figure 23 is an NMR spectrum chart of the blue light- emitting polymer obtained in Example 2.
Figure 24 is an IR spectrum chart of the blue light- emitting polymer obtained in Example 2.
Figure 25 is an NMR spectrum chart of the intermediate obtained in Example 3.
Figure 26 is an NMR spectrum chart of the oxadiazole compound obtained in Example 3.
Figure 27 is an NMR spectrum chart of the vinyl compound obtained in Example 3.
Figure 28 is an IR spectrum chart of the vinyl compound obtained in Example 3.
Figure 29 is an NMR spectrum chart of the blue light- emitting polymer obtained in Example 3.
Figure 30 is an IR spectrum chart of the blue light- emitting polymer obtained in Example 3.
Figure 31 is an NMR spectrum chart of the intermediate obtained in Example 4.
Figure 32 is an NMR spectrum chart of the oxadiazole compound obtained in Example 4.
Figure 33 is an NMR spectrum chart of the vinyl compound obtained in Example 4.
Figure 34 is an IR spectrum chart of the vinyl compound obtained in Example 4.
Figure 35 is an NMR spectrum chart of the blue light- emitting polymer obtained in Example 4.
Figure 36 is an IR spectrum chart of the blue light- emitting polymer obtained in Example 4.
Figure 37 is an NMR spectrum chart of the organic halogen compound represented by the formula (50) obtained in Example 6.
Figure 38 is an IR spectrum chart of the organic halogen compound represented by the formula (50) obtained in Example 6.
Figure 39 is an NMR spectrum chart of the vinyl compound obtained in Example 6.
Figure 40 is an IR spectrum chart of the vinyl compound obtained in Example 6.
Figure 41 is an NMR spectrum chart of the blue light- emitting polymer obtained in Example 6.
Figure 42 is an IR spectrum chart of the blue light- emitting polymer obtained in Example 6.
Figure 43 is an NMR spectrum chart of the organic halogen compound represented by the formula (54) obtained in Example 7.
Figure 44 is an IR spectrum chart of the organic halogen compound represented by the formula (54) obtained in Example 7.
Figure 45 is an NMR spectrum chart of the vinyl compound obtained in Example 7.
Figure 46 is an IR spectrum chart of the vinyl compound obtained in Example 7.
Figure 47 is an NMR spectrum chart of the blue light- emitting polymer obtained in Example 7.
Figure 48 is an IR spectrum chart of the blue light- emitting polymer obtained in Example 7.
Figure 49 is an NMR spectrum chart of the organic halogen compound represented by the formula (58) obtained in Example 9.
Figure 50 is an IR spectrum chart of the organic halogen compound represented by the formula (58) obtained in Example 9.
Figure 51 is an NMR spectrum chart of the blue light- emitting polymer, represented by the formula (59), obtained in Example 9.
Figure 52 is an IR spectrum chart of the blue light- emitting polymer, represented by the formula (59), obtained in Example 9.
Figure 53 is an NMR spectrum chart of the first vinyl compound, represented by the formula (63), obtained in Example 12.
Figure 54 is an IR spectrum chart of the first vinyl compound, represented by the formula (63), obtained in Example 12.
Figure 55 is an NMR spectrum chart of the second vinyl compound, represented by the formula (64), obtained in Example 12.
Figure 56 is an IR spectrum chart of the second vinyl compound, represented by the formula (64), obtained in Example 12.
Figure 57 is an NMR spectrum chart of the first blue light-emitting polymer, represented by the formula (65), obtained in Example 12.
Figure 58 is an IR spectrum chart of the first blue light-emitting polymer, represented by the formula (65), obtained in Example 12.
Figure 59 is an NMR spectrum chart of the second blue light-emitting polymer, represented by the formula (66), obtained in Example 12.
Figure 60 is an IR spectrum chart of the second blue light-emitting polymer, represented by the formula (66), obtained in Example 12.

### Detailed Description of the Preferred Embodiments

One of the blue light-emitting compounds of the present invention has a structure represented by the formula (1):

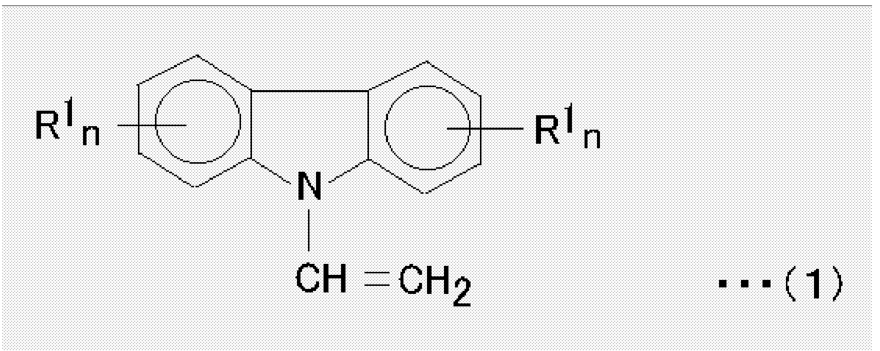

The blue light-emitting compound has an N-vinyl carbazole skeleton, which will be abbreviated to "carbazole skeleton" hereinafter. An R¹ group is bonded to each of the two benzene moieties of the carbazole. Also, a vinyl group is bonded to the nitrogen atom of the carbazole.

"n" in the formula (1) denotes an integer of 1 to 4. One to four R¹s may be bonded to one benzene moiety of the carbazole skeleton.

Two R¹s in the formula (1) may be the same or different from each other. R¹ is a hydrogen atom or an alkyl group having 1 to 10 carbon atoms.

The alkyl group having 1 to 10 carbon atoms for R¹ includes methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group.

R¹ in the formula (1) also stands for a substituent represented by the formula (2) wherein the numerals of 1 to 5 are the numbers showing the positions.

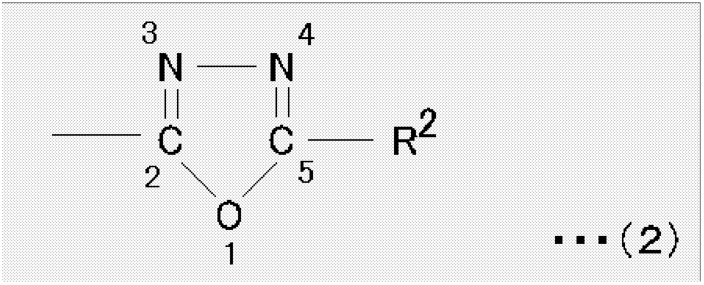

This substituent has a 1,3,4-oxadiazole skeleton, of which the 5-positioned carbon atom is bonded with R².

R² in the formula (2) means an aryl group represented by the formulas (2-1) to (2-8).

The formula (2-1) is:

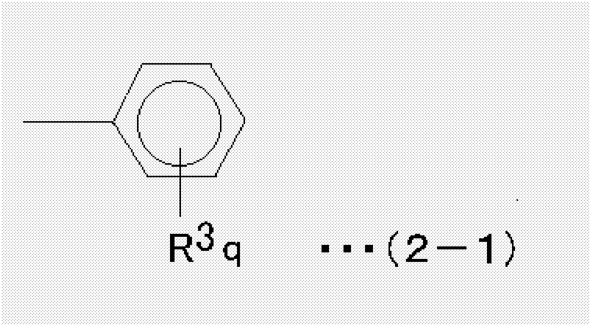

The aryl group shown by the formula (2-1) has a phenyl skeleton, which may be bonded with up to five R³s. R³ is a hydrogen atom or an alkyl group having 1 to 10 carbon atoms, and q denotes an integer of 1 to 5.

Examples of the alkyl group having 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group.

Also, R² in the formula (2) may be an aryl group represented by the formula (2-2) wherein the numerals of 1 to 8 are numbers showing the positions.

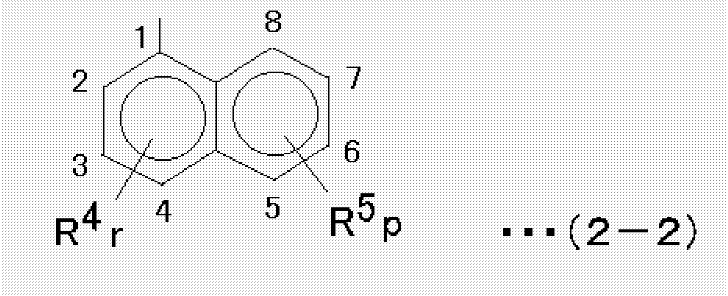

The aryl group represented by the formula (2-2) has a naphthyl skeleton, which may be bonded with R⁴ at the 2-, 3-, and/or 4-position and with R⁵ at the 5-, 6-, 7- and/or 8-position.

R⁴ and R⁵ in this formula mean a hydrogen atom or an alkyl group having 1 to 10 carbon atoms.

Examples of the alkyl group having 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group.

"p" in the formula (2-2) denotes an integer of 1 to 4. The naphthyl skeleton in the formula is bonded with up to four R⁵s. "r" denotes an integer of 1 to 3, and the naphthyl skeleton is also bonded with up to three R⁴s. R⁴ and R⁵ may be the same or different from each other.

R² in the formula (2) also stands for an aryl group represented by the formula (2-3), wherein the numerals of 1 to 8 show the positional numbers.

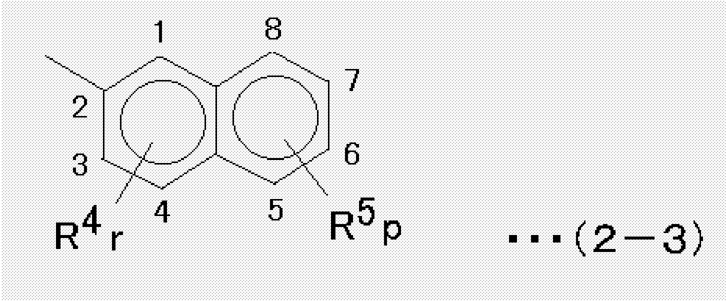

The aryl group represented by the formula (2-3) has a naphthyl skeleton, which may be bonded with R⁴ at the 1-, 3- and/or 4-position and with R⁵ at the 5-, 6-, 7- and/or 8-position.

R⁴, R⁵, p and r mean the same as those defined above. The naphthyl skeleton is bonded with up to three R⁴s and up to four R⁵s. R⁴ and R⁵ may be the same or different from each other.

Furthermore, R² in the formula (2) stands for an aryl group represented by the formula (2-4) wherein the numerals of 1 to 10 are numbers showing the positions.

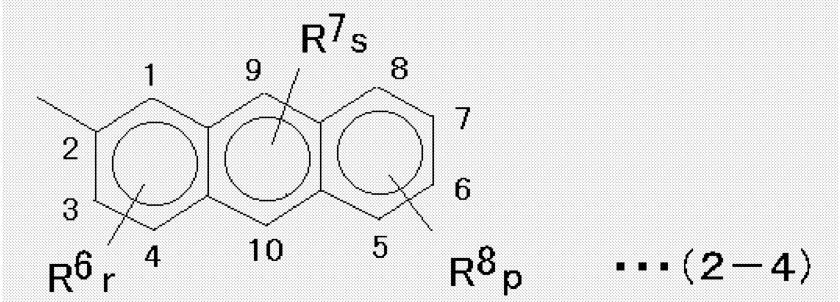

The aryl group represented by the formula (2-4) has a 2-anthryl skeleton, which may be bonded with R⁶ at the 1-, 3- and/or 4-position, with R⁸ at the 5-, 6-, 7-and/or 8-position, and with R⁷ at the 9- and/or 10-position.

Each of R⁶, R⁷ and R⁸ is a hydrogen atom or an alkyl group having 1 to 5 carbon atoms.

Examples of the alkyl group having 1 to 5 carbon atoms are methyl group, ethyl group, propyl group, isopropyl group, n-butyl group, isobutyl group, sec-butyl group, tert-butyl group, n-pentyl group, sec-pentyl group, or tert-pentyl group, etc.

"p" in the formula (2-4) denotes an integer of 1 to 4, and the 2-anthryl skeleton is bonded with up to four R⁸s. "r" in the formula denotes an integer of 1 to 3, and the 2-anthryl skeleton is also bonded with up to three R⁶s. "s" in the formula denotes an integer of 1 to 2, and the 2-anthryl skeleton is bonded with up to two R⁷s.

R⁶, R⁷ and R⁸ in the formula (2-4) may be the same or different from each other.

R² in the formula (2) may still further stand for an aryl group represented by the formula (2-5) wherein the numerals of 1 to 10 means numbers showing the positions.

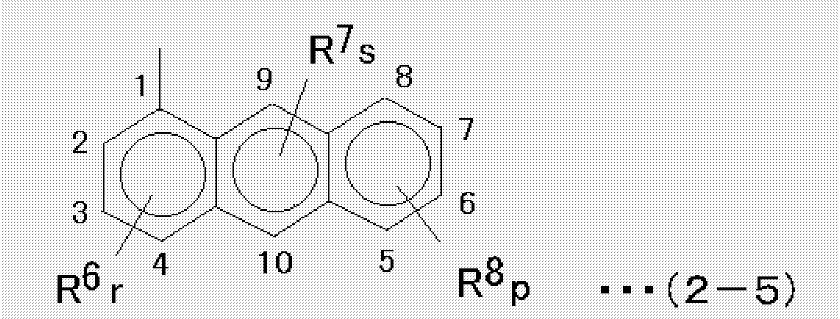

The aryl group has a 1-anthryl skeleton, which may be bonded with R⁶ at the 2-, 3- and/or 4-position, with R⁸ at the 5-, 6-, 7- and/or 8-position, and with R⁷ at the 9- and/or 10-position.

R⁶, R⁷, R⁸, p, r and s in the formula (2-5) mean the same as those defined above. The 1-anthryl skeleton is bonded with up to three R⁶s, up to four R⁸s, and no or up to two R⁷s.

R⁶, R⁷ and R⁸ in the formula may be the same or different from each other.

Moreover, R² in the formula (2) may stand for an aryl group represented by the formula (2-6), wherein the numerals of 1 to 10 are numbers showing the positions.

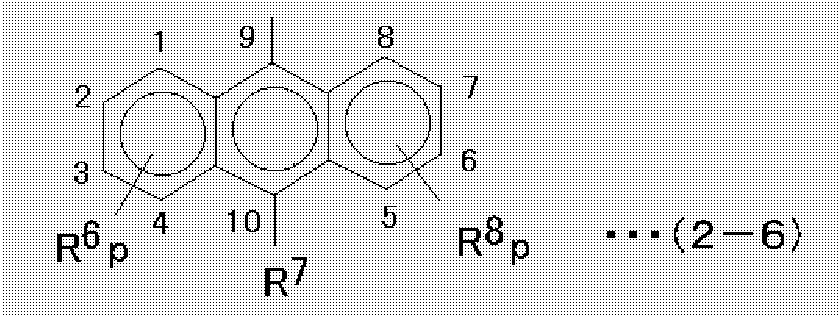

The aryl group represented by the formula (2-6) has a 9-anthryl skeleton, which may be bonded with R⁶ at the 1-, 2-, 3- and/or 4-position, with R⁸ at the 5-, 6-, 7- and/or 8-position and with R⁷at the 10-position.

R⁶, R⁷, R⁸, p, r, and s in the formula (2-6) mean the same as those defined above. The 9-anthryl skeleton is bonded with up to four R⁶s and R⁸s, and one R⁷.

R⁶, R⁷and R⁸ in the formula may be the same or different from each other.

Still further, R² in the formula (2) may stand for an aryl group represented by the formula (2-7) wherein the numerals of 1 to 9 are numbers showing the positions.

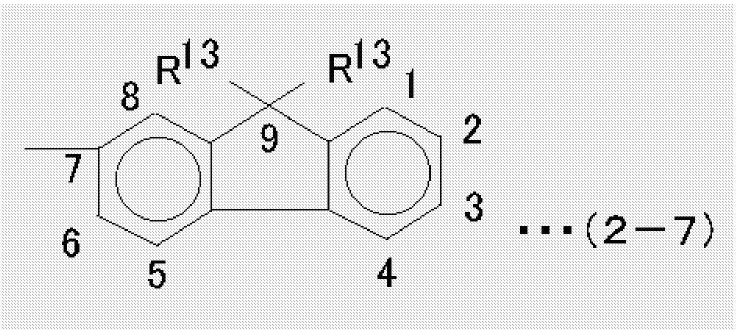

The substituent represented by the formula (2-7) has a fluorene skeleton, of which the 7-positioned carbon atom is bonded with a carbon included in the oxadiazole ring represented by the formula (2) or (3). The 9-positioned carbon atom of the fluorene skeleton is bonded with two R¹³s.

R¹³ is a hydrogen atom or an alkyl group having 1 to 10 carbon atoms. Examples of the alkyl group having 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group. Alkyl groups having 1 to 3 carbon atoms are particularly preferable.

Two R¹³s in the formula (2-7) may the same or different from each other.

R² in the formula (2) also stands for an aryl group represented by the formula (2-8) wherein the numerals of 1 to 4 and 1' to 4' are the numbers showing the positions.

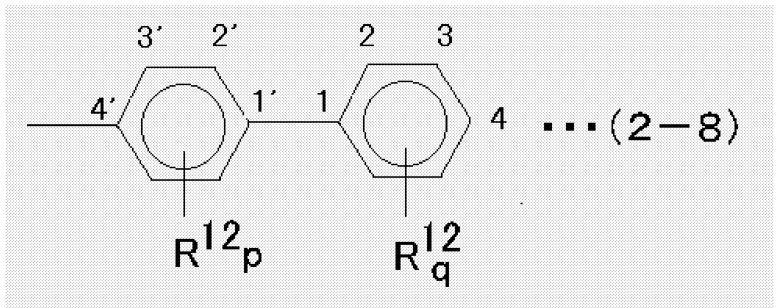

The substituent represented by the formula (2-8) has a biphenyl skeleton, which is bonded with a carbon included in the oxadiazole ring represented by the formula (2) or (3) at, for example, the 4'-position.

R¹² is a hydrogen atom or an alkyl group having 1 to 10 carbon atoms. Examples of the alkyl group having 1 to 10 carbon atoms are methyl group, ethyl group, propyl group, isopropyl group, n-butyl group, isobutyl group, sec-butyl group, tert- butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group.

Two R¹²s in the formula (2-8) may the same or different from each other.

"p" in the formula (2-8) means the same as that defined above, and "q" denotes an integer of 1 to 5. The biphenyl skeleton may be bonded with up to nine R¹²_{s.}

Another blue light-emitting compound of the present invention has a structure represented by formula (1'):

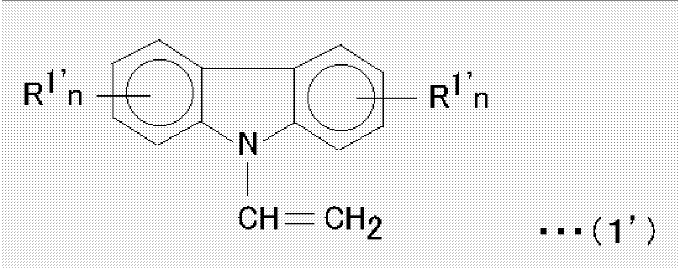

The blue light-emitting compound has an N-vinyl carbazole skeleton, which will be abbreviated to "carbazole skeleton" hereinafter. An R^{1'} group is bonded to each of the two benzene moieties of the carbazole. Also, a vinyl group is bonded to the nitrogen atom of the carbazole.

"n" in the formula (1') denotes an integer of 1 to 4. One to four R^{1'}s may be bonded to one benzene moiety of the carbazole skeleton.

Two R^{1'}s in the formula (1') may be the same or different from each other. R^{1'} is a hydrogen atom or an alkyl group having 1 to 10 carbon atoms.

The alkyl group having 1 to 10 carbon atoms for R^{1'} includes methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group.

R1' in the formula (1') also stands for a substituent represented by the formula (2').

The formula (2') is: 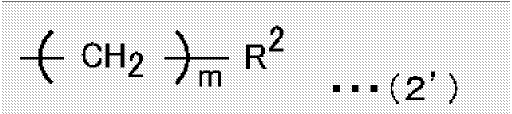 wherein R² is the substituent represented by one of the formulas (2-1) to (2-8).

"m" in the formula (2') denotes an integer of 0 to 3, which means the number of methylene group between the carbon of the benzene moiety and R² in the formula (2').

R² in the formula (2') is the substituent represented by the formula (2'-1):

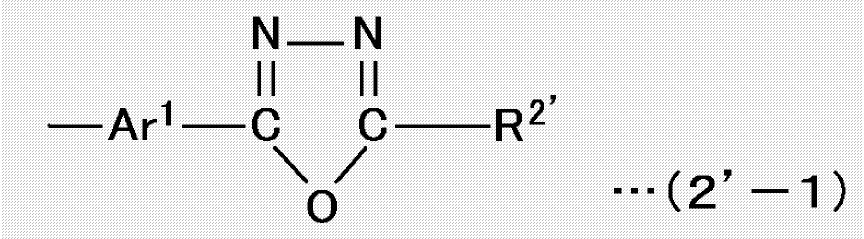

The substituent represented by the formula (2'-1) consists of Ar¹, 1,3,4-oxadiazole and R^{2'}.

Ar¹ is a phenylene group, a naphthylene group, or an anthrylene group, which is made through the removal of two hydrogen atoms from anthracene.

One of the two carbon atoms that 1,3,4-oxadiazole has is bonded with R^{2'} and the other with Ar¹.

When m is 1 to 3, Ar¹ is bonded with a carbon atom of the alkylene group in the formula (2'). On the other hand, when m is 0, it is bonded with a carbon atom of the benzene moiety in the formula (1').

The carbon atom of the alkylene group in the formula (2') or the benzene moiety and the carbon atom of the 1,3,4-oxazole are bonded with Ar¹, having the following positional relationships.

For example, when Ar¹ is a phenylene group and the carbon atom of the alkylene group in the formula (2') or the benzene moiety is bonded with the 1-positioned carbon atom of the phenylene group, the carbon atom of the 1,3,4-oxazole may be bonded with the 3- or 4-positioned carbon atom of the phenylene group.

Also, for example, when Ar¹ is a naphthylene group and the carbon atom of the alkylene group in the formula (2') or the benzene moiety is bonded with the 1-positioned carbon atom of the naphthylene group, the carbon atom of the 1,3,4-oxazole may be bonded with the 3-, 4-, 5-, 6- or 7-positioned carbon atom of the naphthylene group.

Moreover, when Ar¹ is a naphthylene group and the carbon atom of the alkylene group in the formula (2') or the benzene moiety is bonded with the 2-positioned carbon atom of the naphthylene group, the carbon atom of the 1,3,4-oxazole may be bonded with the 4-, 5-, 6-, 7- or 8-positioned carbon atom of the naphthylene group.

Furthermore, when Ar¹ is an anthrylene group and the carbon atom of the alkylene group in the formula (2') or the benzene moiety is bonded with the 1-positioned carbon atom of the anthrylene group, the carbon atom of the 1,3,4-oxazole may be bonded with the 3-, 4-, 5-, 6-, 7-, 8- or 10-positioned carbon atom of the anthrylene group.

Still further, when Ar¹ is an anthrylene group and the carbon atom of the alkylene group in the formula (2') or the benzene moiety is bonded with the 2-positioned carbon atom of the anthrylene group, the carbon atom of the 1 ,3,4-oxazole may be bonded with the 4-, 5-, 6-, 7-, 8-, 9- or 10-positioned carbon atom of the anthrylene group.

Moreover, when Ar¹ is an anthrylene group and the carbon atom of the alkylene group in the formula (2') or the benzene moiety is bonded with the 9-positioned carbon atom of the anthrylene group, the carbon atom of the 1,3,4-oxazole may be bonded with the 2-, 3-, 4-, 5-, 6-, 7-, or 10-positioned carbon atom of the anthrylene group.

R²' in the formula (2'-1 ) is the substituent represented by one of the formulas (2-1) to (2-8).

The blue light-emitting compound represented by the formula (1) may be prepared in the following manners.

Specifically, carbazole and a 2-ethyl halide-p-toluene- sulfonate, which are the starting substances, are heated in a solvent, so that the hydrogen atom bonded to the nitrogen atom of the carbazole is replaced with the alkyl halide.

Examples of the solvent are ethanol, methanol, acetic anhydride, acetic acid, an ether, acetone, etc.

The temperature for the replacement reaction of the hydrogen atom with the alkyl halide is a temperature of 40 to 80°C, preferably 50 to 70°C.

After the reaction is completed, the corresponding N-2- (ethyl halide)carbazole can be obtained through purification and separation by ordinary methods.

Then, the obtained N-2-(ethyl halide)carbazole and phosphoryl chloride are heated in a solvent, so that the corresponding aldehyde compound is obtained by formylation. Then, the aldehyde compound is oxidized into the corresponding carboxylic acid. The obtained carboxylic acid is reacted with a halogenating agent to produce a halogen compound represented by the formula (4).

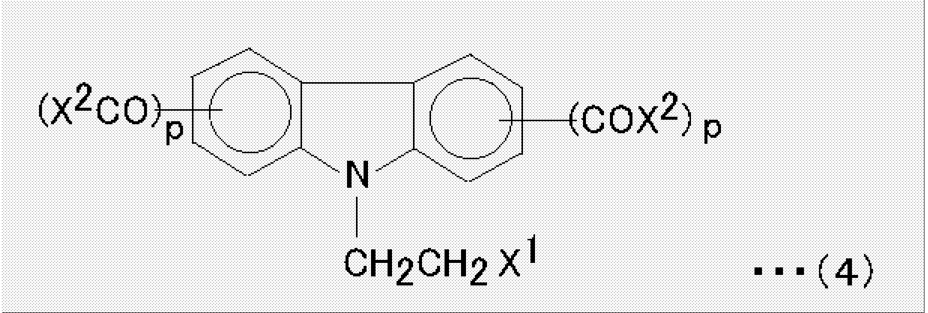

In the formula (4), X¹ and X² mean a halogen atom such as fluorine atom, chlorine atom, bromine atom or iodine atom. "p" denotes an integer of 1 to 4. Each of the benzene moieties may be bonded with up to four haloformyl groups (-COX²).

X¹ and X² in the formula (4) may be the same or different from each other.

The solvent used in the formylation includes, for example, ethanol, methanol, formic acid, acetic anhydride, acetic acid, diethyl ether, an epoxide, an acid anhydride having 5 or less carbon atoms, dichloroethane, dichloromethane, trichloro- ethane, chloroform, carbon tetrachloride, benzene, toluene, pyridine, dioxane, DMF and THF.

A catalyst may be used in the formylation, if necessary. Examples of the catalyst are aluminum chloride, zinc chloride, zinc cyanide, thionyl chloride, phosphoryl chloride, etc.

The temperature suitable for the formylation is from 60 to 100°C, preferably from 75 to 95°C.

For the halogenating agent may be employed a commonly used agent, such as thionyl chloride, sulfenyl chloride, sulfuryl chloride, phosphoryl chloride, phosphorus trichloride, phosphorus pentachloride, hydrogen fluoride, chlorine trifluoride, phosphorus trifluoride, iodine pentafluoride, hydrogen bromide, hypobromous acid or thionyl bromide. Of them are preferred thionyl chloride, sulfenyl chloride and sulfuryl chloride.

After the reactions are completed, the halogenated compound can be obtained through purification and separation by ordinary methods.

Then, the halogenated compound represented by the formula (4) and a hydrazide represented by formula (4a) are heated in a solvent at a temperature of 50 to 80°C. The reaction progresses easily to the production of the intermediate represented by formula (5).

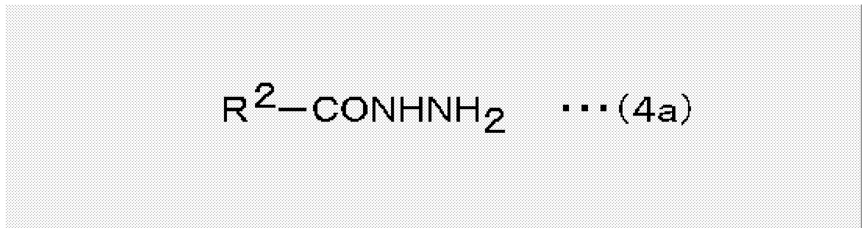

In the formula (4a), R² denotes the same as that defined hereinabove.

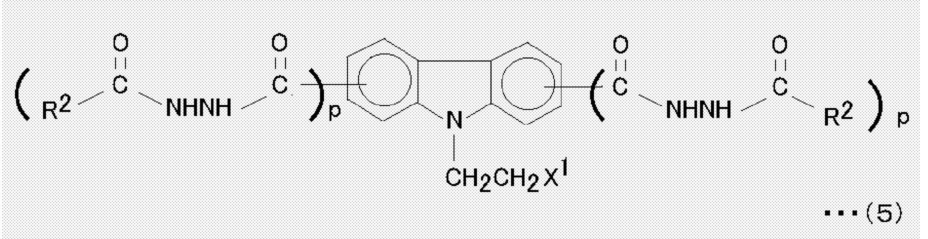

In the formula (5), X¹ means the same as that defined above and "p" denotes an integer of 1 to 4.

The solvent includes, for example, acetic anhydride, acetic acid, an acid anhydride having 5 or less carbon atoms, an aromatic solvent such as benzene and toluene, pyridine, dioxane, DMF and a polycyclic compound solvent such as THF.

The obtained intermediate is then subjected to dehydration and dehalogenation, which results in the formation of the blue light-emitting compound of the invention.

The dehydrating agent employed for the dehydration includes, for example, an inorganic dehydrating agent such as phosphorous pentoxide, phosphorous pentachloride, phosphorous pentasulfide, phosphorous trichloride, thionyl chloride, sulfonylchloride and phosphoryl chloride, acetic anhydride, and an organic dehydrating agent such as an acid anhydride having 5 or less carbon atoms. The inorganic dehydrating agent is preferred to the organic one. The reaction progresses easily at a temperature of 100 to 150°C.

The dehydration reaction takes place between the hydrogen atoms of the hydrazo group (-NHNH-) and the oxygen atom of the carbonyl group, and an oxadiazole ring is formed simultaneously.

The dehalogenation reaction may be carried out in a known way. The reaction progresses easily at a temperature of 50 to 150°C, preferably 70 to 120°C.

The blue light-emitting monomer of the present invention can be obtained through purification and separation by known methods after the completion of the reaction.

The polymerization of the blue light-emitting compounds, which are monomers, by heating them in an organic solvent results in the blue light-emitting polymer of the present invention.

Examples of the organic solvent are benzene, toluene, an ether, THF, DMF, etc. These solvents may be used singly or in combination.

The temperature for the polymerization is 60 to 120°C, preferably 80 to 100°C.

The blue light-emitting compound represented by the formula (1') may be produced in the following manner.

Specifically, carbazole and a 2-ethyl halide-p-toluene- sulfonate, which are the starting substances, are heated in a solvent, so that the hydrogen atom bonded to the nitrogen atom of the carbazole is replaced with the alkyl halide. As a result, an N-2-(ethyl halide)carbazole is obtained.

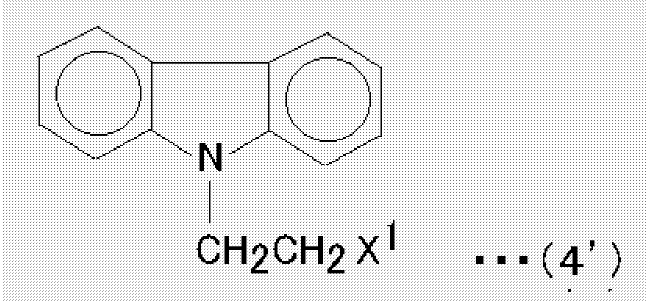

X¹ in the formula (4') means a halogen atom such as fluorine atom, chlorine atom, bromine atom or iodine atom.

Examples of the solvent are ethanol, methanol, acetic anhydride, acetic acid, an ether, acetone, etc.

The temperature for the replacement reaction of the hydrogen atom with the alkyl halide is a temperature of 40 to 80°C, preferably 50 to 70°C.

After the reaction is completed, the corresponding N-2- (ethyl halide)carbazole can be obtained through purification and separation by ordinary methods.

Then, the N-2-(ethyl halide)carbazole and a halogen compound represented by the formula R²-CH₂CH₂X² are heated in a solvent with a catalyst at a temperature of 140 to 160°C. The reaction progresses easily, so that the corresponding organic halogen compound represented by formula (5') is obtained.

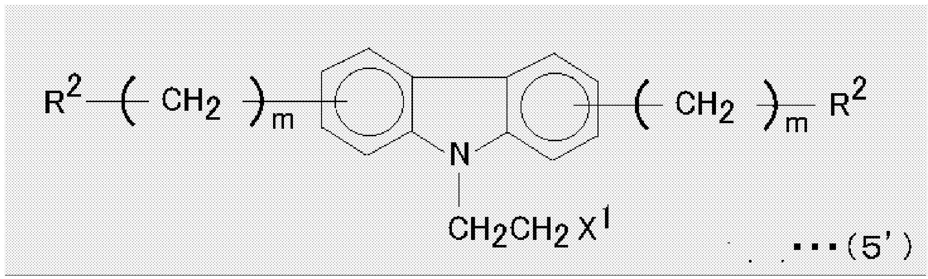

x¹ and R² in the formula (5') mean the same as those defined above. "m" denotes an integer of 0 to 3.

X² in the formula R²-CH₂CH₂X² means a halogen atom such as fluorine atom, chlorine atom, bromine atom or iodine atom.

The organic halogen compound may be obtained by heating an aromatic hydrocarbon in a solvent with a hydrogen halide gas being introduced into the solvent.

The aromatic hydrocarbon includes, for example, benzene, naphthalene, anthracene, biphenyl, or derivatives thereof. Benzene, naphthalene or derivatives thereof are preferable among them.

The derivatives of benzene include, for example, toluene, o-xylene, m-xylene and p-xylene.

The derivatives of naphthalene include, for example, a monomethyl naphthalene such as 1-methyl naphthalene or 2-methyl naphthalene, a dimethyl naphthalene such as 1,2-dimethyl naphthalene, 1,3-dimethyl naphthalene, 1,4-dimethyl naphtha- lene, 1,5-dimethyl naphthalene, 1,6-dimethyl naphthalene or 1,7-dimethyl naphthalene, or a trimethyl naphthalene, such as 1,3,4-trimethyl naphthalene or 1,3,5-trimethyl naphthalene.

The solvent includes, for example, acetic anhydride, acetic acid, an acid anhydride having 5 or less carbon atoms, an aromatic solvent such as benzene and toluene, pyridine, dioxane, DMF and a polycyclic compound solvent such as THF.

Examples of the hydrogen halide gas may be a hydrogen fluoride gas or a hydrogen chloride gas.

The concentration of the hydrogen halide gas may be 100%. The concentration of 1 to 95% is particularly preferable. For the gas to dilute the hydrogen halide gas is preferably an inert gas such as nitrogen gas or argon gas.

The temperature for the reaction is 40 to 60°C, preferably 50 to 55°C.

The catalyst employed in the reaction to produce the organic halogen compound represented by the formula (5') includes, for example, aluminum chloride, zinc or sulfuric acid.

The solvent used in the reaction to produce the organic halogen compound represented by the formula (5') includes, for example, acetic anhydride, acetic acid, an acid anhydride having 5 or less carbon atoms, an aromatic solvent such as benzene and toluene, pyridine, dioxane, DMF and a polycyclic compound solvent such as THF.

The organic halogen compound obtained in this way is subjected to dehydrohalogenation, then purification and separation, which results in the blue light-emitting compound represented by the formula (1').

The blue light-emitting compound is polymerized by the heating in an organic solvent. The resultant is the blue light-emitting polymer represented by the formula (1''') above.

Examples of the solvent are benzene, toluene, an ether, THF, DMF, etc. These solvents may be used singly or in combination of two or more.

The temperature for the polymerization is 70 to 130°C, preferably 90 to 110°C.

The blue light-emitting compounds of the invention, which are those represented by the formulas (1) and (1'), can easily be produced from cheap raw materials through simple reactions. Moreover, since the reactions progress at relatively low temperatures, the production can be carried out safely. Therefore it can be said that these simple processes of producing the blue light-emitting compounds are industrial methods.

Also, the average molecular weights of the blue light-emitting polymers of the present invention, which are those represented by the formulas (1 ") and (1'''), are preferably 10000 to 500000, more preferably 20000 to 300000.

The blue light-emitting compounds and blue light- emitting polymers of the present invention have the carbazole skeletons that have large Π electron clouds. In addition, the carbazole skeletons are protected from environmental influences by the aryl groups represented by R¹ or R^{1'}, which leads to an increase in the densities of the Π electron clouds on each repeating unit. Then, it is surmised that the compounds are made more stable, which, in turn, results in the emission of blue light by the application of a small energy. The blue light-emitting polymers of the present invention are characterized by their repeating unit having the carbazole skeleton, which has large Π electron clouds, with R¹, the polycyclic compound, being bonded to each of their benzene moieties. Because these polymers have stable repeating units, they become chemically stable, which leads to their special properties; they do not easily deteriorate even under severe conditions.

The luminescent element utilizing the blue light- emitting polymers of the present invention will be explained in the followings.

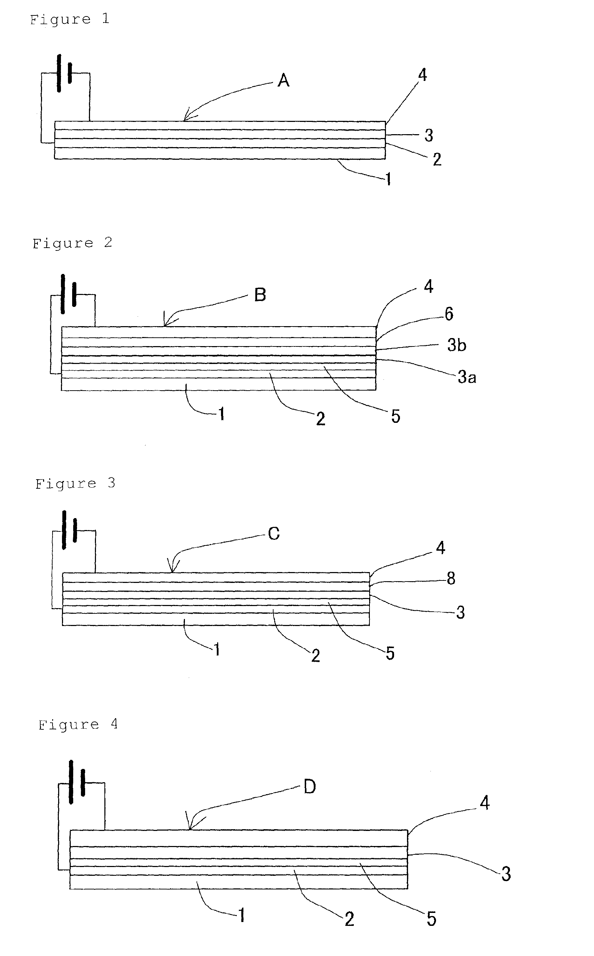

Figure 1 is a schematic illustration that shows the sectional structure of a luminescent element according to the present invention, which is a one-layer type organic EL element. As shown in this figure, the luminescent element A is prepared by layering a light-emitting layer 3 and an electrode layer 4 in this order on a substrate 1 with which a transparent electrode 2 has been provided.

When the luminescent element shown in Figure 1 includes a blue light-emitting polymer of the present invention, a red light-emitting compound and a green light-emitting compound at a balanced composition, it emits white light upon the application of electricity through the transparent electrode 2 and the electrode layer 4. The total amount of the blue light- emitting polymer of the present invention, the red light- emitting compound and the green light-emitting compound, and the proportion of the amount of the blue light-emitting polymer to that of the red light-emitting compound to that of the green light-emitting polymer, included in the layer 3 to let the element emit white light, vary depending on the kind of each compound. They are decided for each luminescent element depending on the kind of each compound included therein. When the luminescent element is intended to emit blue light, the light-emitting layer 3 may include only a blue light-emitting polymer of the present invention. Also, when this luminescent element is intended to emit light of any color other than white and blue, the total amount of the compounds and the polymer and their respective amounts should be changed depending on the color. For example, when the luminescent element including a blue light-emitting polymer of this invention is intended to emit white light, the ratio of the amount of the blue light-emitting polymer to that of the red light-emitting compound to that of the green light-emitting polymer is usually 5-200:10-100:50-20000 in weight, preferably 10-100:50-500: 100-10000.

For the red-light emitting compound is suitable the Nile Red luminescent compound emitting red light represented by the formula (6).

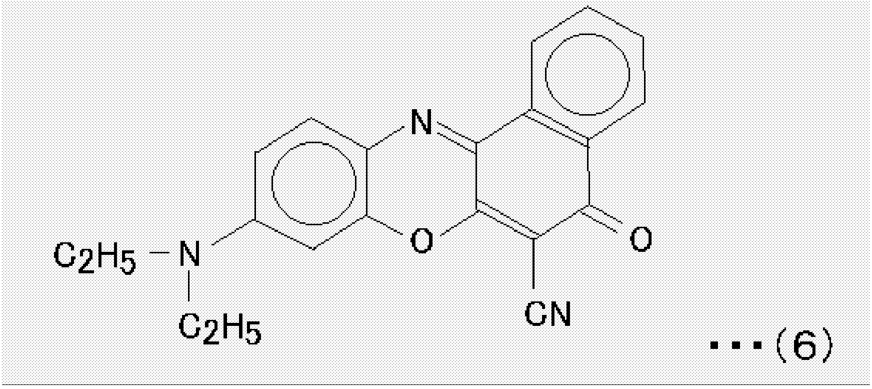

For the green light-emitting compound is suitable a coumarin compound emitting green light, an indophenol compound emitting green light and an indigo compound emitting green light. The coumarin compound represented by the formula (7) is preferable among them.

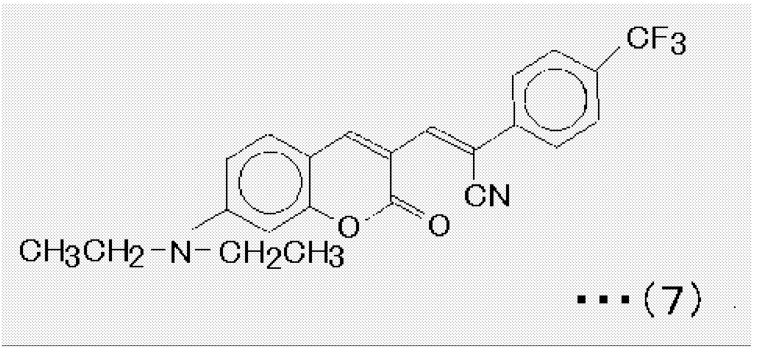

Upon the application of an electric field between the transparent electrode 2 and the electrode layer 4, electrons are injected from the electrode layer 4 and positive holes are injected from the transparent electrode 2. In the light-emitting layer 3, the electrons are recombined with positive holes, which causes the energy level to return to the valence band from the conduction band. This transition of the energy level is accompanied by emission of the energy differential as light.

The luminescent element A shown in Figure 1, when it is shaped to a planar one with a large area, may be used as a planar illuminator, for example a large-area wall illuminator emitting white light when fixed on a wall, or a large-area ceiling illuminator emitting white light when fixed on a ceiling. This light-emitting element may be used as a planar light source in place of a point light source, such as a conventional bulb, and a line light source, such as a conventional fluorescent lamp. In particular, this white light-emitting illuminator can suitably be used to light up walls, ceilings and floors in dwelling rooms, offices and passenger trains, or to make them emit light. Moreover, this luminescent element A may be suitable for the backlight used in displays of computers, cellular phones and ATMs. Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because the light-emitting element A includes a blue light-emitting polymer of the present invention, which has the special chemical structure, in the light-emitting layer, it has a long life. Therefore, light sources employing the luminescent element A will naturally have a long life.

As understood from the foregoing, when the light-emitting layer of the luminescent element A includes a blue light-emitting polymer of the present invention and not the red light-emitting compound or the green light-emitting compound, the luminescent element A emits clear blue light.

The luminescent element A may also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because this luminescent element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon.

When the substrate 1 is opaque, the luminescent element, which includes the red light-emitting compound, the green light-emitting compound and a blue light-emitting polymer of the present invention, is a single-faced illuminator that emits white light from one side of the element. On the other hand, when the substrate 1 is transparent, the luminescent element is a double-faced illuminator that emits white light from both of the substrate and the surface layer opposite to the substrate.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 includes a blue light-emitting polymer of the present invention when the layer 3 is intended to emit blue light. It includes the red light-emitting compound and the green light-emitting compound in addition to a blue light-emitting polymer of the present invention when it is intended to emit white light. The blue light-emitting polymer of the present invention, or the red light-emitting compound, the green light-emitting compound and the blue light-emitting compound of the present invention in the shape of a film may be formed on the transparent electrode 2.

A typical example of forming the blue light-emitting polymer film on the transparent electrode 2 may be the application of a solution of the polymer dissolved in a suitable solvent onto the transparent electrode. The application method includes, for example, a spin cast method, a brush-coating method, etc.

The amount of the blue light-emitting polymer, or the red light-emitting compound, the green light-emitting compound and the blue light-emitting polymer in the light-emitting layer 3 is, typically, 0.01 to 2 weight%, preferably, 0.05 to 0.5 weight%.

The thickness of the light-emitting layer 3 ranges, typically, between 30 nm and 500 nm, preferably between 100 nm and 300 nm. When the thickness is too small, the amount of the emitted light may be insufficient. On the other hand, when the thickness is too large, voltage required to drive the element may be too high, which is not desirable. Besides, the large thickness may reduce the flexibility of the element necessary to shape a planar, tubular, curved or ring article.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode may easily be formed on the surface of light-emitting layer 3, which, in turn, has been formed on substrate 1, by the technique of metal deposition.

When either of the deposition or the application is employed, a buffer layer should be inserted between the electrode layer and the light-emitting layer.

Materials for the buffer layer are, for example, an alkaline metal compound such as lithium fluoride, an alkaline earth metal compound such as magnesium fluoride, an oxide such as an aluminum oxide, and 4,4'-biscarbazole biphenyl(Cz-TPD). Also, materials for forming the buffer layer between the cathode made of ITO, etc. and the organic layer are, for example, m-MTDATA (4,4',4''-tris(3-methylphenyl-phenylamino)triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescent element, improve the quantum efficiency of luminescence, and achieve an increase in the luminance of the emitted light.

Next, the second example of the luminescent element in accordance with this invention is shown in Figure 2. This figure is an illustration showing the sectional layer structure of an example of the luminescent element, which is a multi-layer organic EL element.

As shown in Figure 2, the luminescent element B comprises a substrate 1, and a transparent electrode 2, a hole- transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises the light-emitting sublayers 3a and 3b. The light- emitting sublayer 3a is a deposited film including light- emitting compounds. The light-emitting sublayer 3b is a layer that functions as a host.

Examples of the hole-transporting substance included in the hole-transporting layer 5 are a triphenylamine compound such as N,N'-diphenyl-N,N'-di(m-tolyl)-benzidine (TPD) and α-NPD, a hydrazon compound, a stilbene compound, a heterocyclic compound, a Π electron star burst positive hole transporting substance, etc.

Examples of the electron-transporting substance included in the electron-transporting layer 6 are an oxadiazole derivative such as 2-(4-tert-butylphenyl)-5-(4-biphenylyl)- 1,3,4-oxadiazole and 2,5-bis(1-naphthyl)-1,3,4-oxadiazole (BND), and 2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The luminescent element B shown in Figure 2 employs Alq3 as electron-transporting substance in the electron- transporting layer 6.

The thickness of each layer is the same as that in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of this invention is shown in Figure 3. This figure is an illustration showing the sectional layer structure of an example of the luminescent element, which is a multi-layer organic EL element.

The luminescent element C shown in Figure 3 comprises a substrate 1, and a transparent electrode 2, a hole- transporting layer 5, a light-emitting layer 3, an electron- transporting layer 8, and an electrode layer 4, wherein the transparent electrode and the layers are laid on substrate 1 one by one in this order.

The luminescent element C functions in the same way as the luminescent element B.

Another example of the luminescent element of this invention is shown in Figure 4. The luminescent element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer low molecular weight organic luminescent element having a hole-transporting layer that includes a hole-transporting substance and an electron-transporting luminescent layer that includes a blue light- emitting element of the invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a luminescent layer that includes a host pigment and a blue light-emitting polymer of this invention as a guest pigment, wherein the luminescent layer is laid on the hole-transporting layer and these layers are sandwiched between the cathode and the anode. Another example is a two-layer organic luminescent element comprising a hole-transporting layer that includes a hole- transporting substance and an electron-transporting luminescent layer that is prepared through a co-deposition of a blue light-emitting polymer of the invention and an electron-transporting substance, the latter layer being laid on the former, and these two layers being sandwiched between the cathode and the anode. A specific example of the second embodiment is a two-layer pigment-injected luminescent element comprising a hole-transporting layer and an electron- transporting luminescent layer that includes a host pigment and a blue light-emitting polymer of this invention as a guest pigment, wherein the luminescent layer is laid on the hole-transporting layer and these layers are sandwiched between the cathode and the anode. A further example is a three-layer organic luminescent element comprising a hole-transporting layer, a luminescent layer including a blue light-emitting polymer of this invention that is laid on the hole-transporting layer, and an electron-transporting layer that is laid on the luminescent layer, these layers being sandwiched between the cathode and the anode.

Also, it is preferred if the luminescent layer includes, as a sensitizing agent, rubrene, especially both of rubrene and A1q3.

A blue light-emitting element utilizing a blue light- emitting polymer of this invention, or a white light-emitting element utilizing the red light-emitting compound, the green light-emitting compound and a blue light-emitting polymer of the present invention may generally be used for an organic EL element driven by direct current, and also by pulses and alternating current.

### Examples

### (Example 1)

### - Synthesis 1: Synthesis of a Blue Light-emitting Polymer -

### <Synthesis of Organic Halogen Compound>

In a 2L three-necked flask were placed 46.49 g of carbazole, 100 g of 2-chloroethyl-p-toluenesulfonate, 52.9 g of sodium hydroxide, 33 ml of water and 1200 ml of acetone. The flask containing the mixture was placed in a water bath and the mixture was heated to 60°C. The reaction was allowed to continue for 19 hours. After the termination of the reaction, the obtained mixture was filtered while it was hot. Then, the filtrate was concentrated and dried up to solids. To the obtained solids were added 1500 ml of toluene. The solution thus made was filtered, and the obtained filtrate was concentrated and dried up. 25 g of a milky white solid matter was obtained.

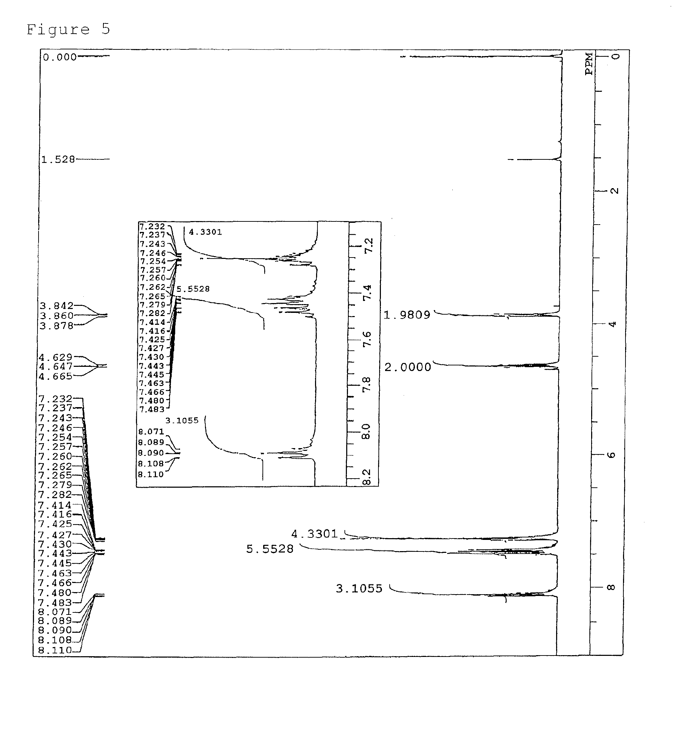

An NMR spectrum chart of the solid matter is shown in Figure 5. This milky white solid matter was identified as N-(2-chloroethyl)carbazole having the structure represented by formula (8).

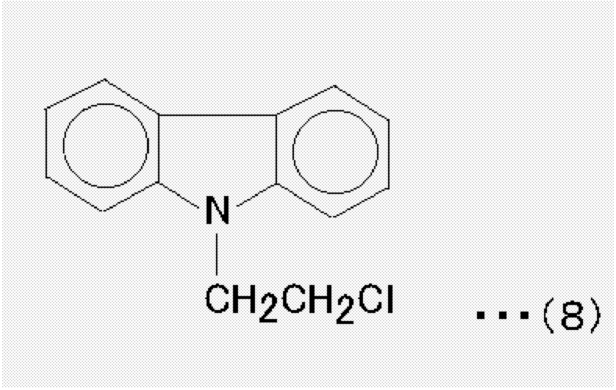

### <Formylation of the Organic Halogen Compound>

In a 500 ml three-necked flask, 28.1 g of the obtained organic halogen compound, 196 g of phosphoryl chloride, 93.6 g of DMF and 200 ml of dichloroethane were placed. The flask containing the mixture was placed in a water bath and the mixture was heated to 85°C. The reaction was allowed to continue for 2.5 hours. After the termination of the reaction, the resultant solution was cooled with ice. After the cooling, the solution was introduced into ice water, and made alkaline by the addition of sodium hydroxide. Then, chloroform was added to the alkaline solution, and the resultant mixture was extracted and separated. The separated oil layer was dried up, and 200 ml of methanol was added to the dried. The resulting mixture was then stirred sufficiently and filtered. The solids obtained by the filtration were dried to a solid matter of 25 g.

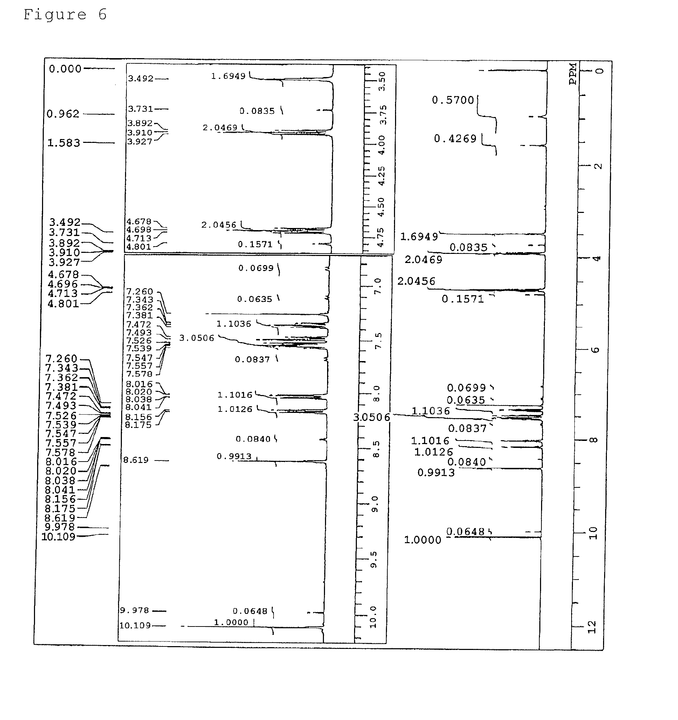

An NMR spectrum chart of the solid matter is shown in Figure 6. The solid matter was identified as an aldehyde compound having the structure represented by formula (9).

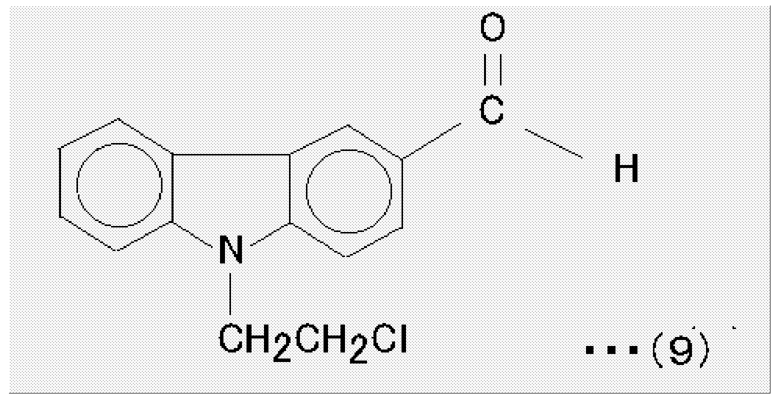

### <Synthesis of Carboxylic Acid Compound>

In a 1 L four-necked flask were placed 25 g of the obtained aldehyde compound and 367 ml of water. The solution in the flask was heated in a water bath up to 80°C. Then, a potassium permanganate aqueous solution, which consisted of 20.7 g of potassium permanganate and 437 ml of water, was added to the solution in the flask, and the contents in the flask was heated for 30 minutes with being stirred. Then, 20 ml of 10% potassium hydroxide aqueous solution was added to the flask and the resulting solution was filtered. Next, the filtrate was made acid, and the precipitated solids were collected by filtration and dried. 3.1 g of an ocher solid matter was obtained.

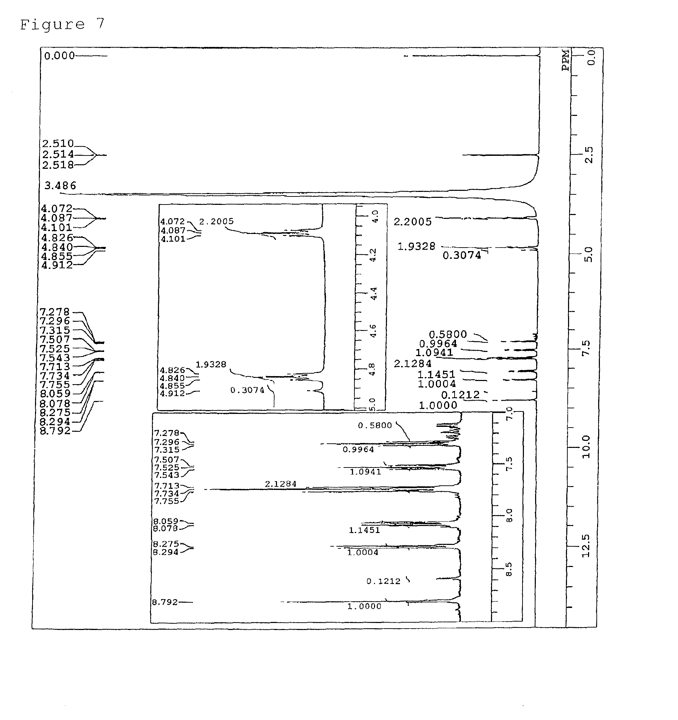

An NMR spectrum chart of the obtained solid matter is shown in Figure 7. The solid matter was identified as a carboxylic acid compound having the structure represented by formula (10).

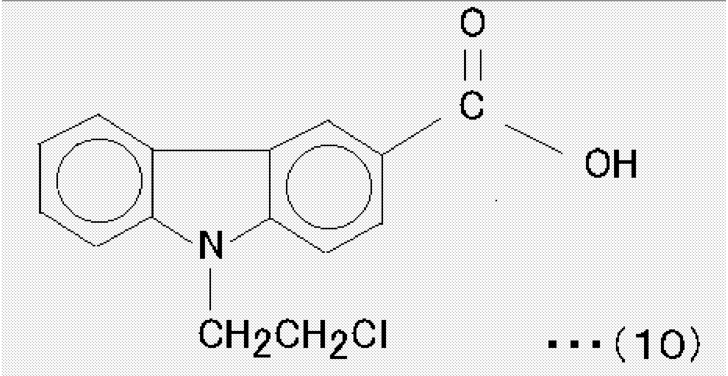

### <Synthesis of Acid Chloride Compound>

A 200 ml pear-shaped flask was charged with 3.0 g of the carboxylic acid compound and 50 ml of thionyl chloride. The solution in the pear-shaped flask was heated in a silicone oil bath to 80°C and allowed to react for 2 hours. After the completion of the reaction, the solids obtained by removing the solvent were dissolved in 100 ml of THF. Then, the solvent was removed again and 3.0 g of brownish green powdery solids were obtained.

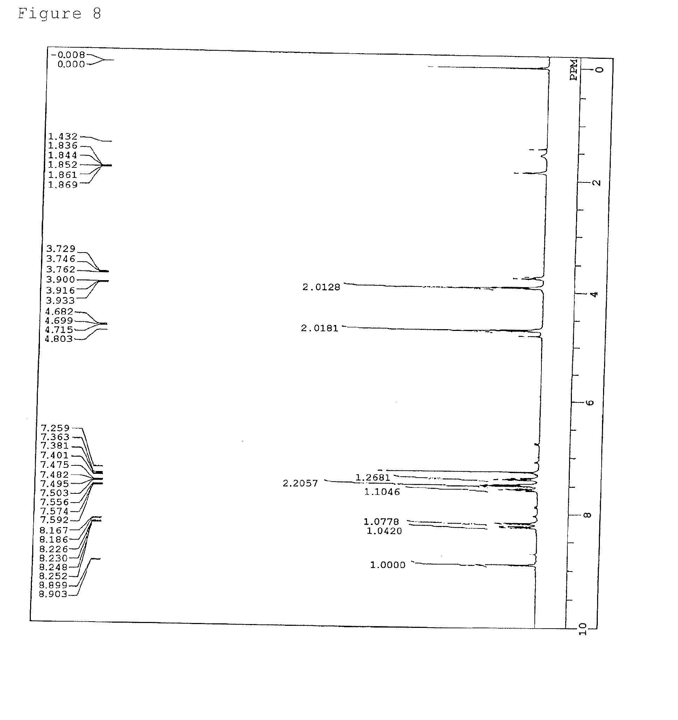

An NMR spectrum chart of the obtained solids is shown in Figure 8. The obtained was identified as an acid chloride compound having the structure represented by formula (11).

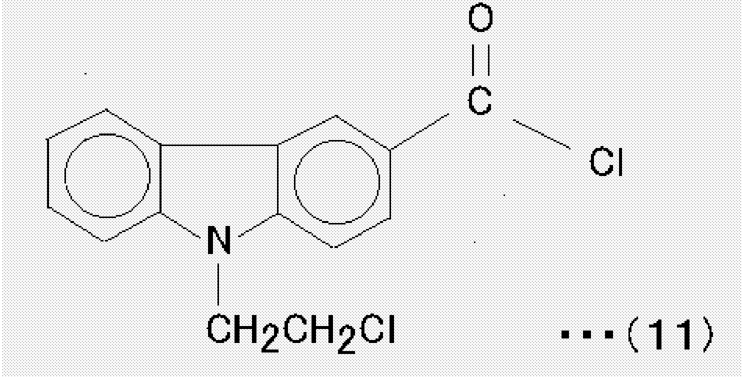

### <Synthesis of Intermediate>

In a 200 ml three-necked flask were placed 1.5 g of the acid chloride compound, 0.92 g of 1-naphthyl hydrazide, 0.47 g of pyridine and 100 ml of THF. The solution in the three-necked flask was heated in a water bath to 60°C and allowed to react for 2 hours. After the completion of the reaction, the solution was cooled with ice. Water was added to the solution, and the precipitated solids were collected by filtration and dried. 2.1 g of a solid matter was obtained.

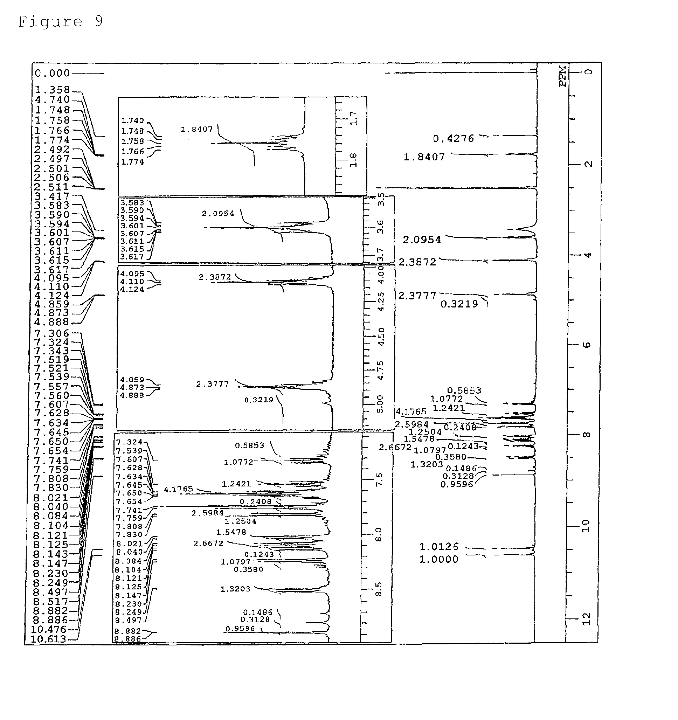

An NMR spectrum chart of the obtained solid matter is shown in Figure 9. The obtained was identified as an intermediate having the structure represented by formula (12).

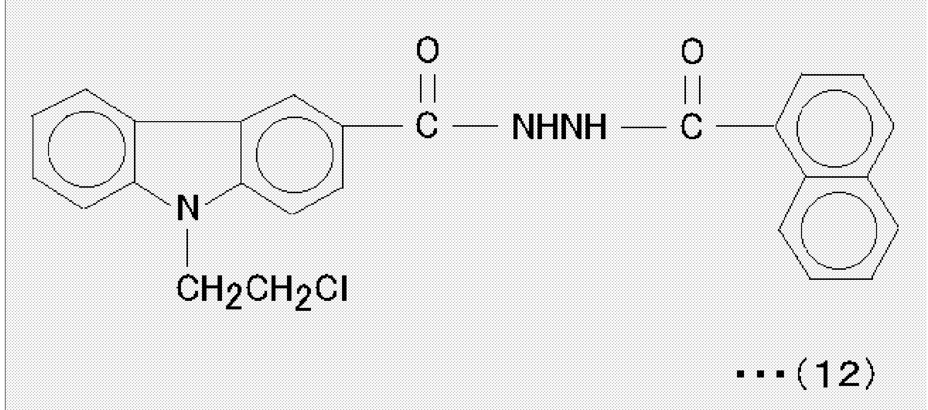

### <Dehydration and Cyclization>

A 200 ml three-necked flask was charged with 2.1 g of the intermediate and 100 ml of phosphoryl chloride. The solution in the three-necked flask was heated in a silicone oil bath to 120°C and allowed to react for 5 hours. After the completion of the reaction, the solvent was removed and the remaining was introduced into ice water. Solids were precipitated. The solids were dissolved in chloroform, and the resultant solution was washed with water, concentrated and dried up. 1.7 g of a yellow solid matter was obtained.

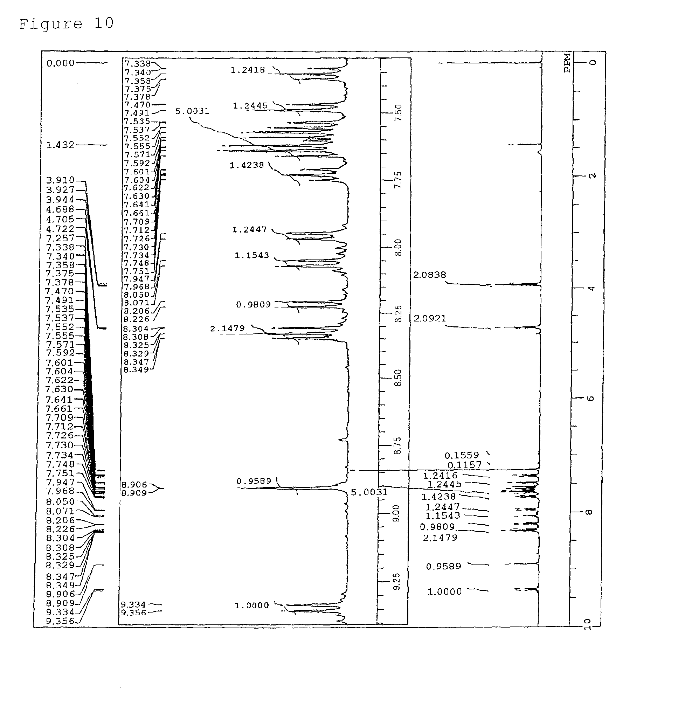

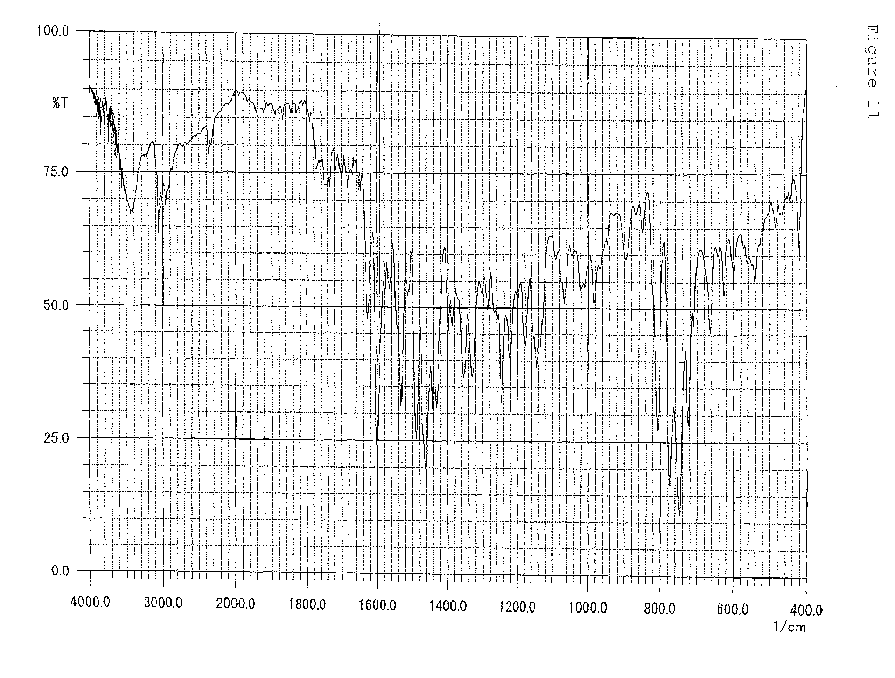

An NMR spectrum chart of the obtained solid matter is shown in Figure 10 and an IR spectrum chart thereof in Figure 11. The obtained was identified as an oxadiazole compound having the structure represented by formula (13).

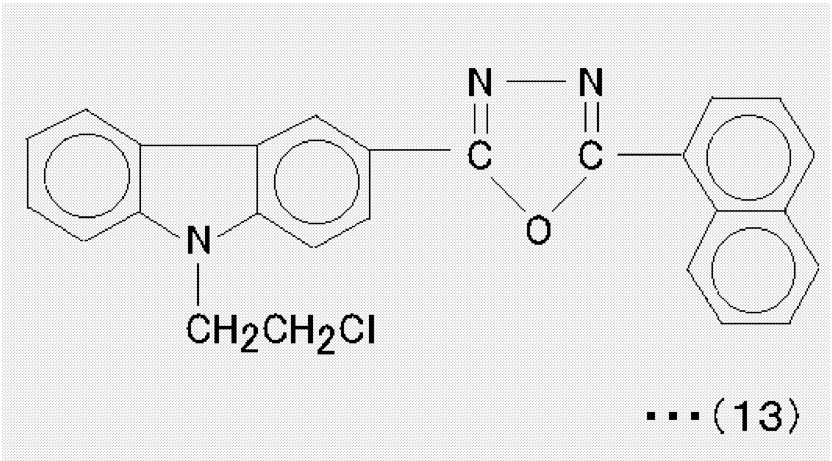

### <Dehydrohalogenation>

In a 300 ml pear-shaped flask were placed 1.7 g of the obtained oxadiazole compound, 2.3 g of potassium hydroxide and 100 ml of ethanol. The solution in the flask was heated in a silicone oil bath for 30 minutes at 80°C. Then, 50 ml of 1,4-dioxane was added to the solution and the resultant solution was heated for 2 hours at 110°C. After the completion of the reaction, the contents in the flask were introduced into ice water, and the resultant mixture was extracted with 400 ml of chloroform. The extract was washed with water and dried. Then a solid matter was obtained. A column, which had been filled with silica gel, was charged with the solid matter, and the solid matter was purified with chloroform as a developer. 0.9 g of a milky white solid matter was obtained.

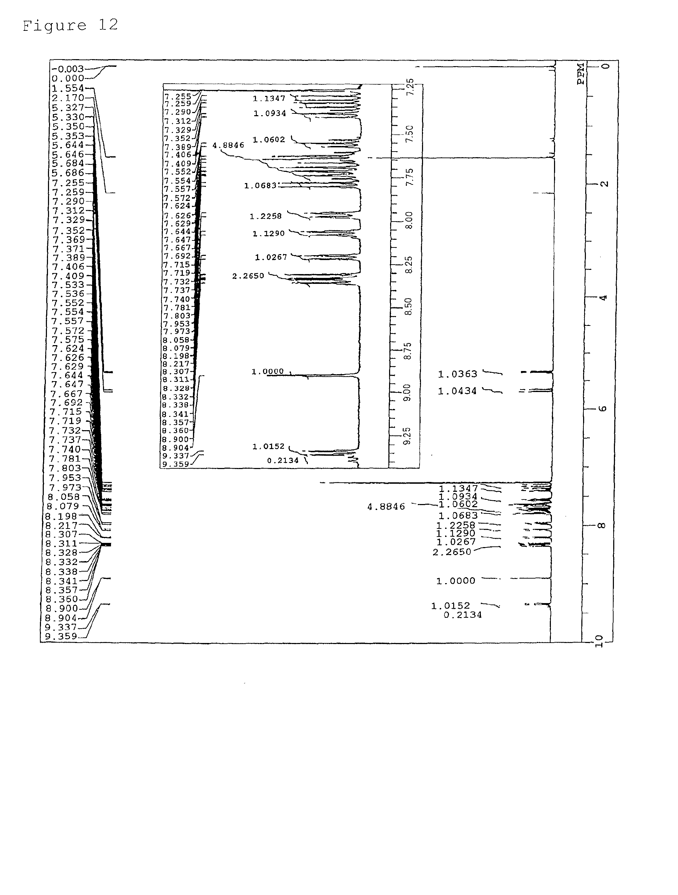

An NMR spectrum chart of the obtained solid matter is shown in Figure 12. The obtained was identified as a vinyl compound having the structure represented by formula (14).

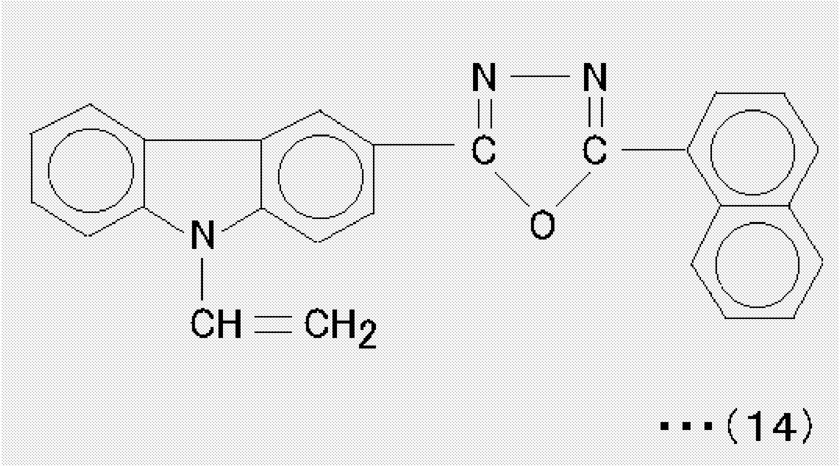

### <Polymerization 1>

Next, the vinyl compound was polymerized using a radical polymerization initiator.

Specifically, 480 mg of the vinyl compound, 10 mg of AIBN and 30 ml of toluene were placed in a polymerizing tube. The solution in the tube was heated in a silicone oil bath for 3 hours at 80°C, and further heated for 15 hours at 100°C. Then, the contents in the tube were cooled naturally and concentrated. The solids thus obtained were purified by the reprecipitation method. 0.19 g of a white solid matter was obtained. The solid matter had a melting range between 260°C and 295°C.

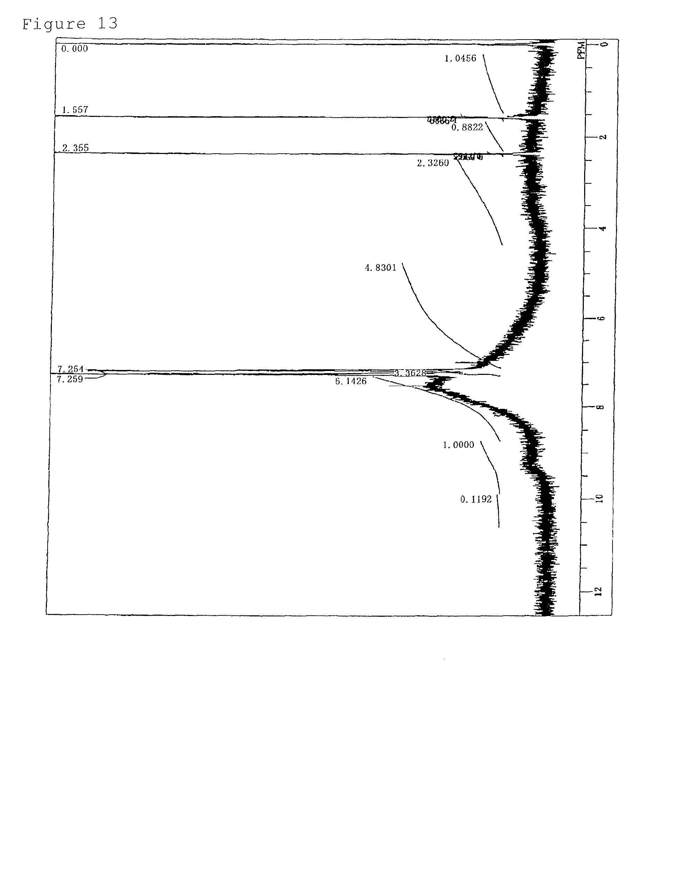

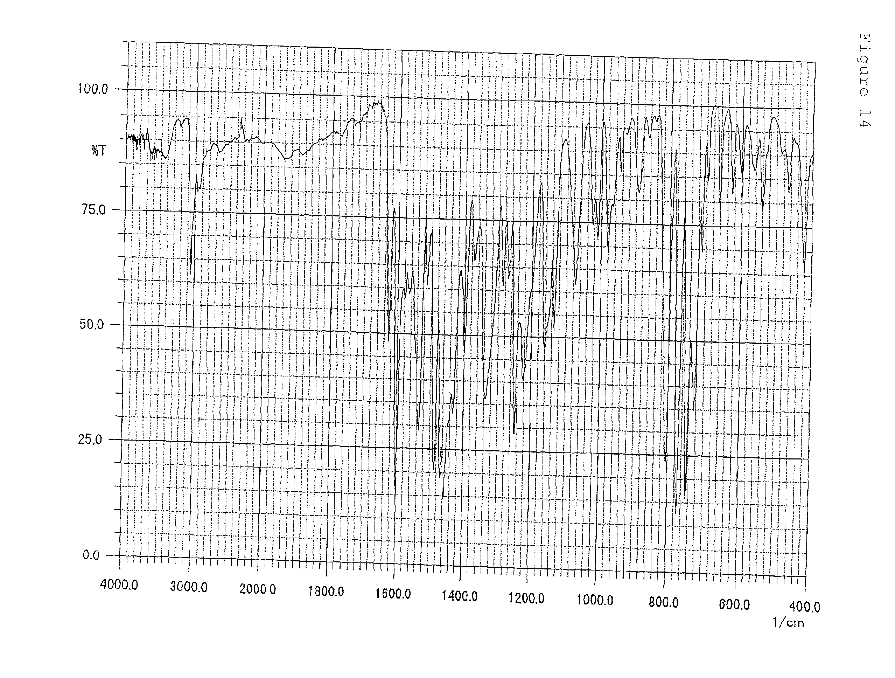

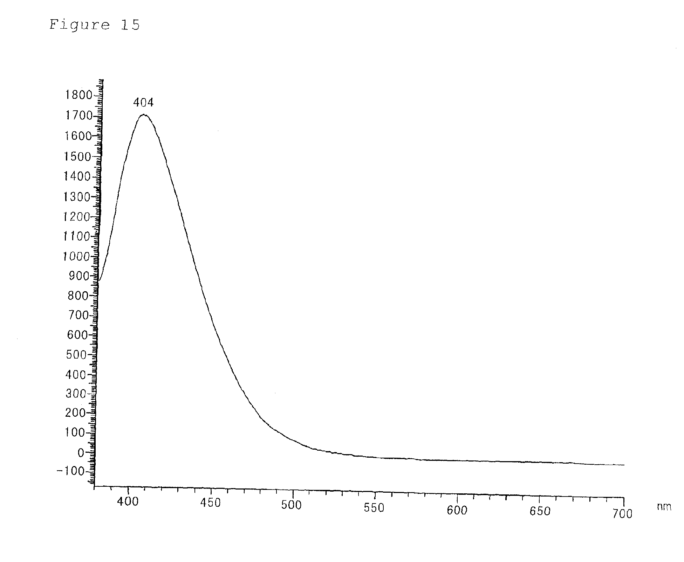

An NMR spectrum chart of the obtained solid matter is shown in Figure 13, an IR chart thereof in Figure 14 and a fluorescence spectrum chart thereof in Figure 15. The obtained solid matter was identified as a blue light-emitting polymer having the repeating unit represented by formula (15).

### <Polymerization 2>

Also, the vinyl compound was polymerized in the presence of a transition-metal catalyst.

Specifically, a 200 ml pear-shaped flask was charged with 0.5 g of the vinyl compound, 60 ml of chlorobenzene and 75 µl of the solution that included tungsten chloride (VI) dissolved in chlorobenzene so that the concentration of tungsten chloride was 0.05 mol/L. The solution in the pear-shaped flask was heated in a silicone oil bath for 12 hours at 200°C. After the termination of the reaction, the resultant contents were concentrated, filtered and dried up. Then yellow powder was obtained. The powder was washed with methanol, dissolved in chloroform and filtered. The filtrate was introduced into methanol, and crystals were precipitated. The crystals were taken out and dried up. 200 mg of a white solid matter was obtained.

The melting point of the white solid matter was measured and it was about 181°C.

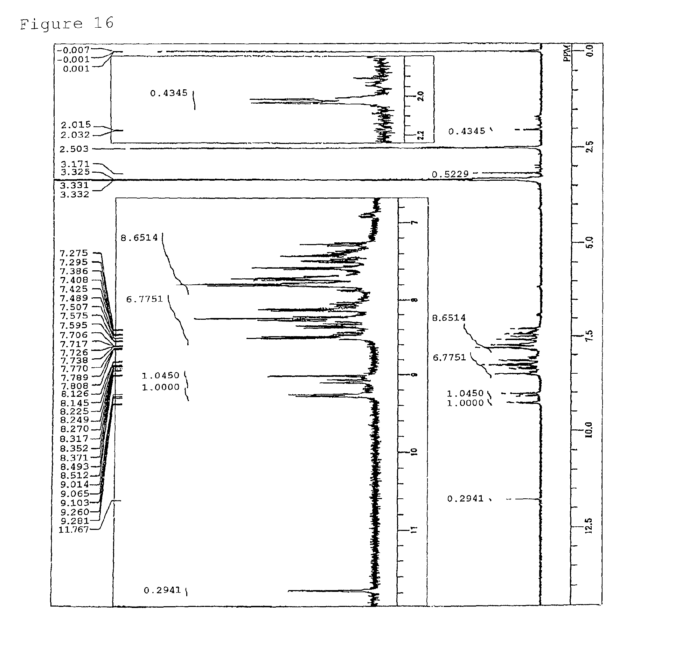

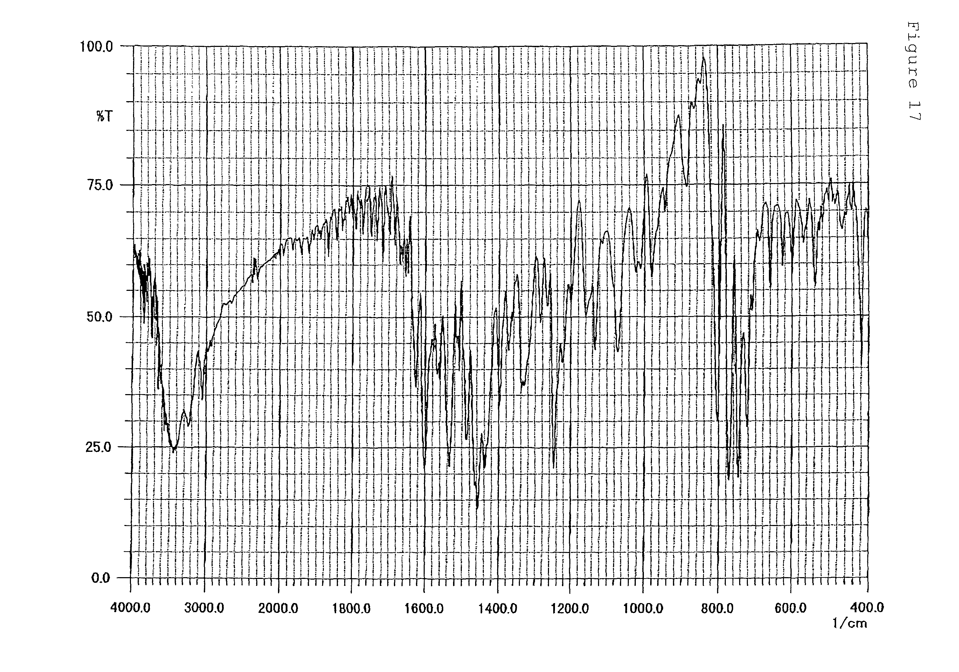

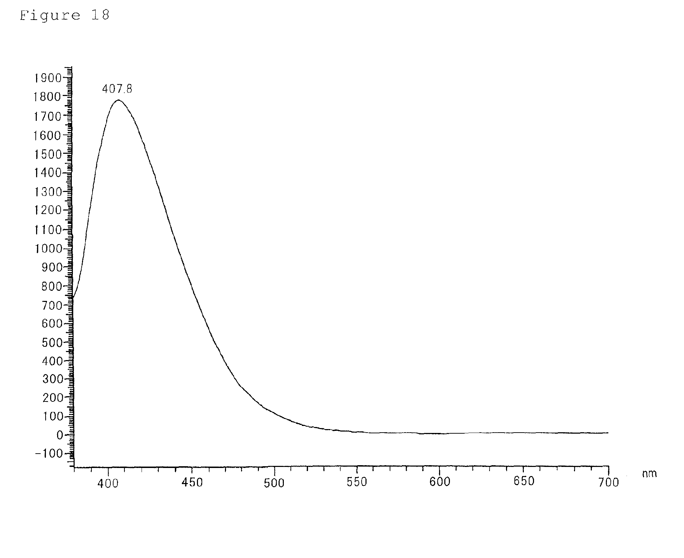

An NMR spectrum chart of the obtained solid matter is shown in Figure 16, an IR chart thereof in Figure 17 and a fluorescence spectrum chart thereof in Figure 18. The obtained solid matter was identified as a blue light-emitting polymer having a repeating unit represented by formula (15).

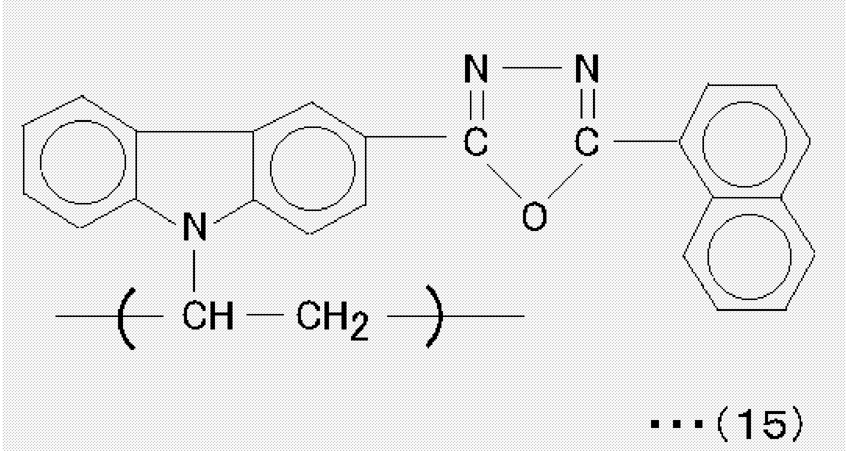

### (Example 2)

### - Synthesis 2: Synthesis of a Blue Light-emitting Polymer -

### <Synthesis of Intermediate>

In a 200 ml three-necked flask were placed 1.5 g of the acid chloride compound represented by the formula (11), 1.3 g of the hydrazide represented by formula (16), 1.1 g of pyridine and 60 ml of THF. The solution in the three-necked flask was heated in a water bath to 60°C and allowed to react for 2 hours. After the completion of the reaction, the solution was cooled with ice. Water was added to the solution, so that solids were precipitated. The solids were collected by filtration and dried. 3.3 g of a solid matter was obtained.

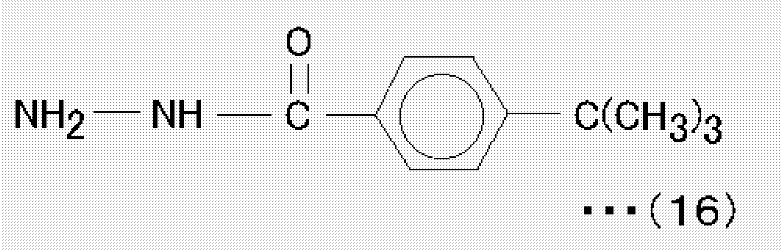

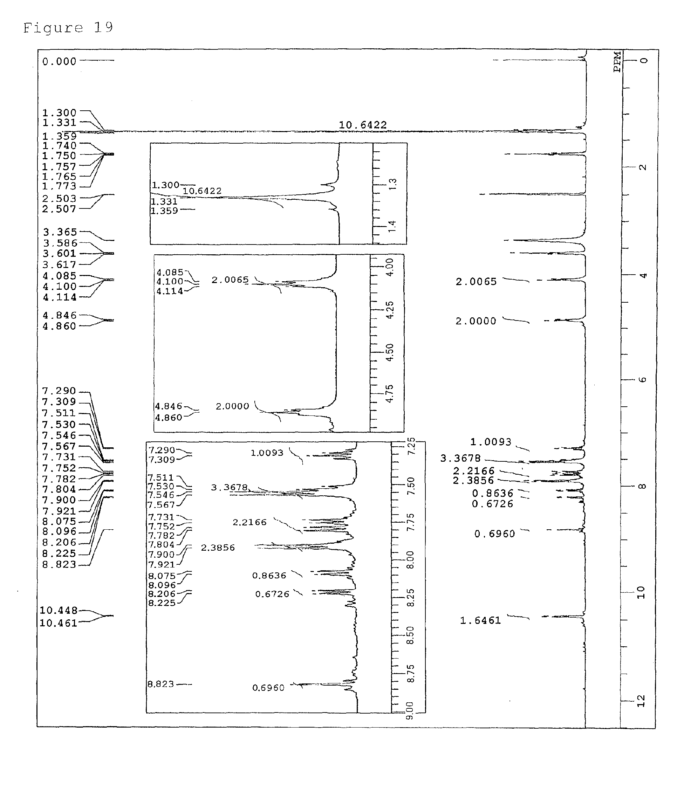

An NMR spectrum chart of the obtained solid matter is shown in Figure 19. The obtained was identified as an intermediate having the structure represented by formula (17).

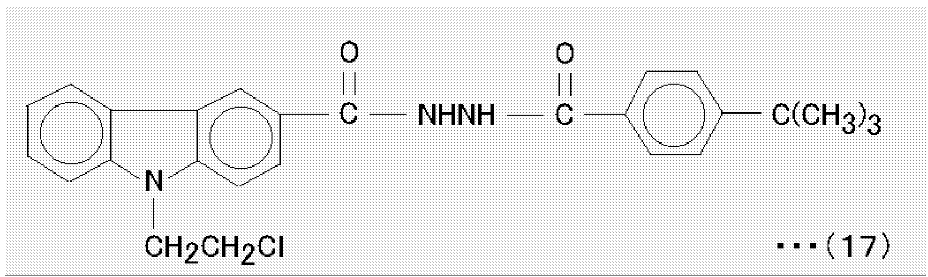

### <Dehydration and Cyclization>

A 200 ml three-necked flask was charged with 3.3 g of the intermediate and 50 ml of phosphoryl chloride. The solution in the three-necked flask was heated in a silicone oil bath to 120°C and allowed to react for 5 hours. After the completion of the reaction, the solvent was removed. The remaining was introduced into ice water, so that solids were precipitated. Then the solids were dissolved in chloroform. The resultant solution was washed with water and concentrated. As a result, a liquid was obtained. A column, which had been filled with silica gel, was charged with the liquid, and the liquid was purified with a 1:1 mixture of chloroform and toluene as a developer. 0.46 g of a yellow solid matter was obtained.

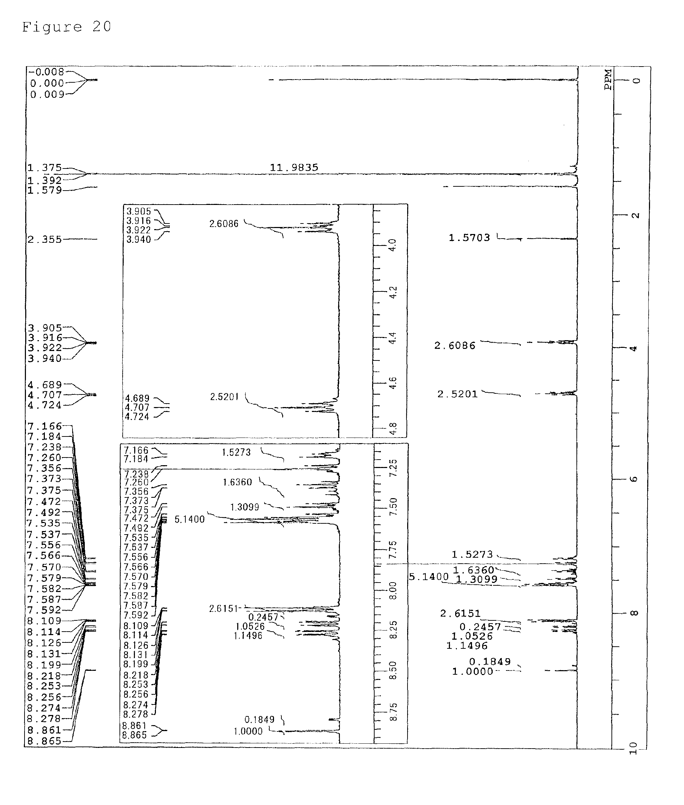

An NMR spectrum chart of the obtained solid matter is shown in Figure 20. The obtained was identified as an oxadiazole compound having the structure represented by formula (18).

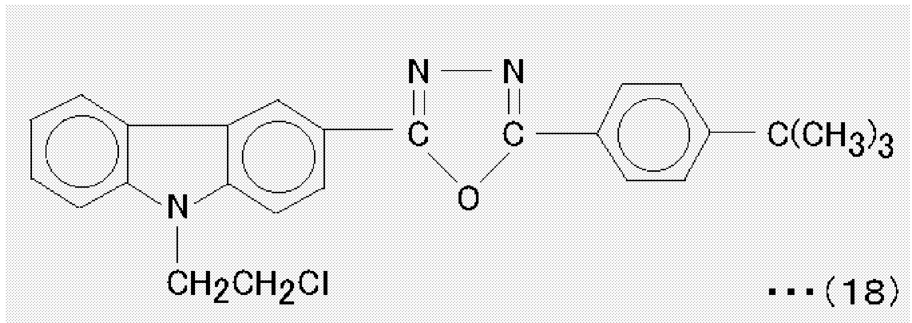

### <Dehydrohalogenation>

In a 50 ml pear-shaped flask were placed 0.46 g of the obtained oxadiazole compound, 0.5 g of potassium hydroxide, 20 ml of ethanol and 10 ml of 1,4-dioxane, The solution in the flask was heated in a silicone oil bath for 30 minutes at 80°C. Then, 50 ml of 1,4-dioxane was added to the solution in the flask and the heating was continued for another 2 hours at 110°C. After the completion of the reaction, the contents in the flask were introduced into ice water. 400 ml of chloroform was added to the resultant mixture, and extraction was carried out. The extract was washed with water and dried. A liquid was obtained. The liquid was dissolved in a 1:1 mixture of toluene and chloroform, and the resultant solution was concentrated and dried up. 0.33 g of a light yellow solid matter was obtained.

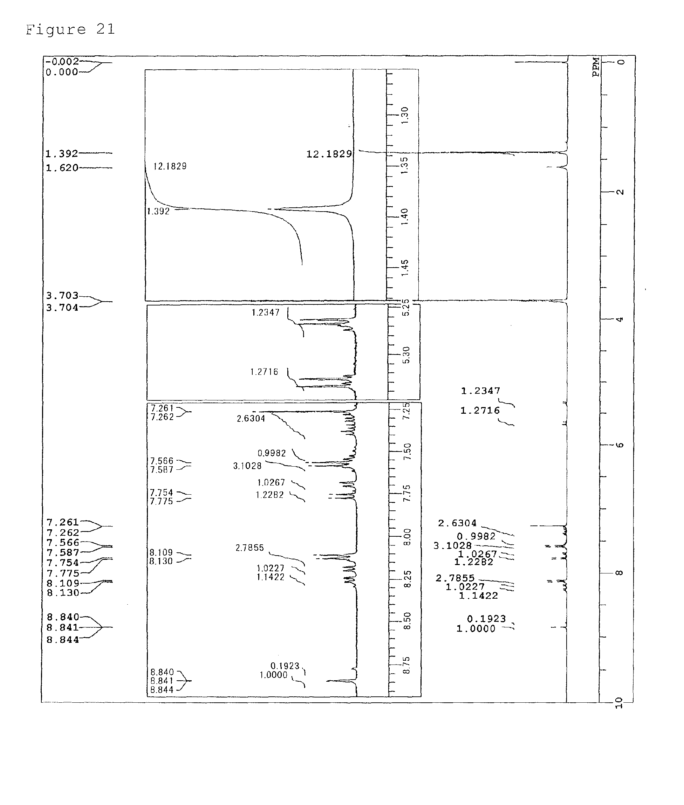

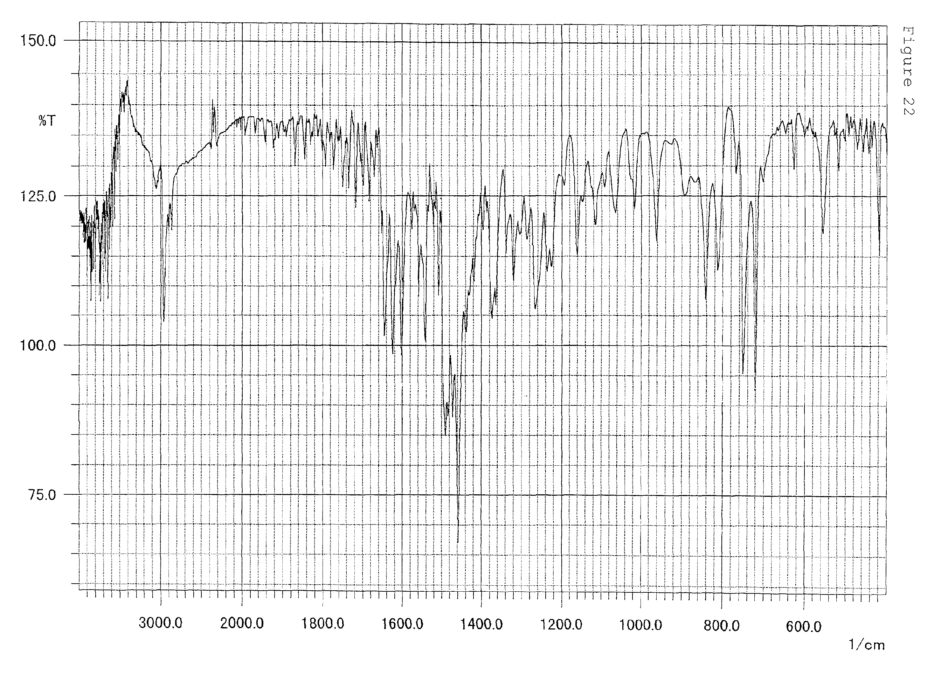

An NMR spectrum chart of the obtained solid matter is shown in Figure 21, and an IR spectrum chart thereof in Figure 22. The obtained was identified as a vinyl compound having the structure represented by formula (19).

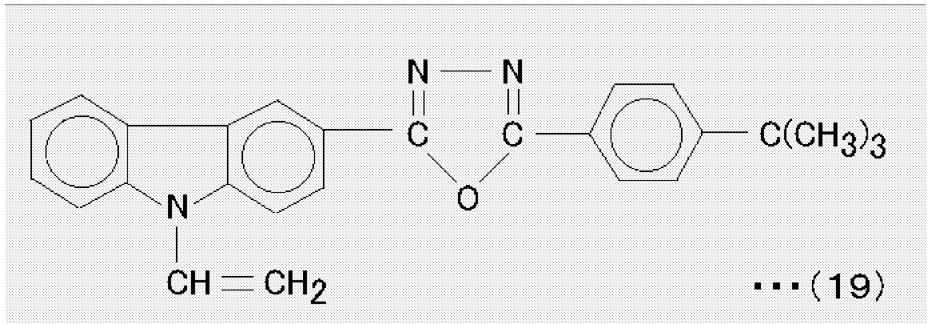

### <Polymerization>

Next, the vinyl compound was polymerized using a radical polymerization initiator.

Specifically, 0.2 g of the vinyl compound, 0.82 mg of AIBN and 15 g of chlorobenzene were placed in a polymerizing tube. The solution in the tube was heated in a silicone oil bath for 20 hours at 85°C. Then, the contents in the tube were cooled naturally. 30 ml of methanol was added to the contents and the resultant was filtered. Light yellow solids were obtained. The obtained solids were dissolved in a mixture of 5 ml of toluene and 20 ml of methanol for reprecipitation and the precipitate was collected by filtration. The solids thus obtained were dried up. As a result, 34 mg of a white solid matter was obtained.

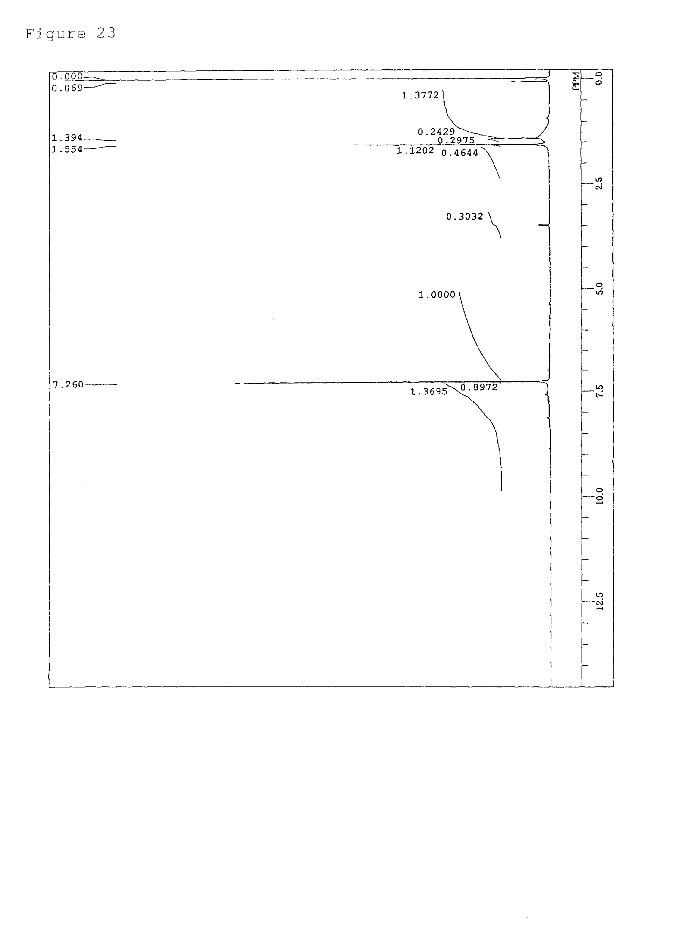

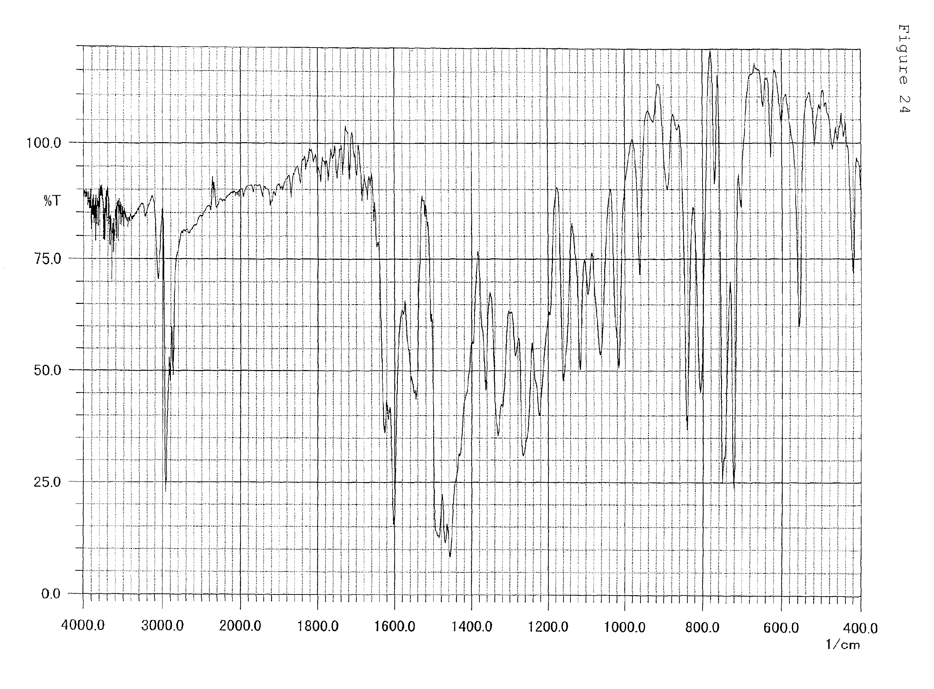

An NMR spectrum chart of the obtained solid matter is shown in Figure 23 and an IR chart thereof in Figure 24. The obtained solid matter was identified as a blue light-emitting polymer having the repeating unit represented by formula (20).

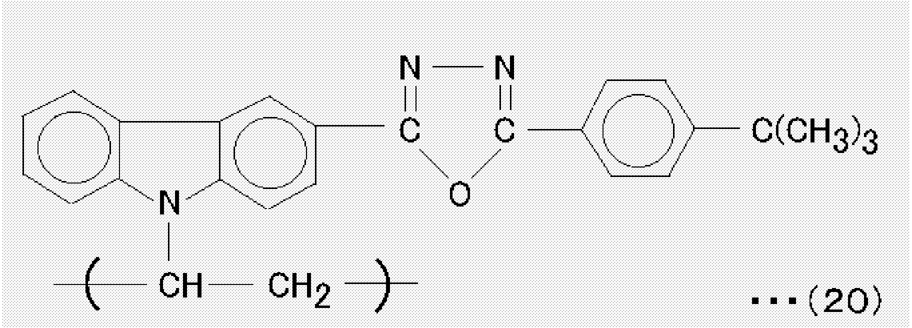

### (Example 3)

### - Synthesis 3: Synthesis of a Blue Light-emitting Polymer -

### <Synthesis of Intermediate>

In a 200 ml three-necked flask were placed 1.5 g of the acid chloride compound represented by the formula (11), 1.9 g of the hydrazide represented by formula (21), 1.1 g of pyridine and 100 ml of THF. The solution in the three-necked flask was heated in a water bath to 60°C and allowed to react for 2 hours. After the completion of the reaction, the solution was cooled with ice. Water was added to the solution, so that solids were precipitated. The solids were collected by filtration and dried. As a result, 3.7 g of a light brown solid matter was obtained.

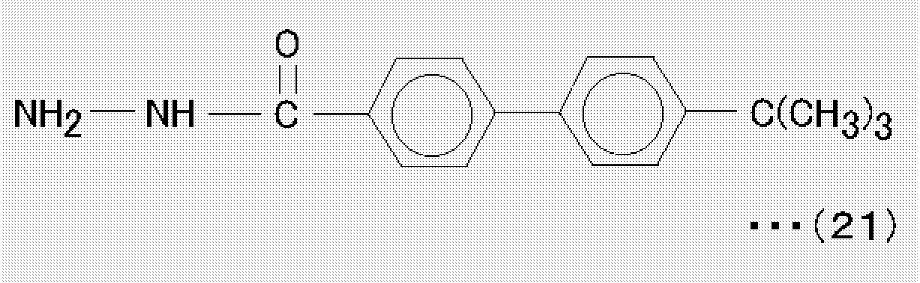

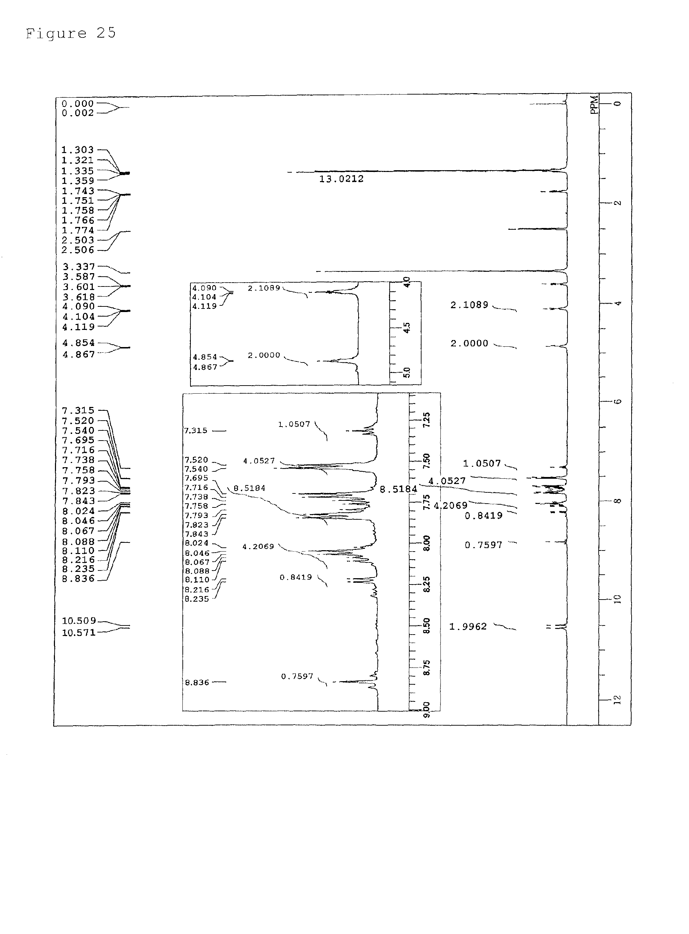

An NMR spectrum chart of the obtained solid matter is shown in Figure 25. The obtained was identified as an intermediate having the structure represented by formula (22).

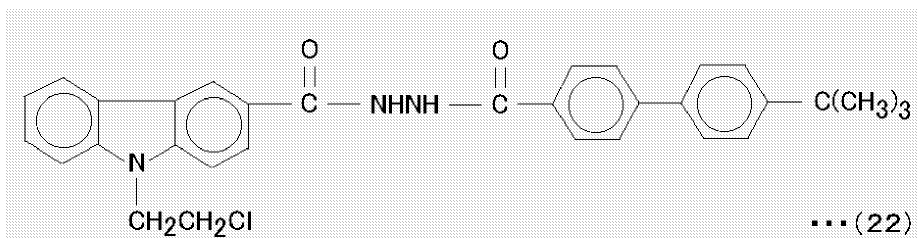

### <Dehydration and Cyclization>

A 200 ml three-necked flask was charged with 3.7 g of the intermediate and 50 ml of phosphoryl chloride. The solution in the three-necked flask was heated in a silicone oil bath to 120°C and allowed to react for 15 hours. After the completion of the reaction, the solvent was removed. The remaining was introduced into ice water, so that solids were precipitated. Then the solids were dissolved in chloroform. The resultant solution was washed with water, concentrated and dried up. As a result, 1 g of a light yellow solid matter was obtained.

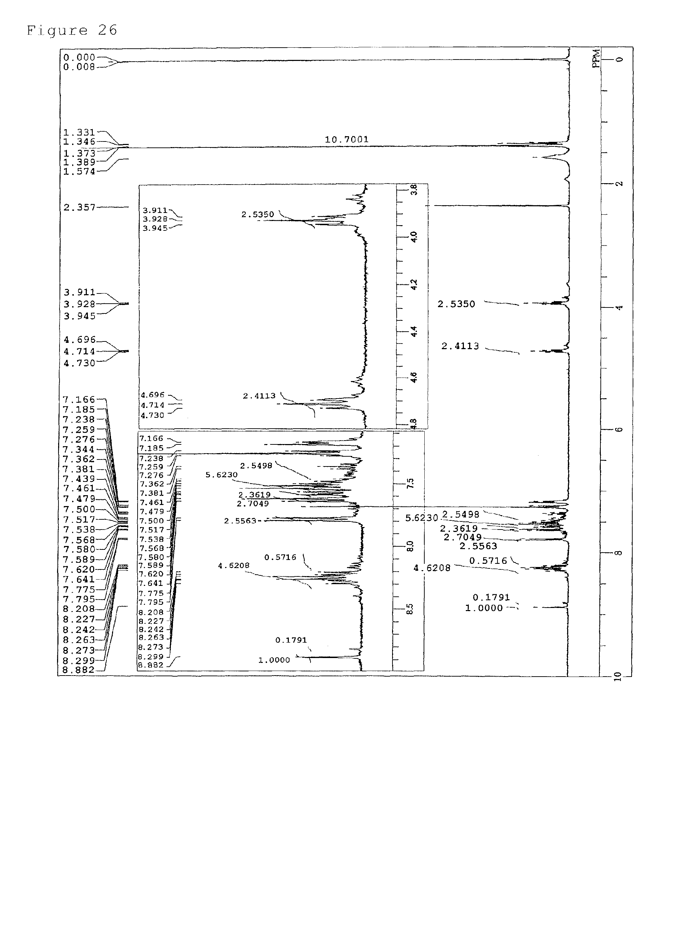

An NMR spectrum chart of the obtained solid matter is shown in Figure 26. The obtained was identified as an oxadiazole compound having the structure represented by formula (23).

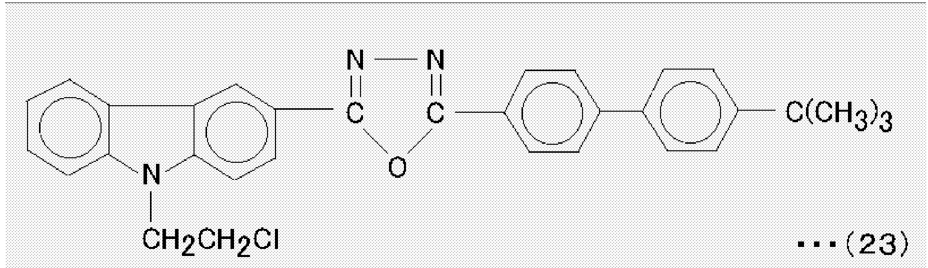

### <Dehydrohalogenation>

In a 200 ml pear-shaped flask were placed 1.0 g of the obtained oxadiazole compound, 1.0 g of potassium hydroxide, 40 ml of ethanol and 20 ml of 1,4-dioxane. The solution in the flask was heated in a silicone oil bath for 30 minutes at 80°C. Then, 50 ml of 1,4-dioxane was added to the solution and the heating was continued for another 2 hours at 110°C. After the completion of the reaction, the contents in the flask were introduced into ice water. 400 ml of chloroform was added to the resultant mixture, and extraction was carried out. The extract was washed with water and dried. Solids were obtained. A column, which had been filled with silica gel, was charged with the solids, and they were purified with chloroform as a developer. As a result, 0.77 g of a light yellow solid matter was obtained.

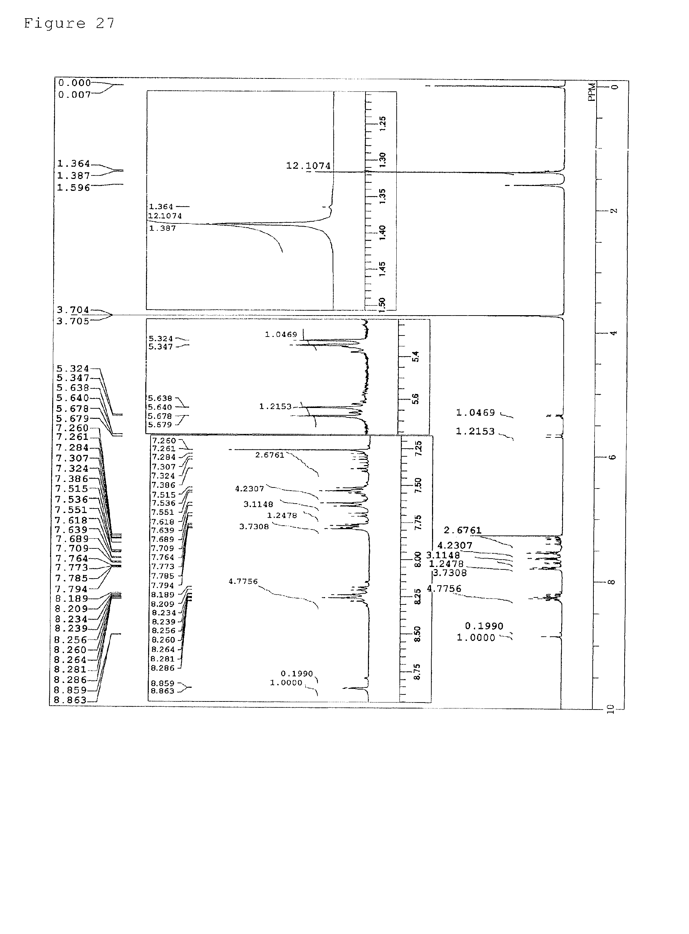

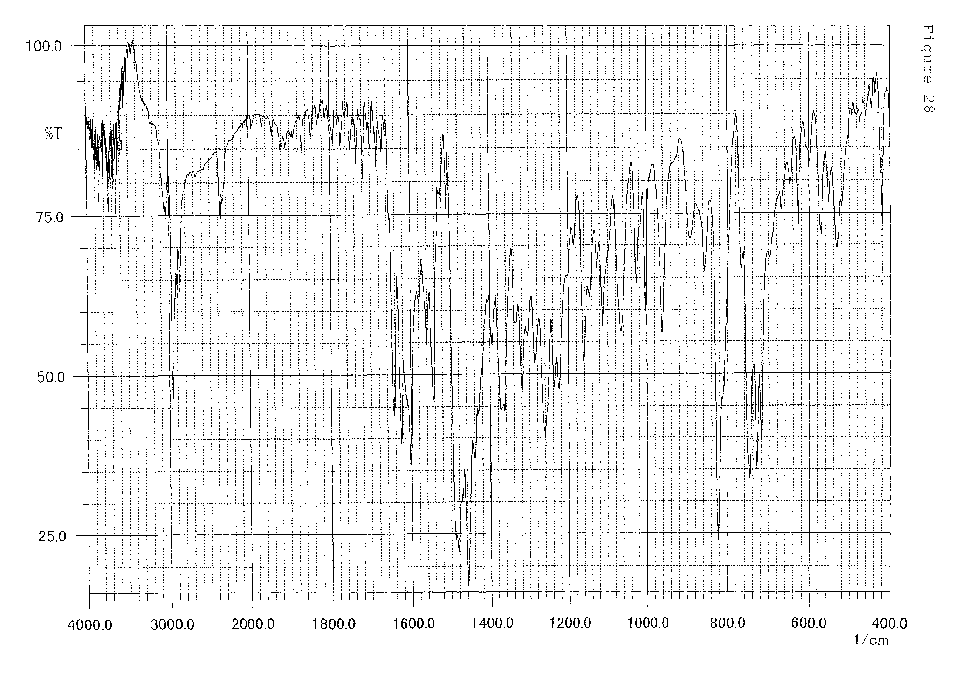

An NMR spectrum chart of the obtained solid matter is shown in Figure 27, and an IR spectrum chart thereof in Figure 28. The obtained was identified as a vinyl compound having the structure represented by formula (24).

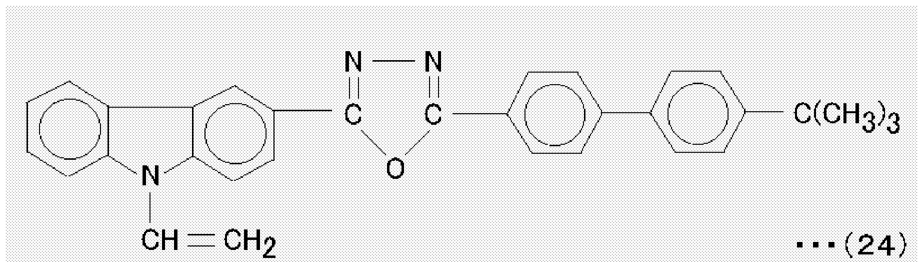

### <Polymerization>

Next, the vinyl compound was polymerized using a radical polymerization initiator.

Specifically, 0.2 g of the vinyl compound, 1.35 mg of AIBN and 15 g of chlorobenzene were placed in a polymerizing tube. The solution in the tube was heated in a silicone oil bath for 3 hours at 80°C, and further heated for 15 hours at 100°C. Then, the contents in the tube were cooled naturally and concentrated. The solids thus obtained were purified by the reprecipitation method. As a result, 6 mg of a white solid matter was obtained.

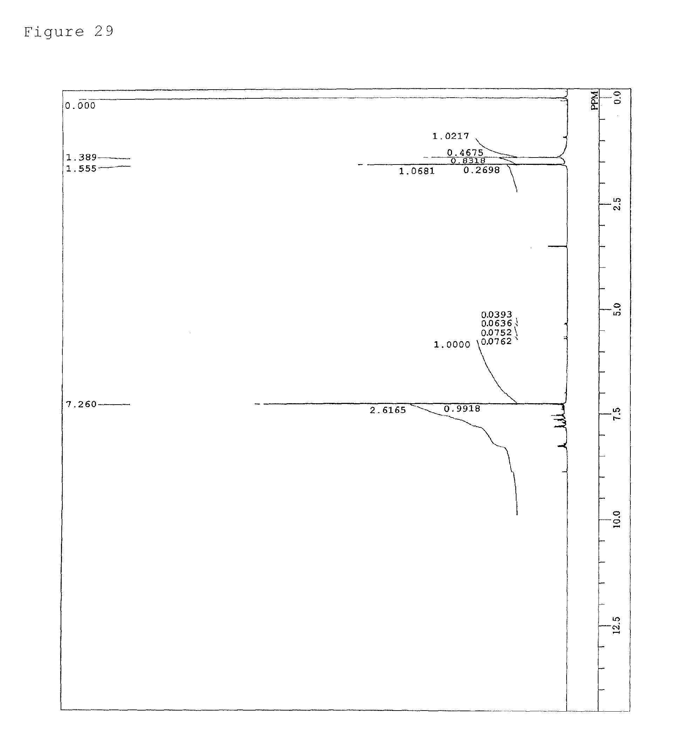

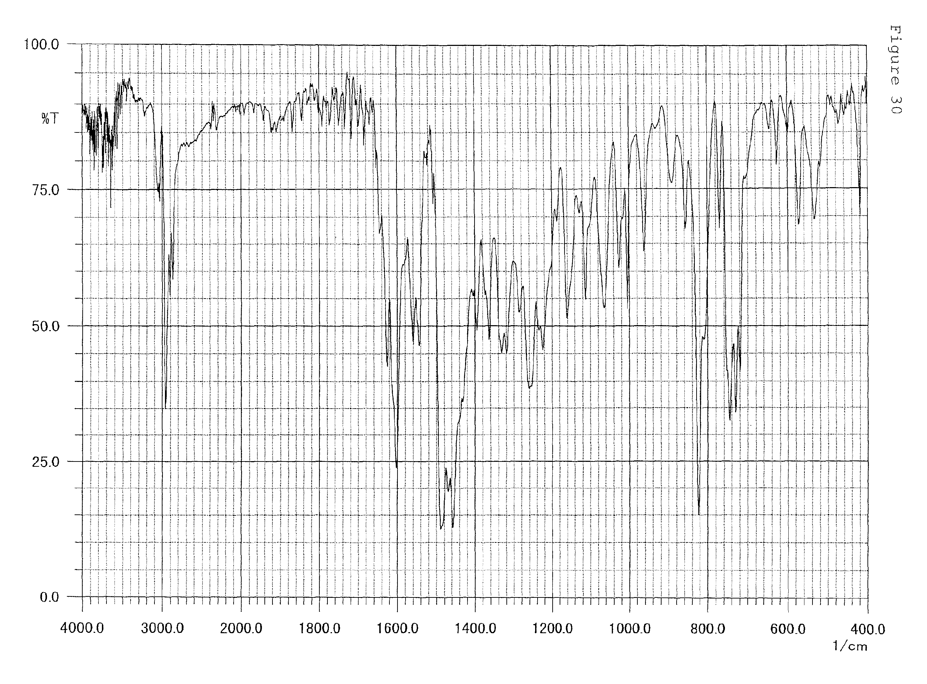

An NMR spectrum chart of the obtained solid matter is shown in Figure 29 and an IR chart thereof in Figure 30. The obtained solid matter was identified as a blue light-emitting polymer having the repeating unit represented by formula (25).

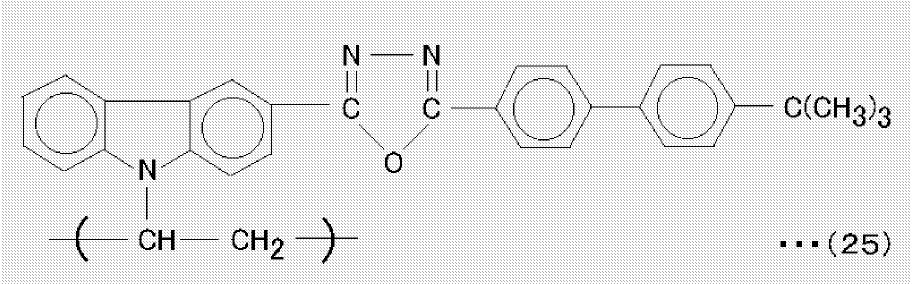

### (Example 4)

### - Synthesis 4: Synthesis of a Blue Light-emitting Polymer -

### <Synthesis of Intermediate>

In a 200 ml three-necked flask were placed 2.25 g of the acid chloride compound represented by the formula (11), 2.0 g of the hydrazide represented by formula (26), 0.73 g of pyridine and 165 ml of THF. The solution in the three-necked flask was heated in a water bath to 60°C and allowed to react for 2 hours. After the completion of the reaction, the solution was cooled with ice. Water was added to the cooled solution, so that solids were precipitated. The solids were collected by filtration and dried. 3.4 g of a solid matter was obtained.

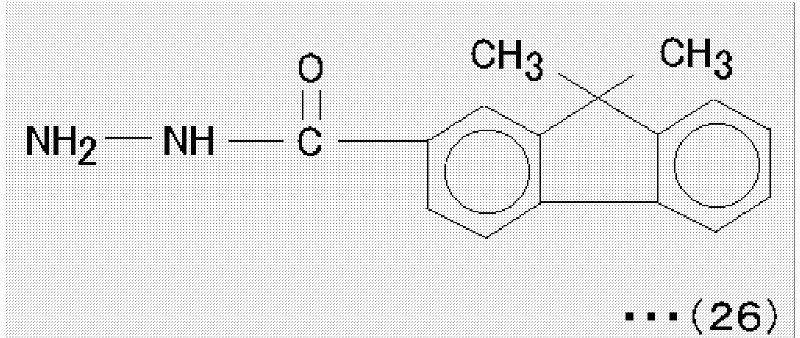

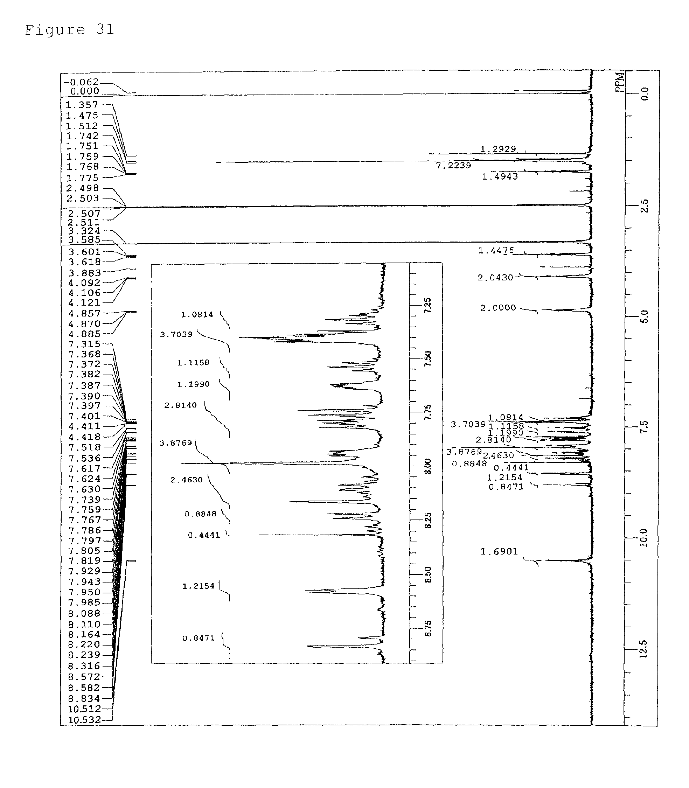

An NMR spectrum chart of the obtained solid matter is shown in Figure 31. The obtained was identified as an intermediate having the structure represented by formula (27).

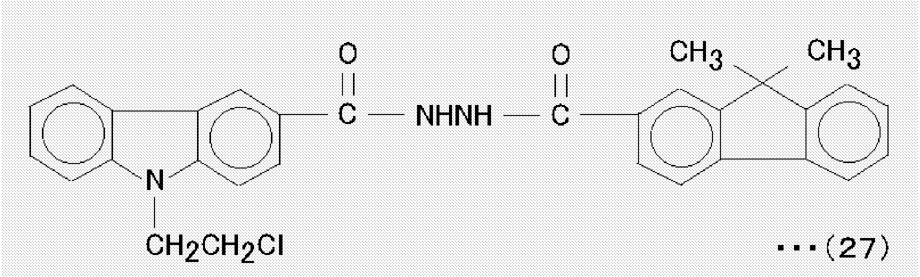

### <Dehydration and Cyclization>

A 200 ml three-necked flask was charged with 3.3 g of the intermediate and 80 ml of phosphoryl chloride. The solution in the three-necked flask was heated in a silicone oil bath to 120°C and allowed to react for 5 hours. After the completion of the reaction, the solvent was removed. The remaining was introduced into ice water, so that solids were precipitated. Then the solids were dissolved in chloroform. The resultant solution was washed with water, concentrated and dried up. As a result, 2.2 g of a yellow solid matter was obtained.

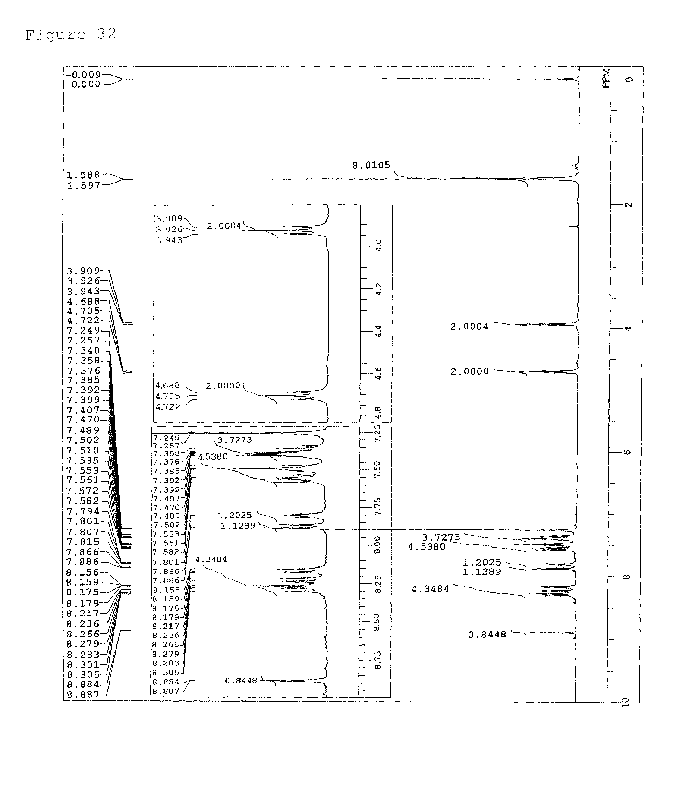

An NMR spectrum chart of the obtained solid matter is shown in Figure 32. The obtained was identified as an oxadiazole compound having the structure represented by formula (28).

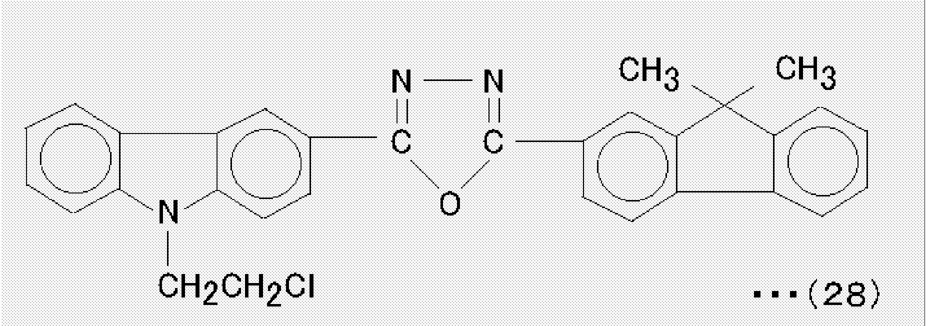

### <Dehydrohalogenation>

In a 300 ml pear-shaped flask were placed 2.2 g of the obtained oxadiazole compound, 2.2 g of potassium hydroxide, 30 ml of ethanol and 35 ml of 1,4-dioxane. The solution in the flask was heated in a silicone oil bath for 30 minutes at 80°C. Then, 50 ml of 1,4-dioxane was added to the solution and the heating was continued for another 2 hours at 110°C. After the completion of the reaction, the contents in the flask were introduced into ice water. 400 ml of chloroform was added to the resultant mixture, and extraction was carried out. The extract was washed with water and dried. Solids were obtained. A column, which had been filled with silica gel, was charged with the solids, and they were purified with chloroform as a developer. As a result, 1.1 g of an orange solid matter was obtained.

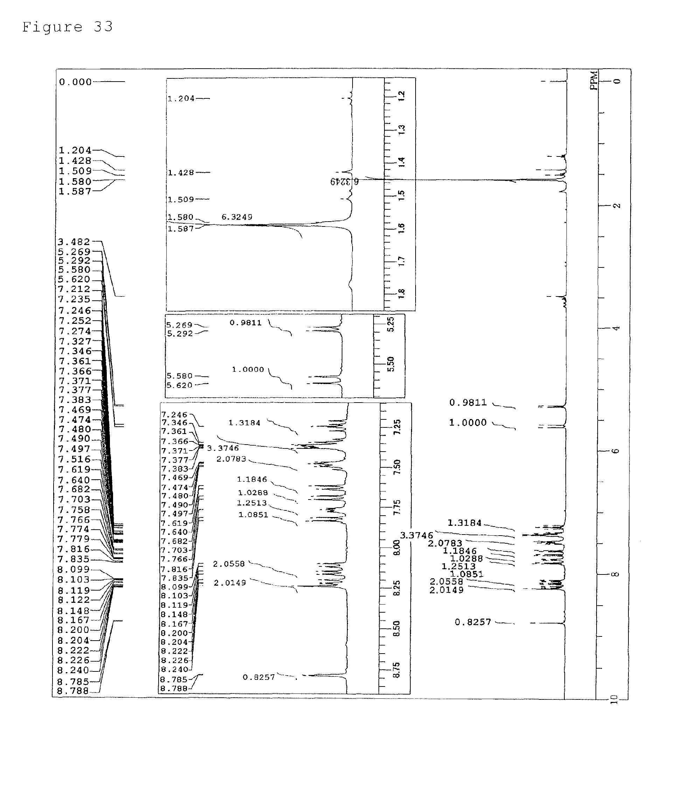

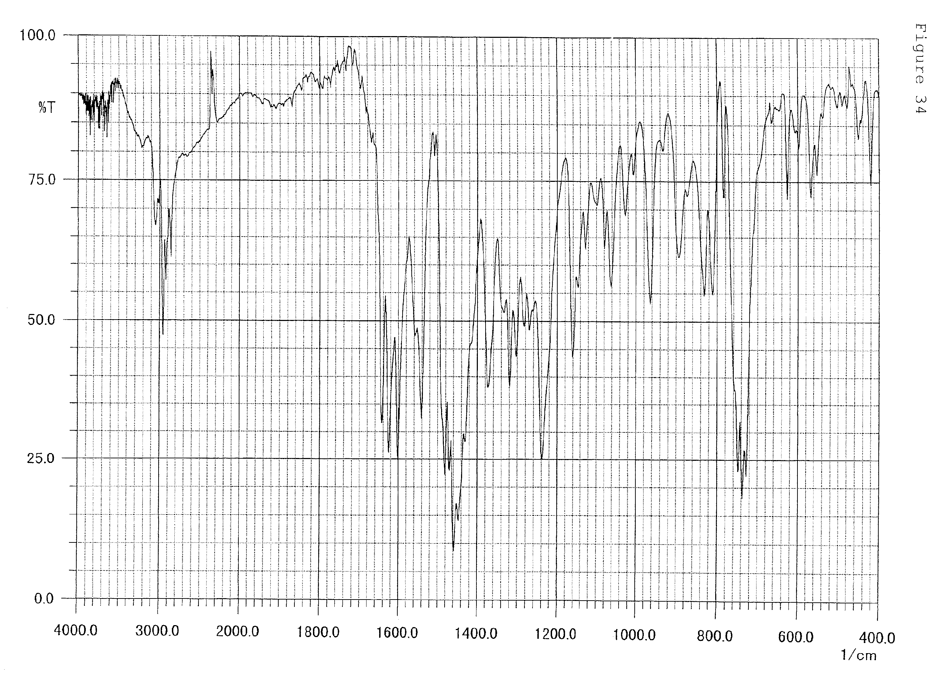

An NMR spectrum chart of the obtained solid matter is shown in Figure 33, and an IR spectrum chart thereof in Figure 34. The obtained was identified as a vinyl compound having the structure represented by formula (29).

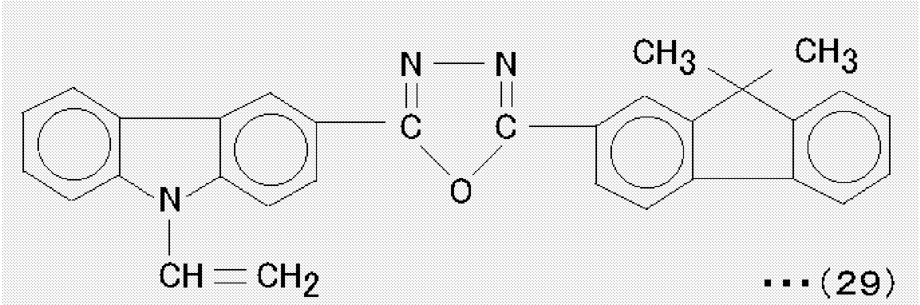

### <Polymerization>

Next, the vinyl compound was polymerized using a radical polymerization initiator.

Specifically, 0.2 g of the vinyl compound, 1.4 mg of AIBN and 15 g of chlorobenzene were placed in a polymerizing tube. The solution in the tube was heated in a silicone oil bath for 3 hours at 80°C, and further heated for 15 hours at 100°C. Then, the contents in the tube were cooled naturally and concentrated. The solids thus obtained were purified by the reprecipitation method. As a result, 0.1 g of a white solid matter was obtained.

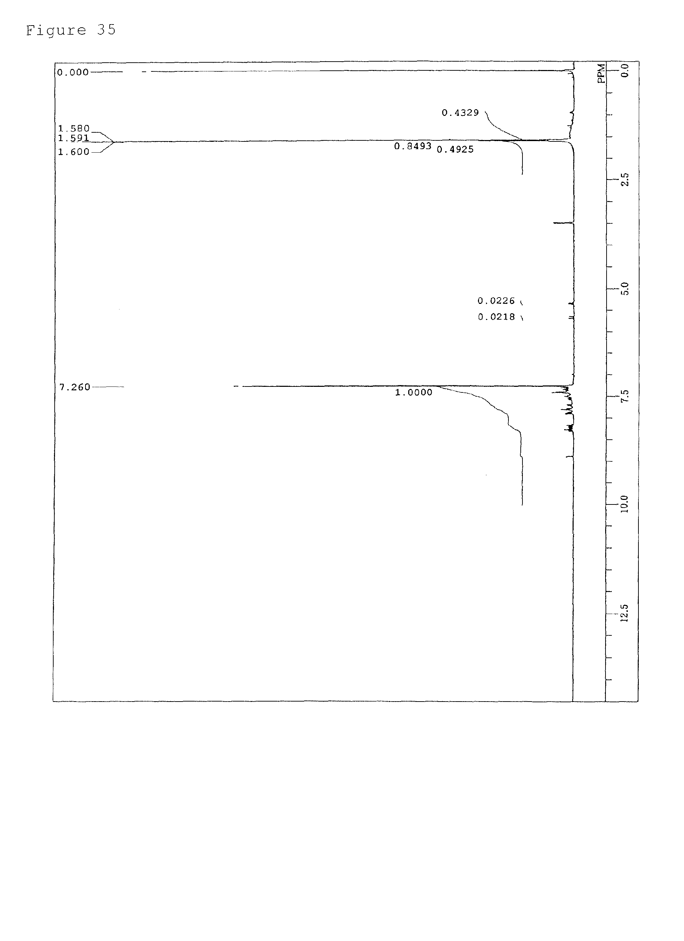

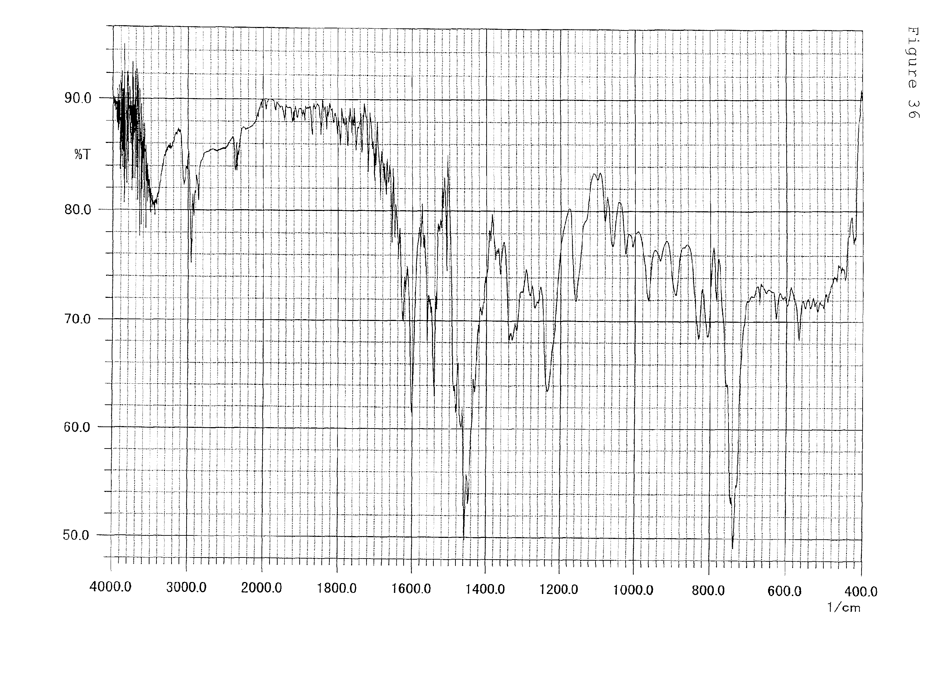

An NMR spectrum chart of the obtained solid matter is shown in Figure 35 and an IR chart thereof in Figure 36. The obtained solid matter was identified as a blue light-emitting polymer having the repeating unit represented by formula (30).

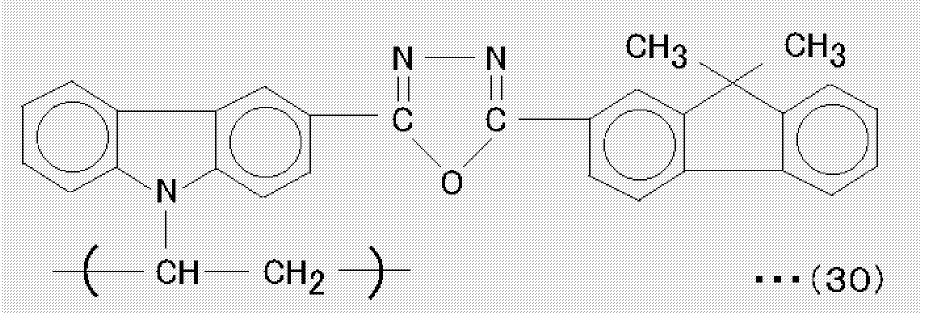

### (Example 5)

### - Synthesis 5: Synthesis of a Blue Light-emitting Polymer -

### <Alkylation>

In a 300 ml three-necked flask were placed 6 g of 1-chloromethyl-2,4-dimethylnaphthalene, 5.6 g of N-(2-chloro- ethyl)carbazole, which is represented by the formula (8), 1.9 g of zinc and 100 ml of ortho-dichlorobenzene. The solution in the three-necked flask was heated in a silicone oil bath to 155°C and allowed to react for 2 hours. After the completion of the reaction, the resultant solution was introduced into ice water. Then, chloroform was added to the resultant mixture and extraction was carried out. Chloroform was distilled away from the extract and the remaining was dried up. 6.6 g of a reddish brown paste was obtained.

From an NMR spectrum chart and an IR spectrum chart of the paste, the obtained was identified as an organic halogen compound having the structure represented by formula (46).

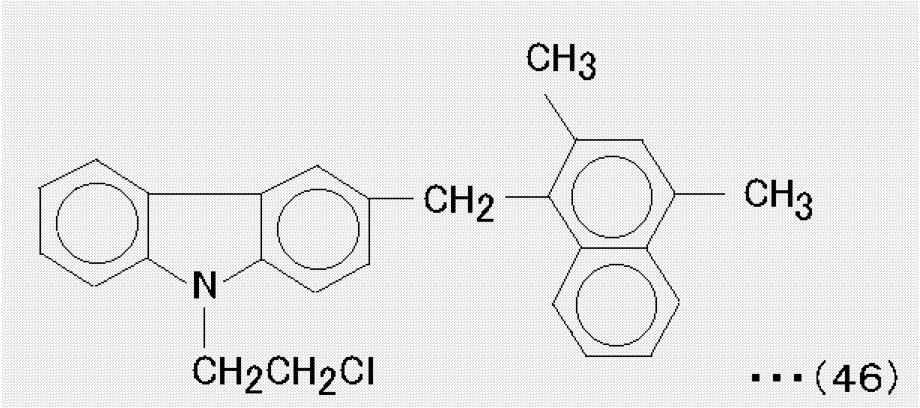

### <Dehydrohalogenation>

In a 300 ml pear-shaped flask were placed 5.9 g of the organic halogen compound represented by the formula (46), 3.0 g of potassium hydroxide, 80 ml of ethanol and 120 ml of 1,4-dioxane. The solution in the flask was heated in a silicone oil bath to 140°C and allowed to react for 14 hours. After the completion of the reaction, the contents in the flask were concentrated so that the volume of the concentrate was 1/3 time as large as that of the contents just after the completion of the reaction. Then, the concentrate was introduced into ice water, and extraction was carried out with chloroform. The extract was washed with water and dried. A solid matter was obtained. A column, which had been filled with silica gel, was charged with the solid matter, and the solid matter was purified with chloroform as a developer. As a result, a paste was obtained.

From an NMR spectrum chart and an IR spectrum chart of the paste, the obtained was identified as a vinyl compound having the structure represented by formula (47).

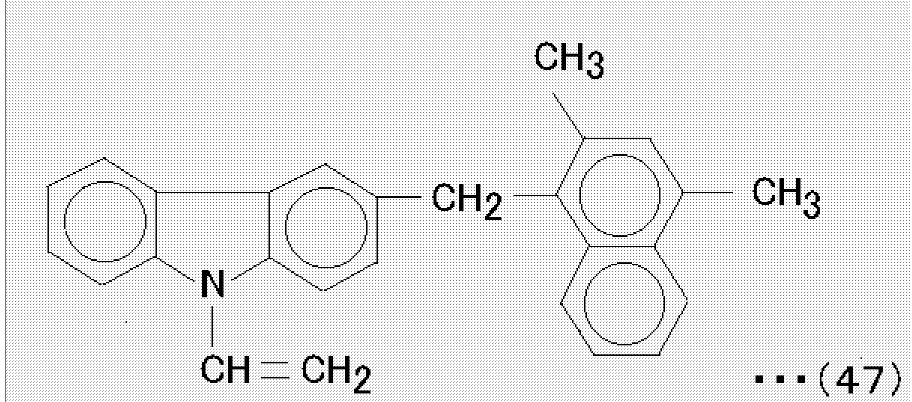

### <Polymerization>

15 g of the vinyl compound, 13.6 mg of AIBN and 10 ml of chlorobenzene were placed in a polymerizing tube. The solution in the tube was heated in a silicone oil bath to 85°C and allowed to react for 20 hours. Then, the contents in the tube were cooled naturally, concentrated and filtered. Precipitates were obtained. The precipitates were dissolved in chloroform, active carbon was added thereto and the resultant mixture was heated and then filtered. The filtrate was dripped in 200 ml of methanol, and the resultant was filtered. The precipitate thus obtained was dried to a solid matter.

From an NMR spectrum chart and an IR spectrum chart of the solid matter, the obtained was identified as a blue light-emitting polymer having the repeating unit represented by formula (48).

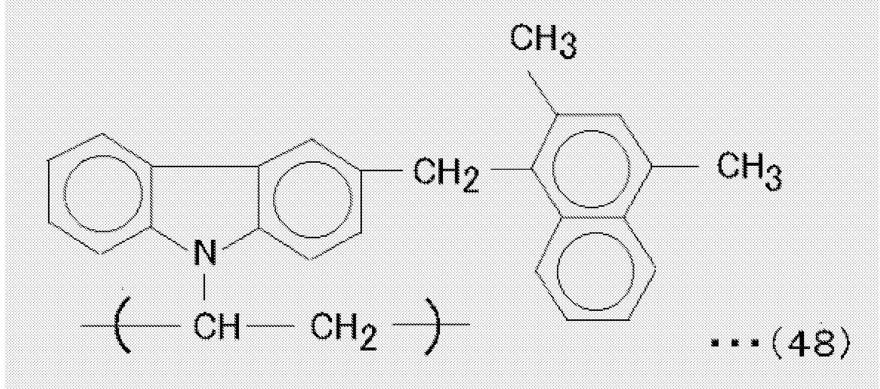

### (Example 6)

### - Synthesis 6: Synthesis of a Blue Light-emitting Polymer -

### <Alkylation>

In a 300 ml three-necked flask were placed 8 g of N-(2-chloroethyl)carbazole, which is represented by the formula (8), 2.7 g of zinc and 50 ml of ortho-dichlorobenzene. The solution in the three-necked flask was heated in a silicone oil bath to 150°C and allowed to react for 2 hours while the solution prepared by dissolving 6.2 g of 1-trimethyl naphthalene in 30 ml of ortho-dichlorobenzene was being dripped in the solution. After the completion of the reaction, the resultant solution was cooled with ice. The solution was introduced into ice water. To the resultant mixture was added sodium hydroxide, so that the mixture turned alkaline. Then, 200 ml of chloroform was added and extraction was carried out. Moisture was removed by adding 80 g of sodium sulfate and chloroform was distilled away. 12.7 g of a light yellowish green paste was obtained. A column, which had been filled with silica gel, was charged with the paste, and the paste was purified with toluene as a developer. As a result, 10.3 g of a light yellowish green paste was obtained.

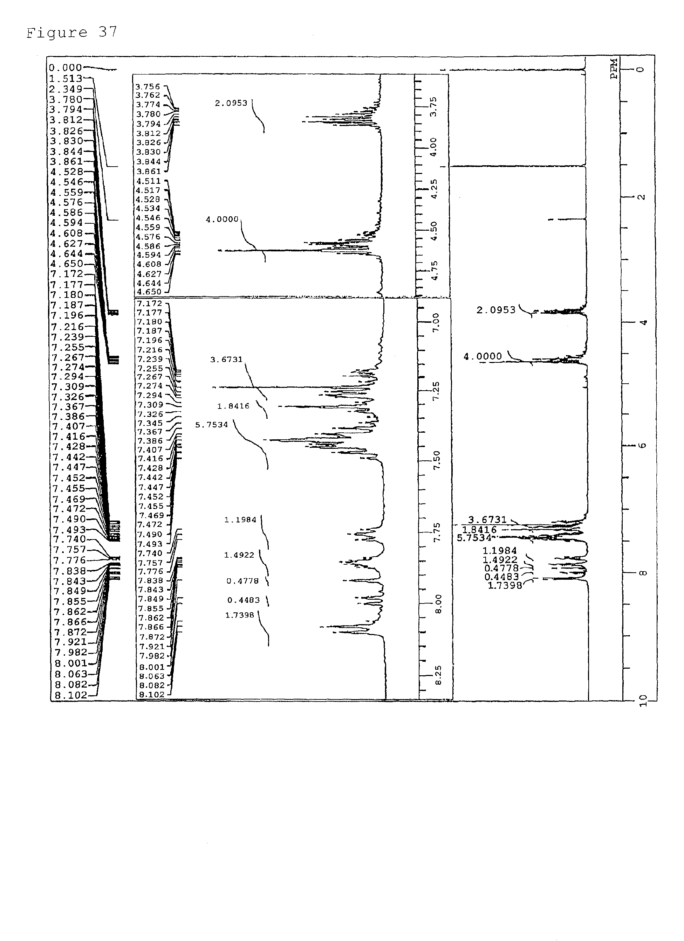

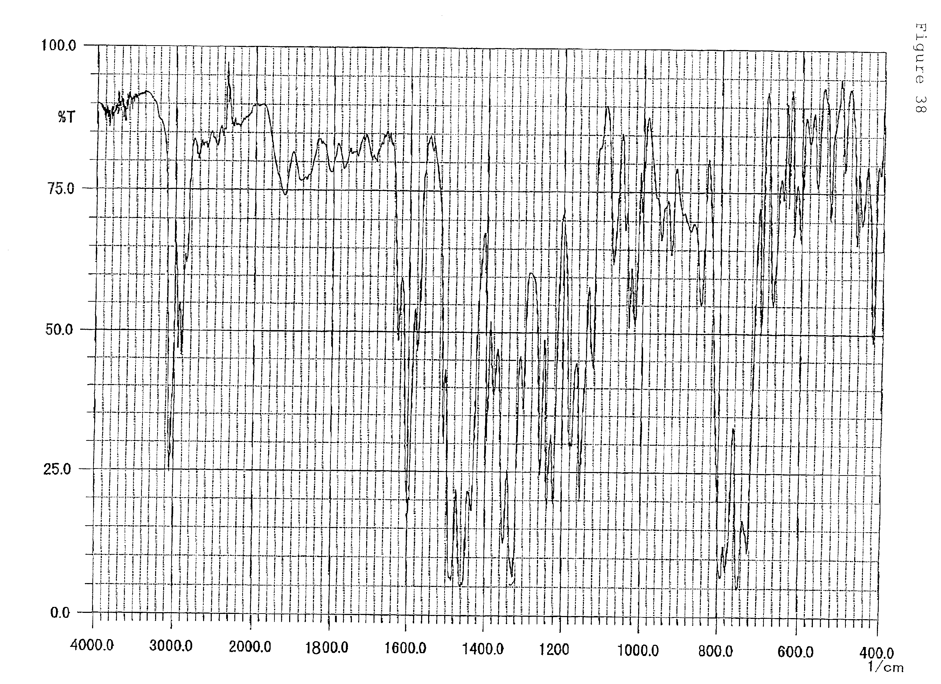

An NMR spectrum chart and an IR spectrum chart of the paste are shown in Figures 37 and 38 respectively. The obtained was identified as an organic halogen compound having the structure represented by formula (50).

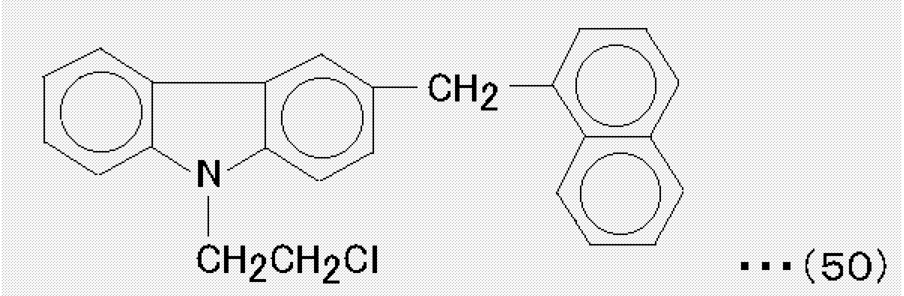

### <Dehydrohalogenation>

In a 300 ml pear-shaped flask were placed 5.8 g of the organic halogen compound represented by the formula (50), 4 g of potassium hydroxide, 60 ml of ethanol and 100 ml of 1,4-dioxane. The solution in the flask was heated in a silicone oil bath at 100°C and allowed to react for 2 hours. After the completion of the reaction, the resultant solution was concentrated. After the concentrate was introduced into ice water, extraction was carried out with 200 ml of chloroform. The extract was washed with water and dried up to a paste. A column, which had been filled with silica gel, was charged with the paste, and the paste was purified with toluene as a developer. As a result, 4.5 g of a light yellowish green paste was obtained.

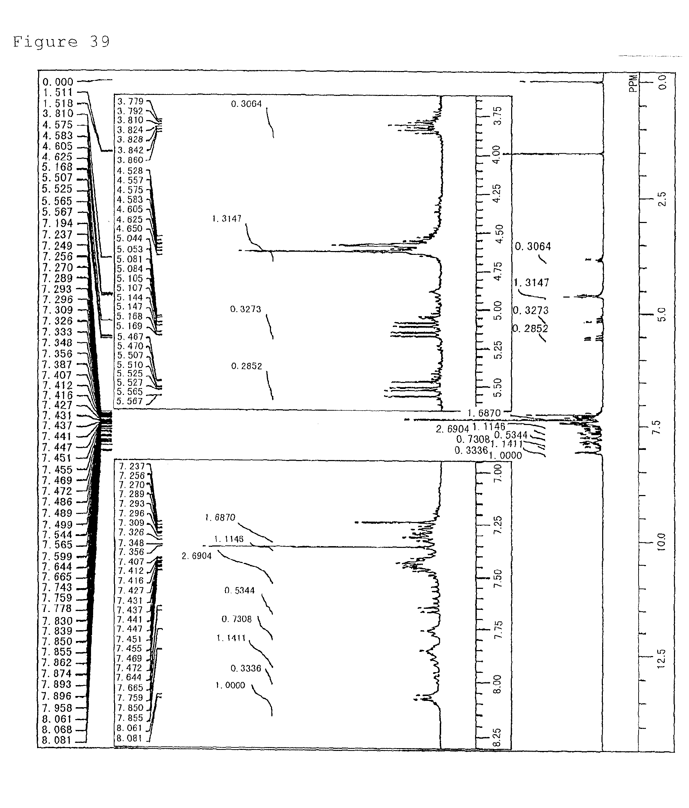

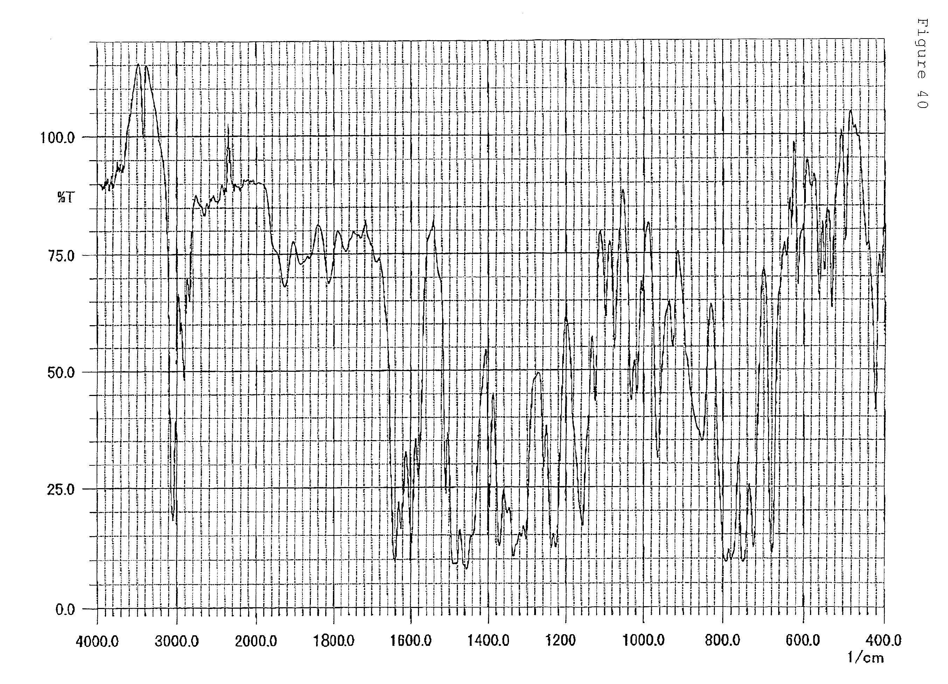

An NMR spectrum chart and an IR spectrum chart of the light yellowish green paste are shown in Figures 39 and 40 respectively. The obtained was identified as a vinyl compound having the structure represented by formula (51).

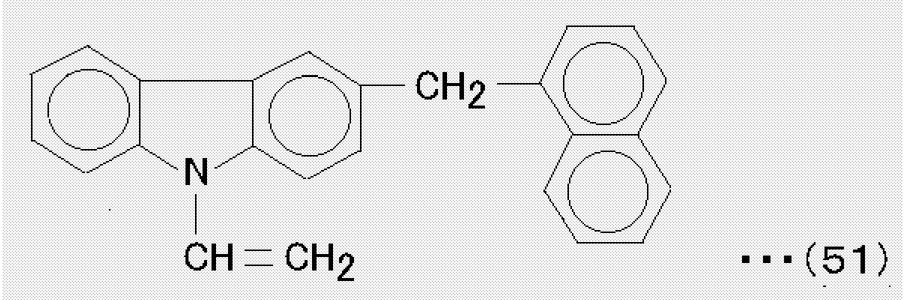

### <Polymerization>

2 g of the vinyl compound, 10 mg of AIBN and 10 ml of chlorobenzene were placed in a polymerizing tube. The solution in the tube was heated in a silicone oil bath to 85°C and allowed to react for 20 hours. After the completion of the reaction, the contents in the tube were cooled naturally, and concentrated. The concentrate was dripped in 200 ml of methanol and the resultant was filtered. Precipitates were obtained. The precipitates were dissolved in chloroform, and the solution was filtered. The filtrate thus obtained was dripped in 200 ml of methanol, and the resultant was filtered again. The collected precipitates were dried up. 0.45 g of a white solid matter was obtained.

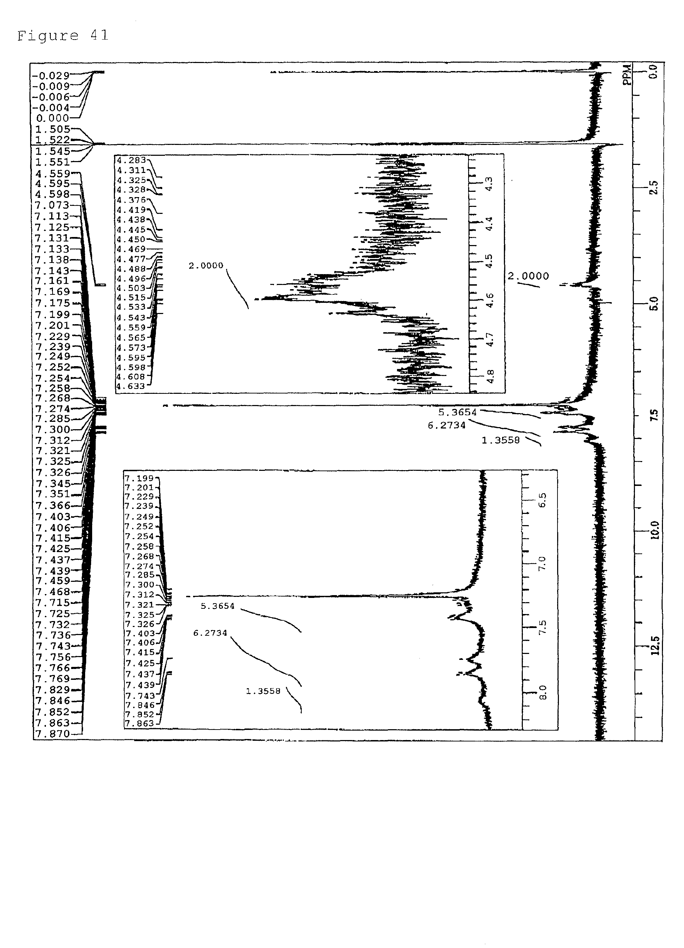

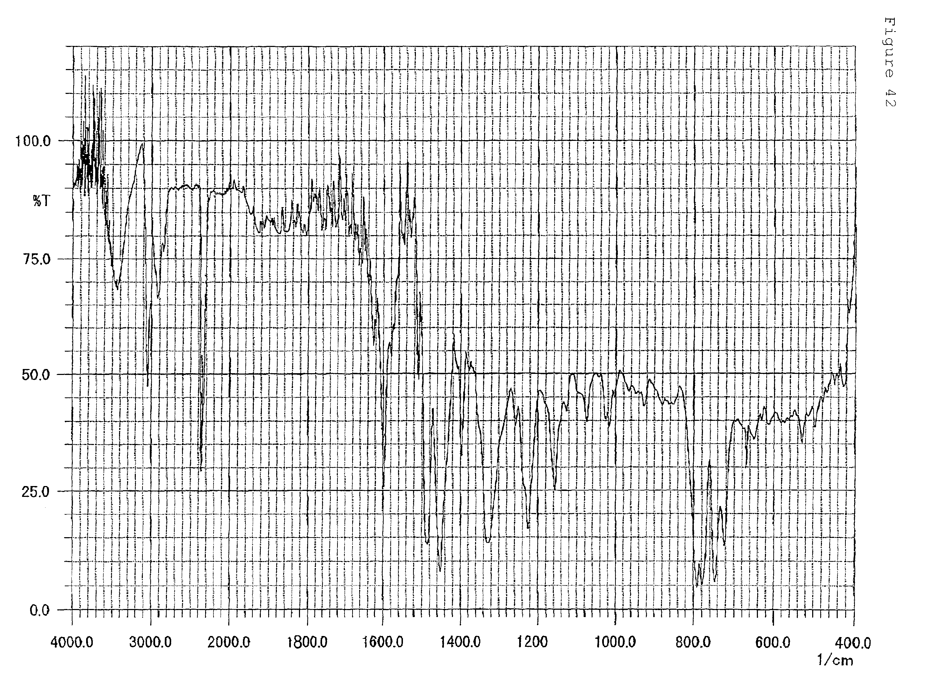

An NMR spectrum chart and an IR spectrum chart of the solid matter are shown in Figures 41 and 42 respectively. The obtained was identified as a blue light-emitting polymer having the repeating unit represented by formula (52).

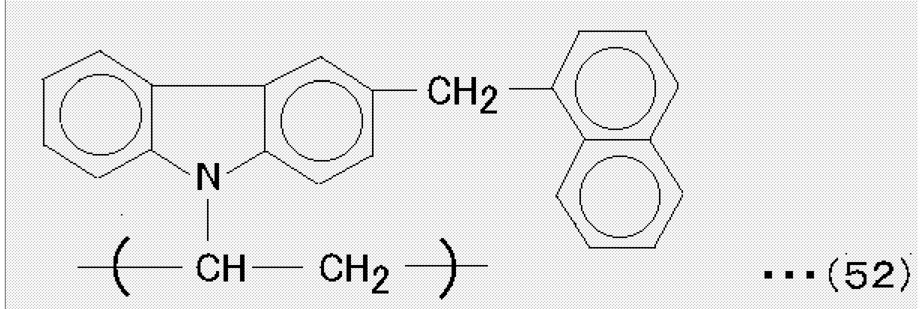

### (Example 7)

### - Synthesis 7: Synthesis of a Blue Light-emitting Polymer -

### <Alkylation>

In a 300 ml three-necked flask were placed 18.6 g of N-(2-chloroethyl)carbazole, which is represented by the formula (8), 6.3 g of zinc and 100 ml of ortho-dichlorobenzene. The solution in the three-necked flask was heated in a silicone oil bath to 150°C and allowed to react for 2 hours while the solution prepared by dissolving 12.5 g of 2,4-dimethylbenzyl chloride in 40 ml of ortho-dichlorobenzene was being dripped in the solution. After the completion of the reaction, the resultant mixture was introduced into ice water, to which sodium hydroxide was added, so that the mixture turned alkaline. Then, 300 ml of chloroform was added and extraction was carried out. Moisture was removed by adding 100 g of sodium sulfate and chloroform was distilled off. 28.2 g of a paste was obtained. A column, which had been filled with silica gel, was charged with the paste, and the paste was purified with toluene as a developer. As a result, 22 g of a greenish brown paste was obtained.

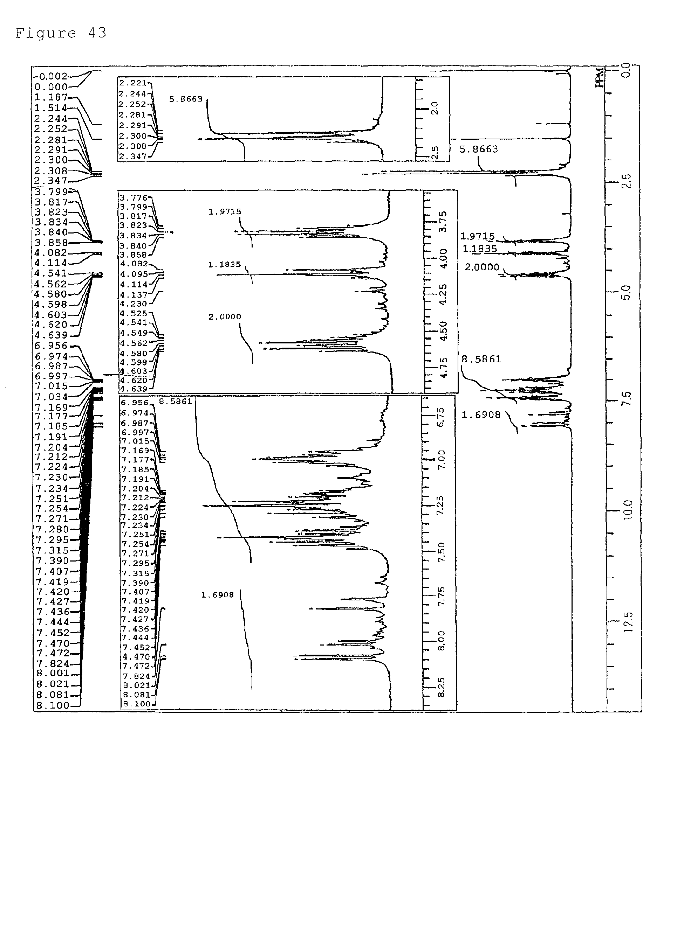

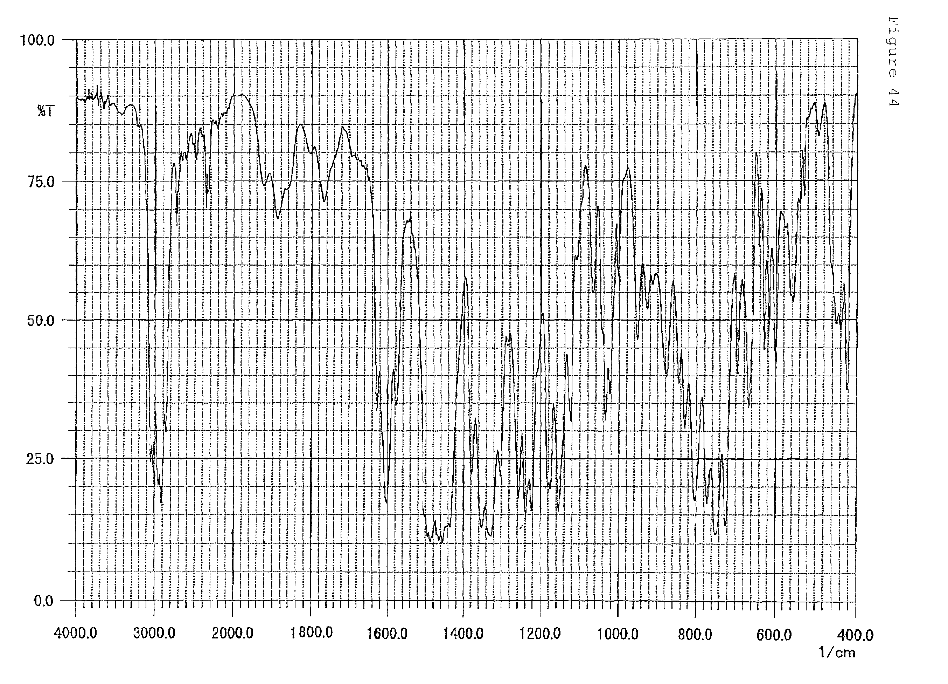

An NMR spectrum chart and an IR spectrum chart of the paste are shown in Figures 43 and 44 respectively. The obtained was identified as an organic halogen compound having the structure represented by formula (54).

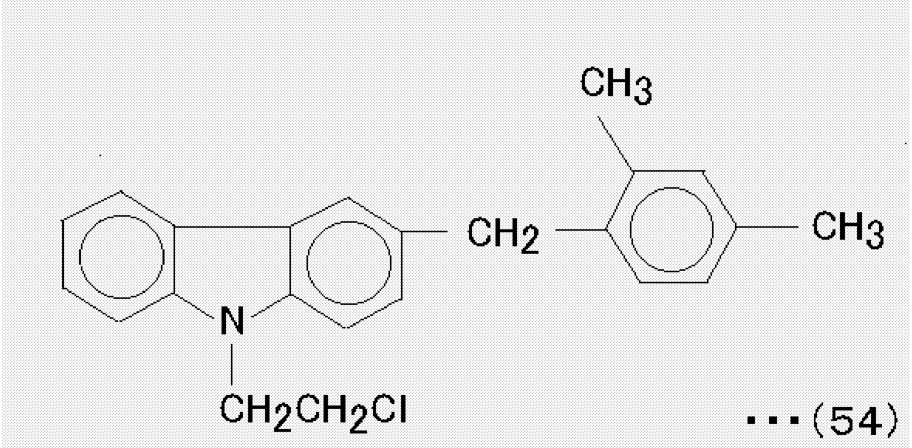

### <Dehydrohalogenation>

In a 300 ml pear-shaped flask were placed 22 g of the organic halogen compound represented by the formula (54), 11 g of potassium hydroxide, 80 ml of ethanol and 120 ml of 1,4-dioxane. The solution in the flask was heated in a silicone oil bath to 100°C and allowed to react for 2.5 hours. After the completion of the reaction, the resultant solution was concentrated. After the concentrate was dripped in ice water, extraction was carried out with 300 ml of chloroform. The extract was washed with water and dried up to a paste. A column, which had been filled with silica gel, was charged with the paste, and the paste was purified with toluene as a developer. As a result, 11 g of a greenish brown paste was obtained.

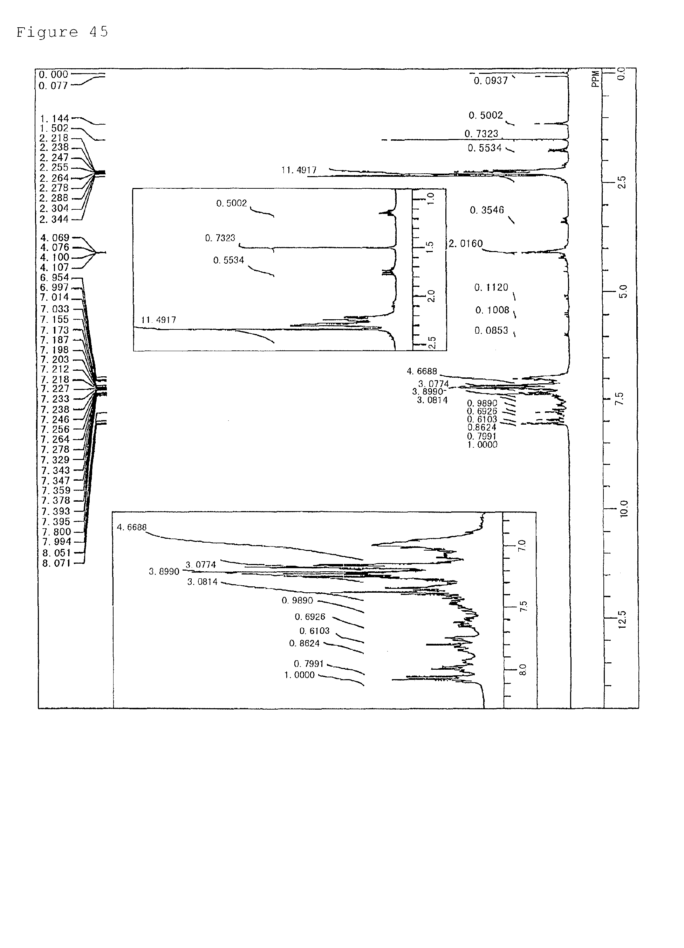

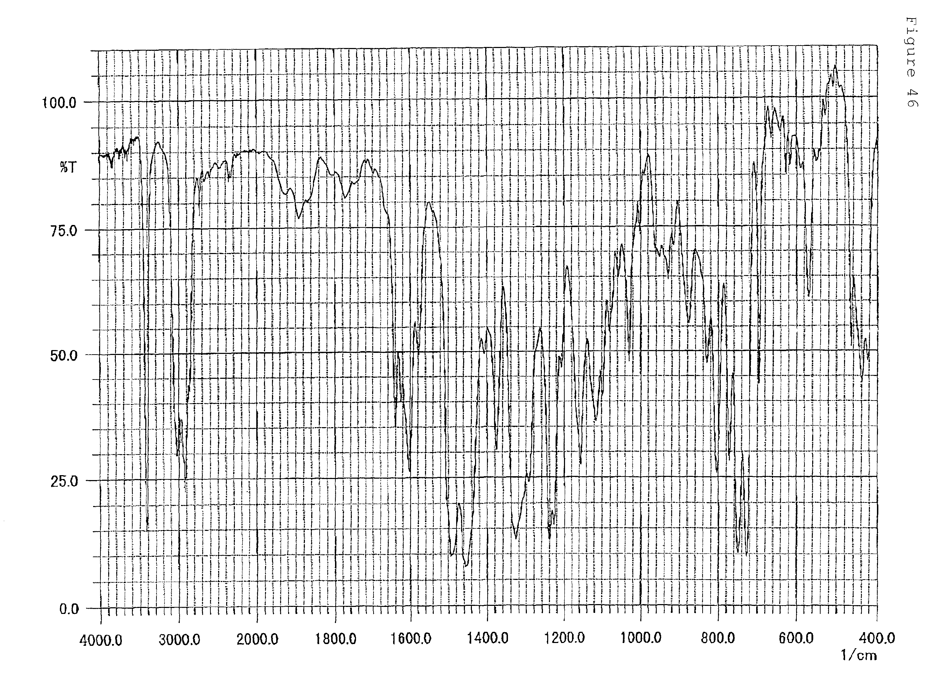

An NMR spectrum chart and an IR spectrum chart of the obtained paste are shown in Figures 45 and 46 respectively. From these data the obtained was identified as a vinyl compound having the structure represented by formula (55).

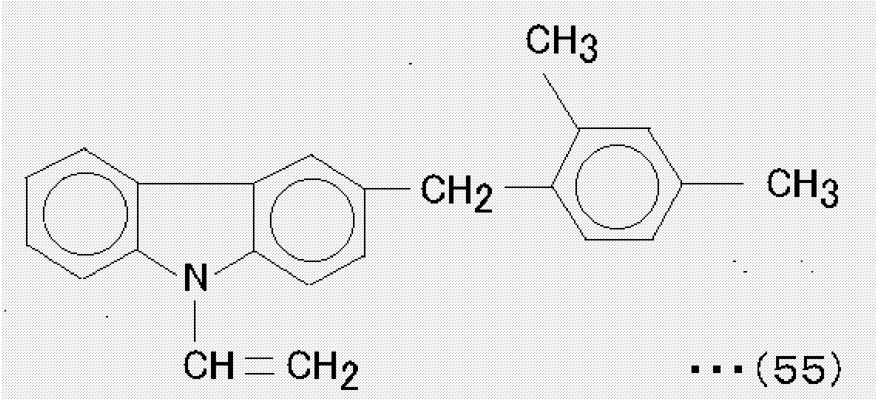

### <Polymerization>

5 g of the vinyl compound represented by the formula (55), 13 mg of AIBN and 50 ml of chlorobenzene were placed in a polymerizing tube. The solution in the tube was heated in a silicone oil bath to 80°C and allowed to react for 20 hours. After the completion of the reaction, the contents in the tube were cooled naturally, and concentrated. The concentrate was dripped in 200 ml of methanol and the resultant was filtered. Precipitates were obtained. The precipitates were dissolved in chloroform, active carbon was added thereto and the resulting mixture was heated and filtered. The filtrate thus obtained was dripped in 200 ml of methanol, and the resultant was filtered. The precipitates were collected and dried. 0.2 g of a white solid matter was obtained.

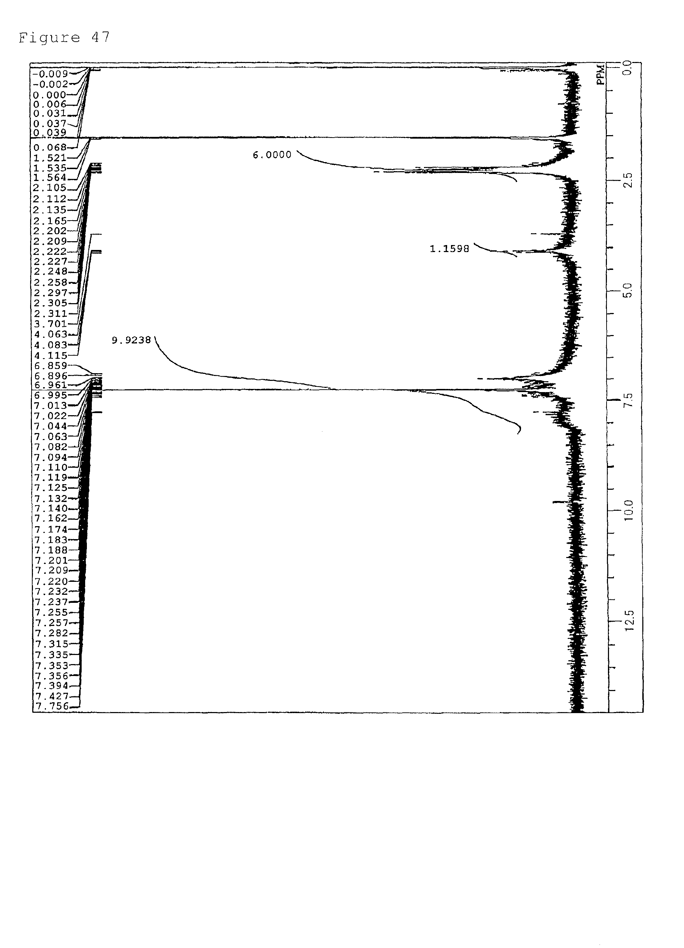

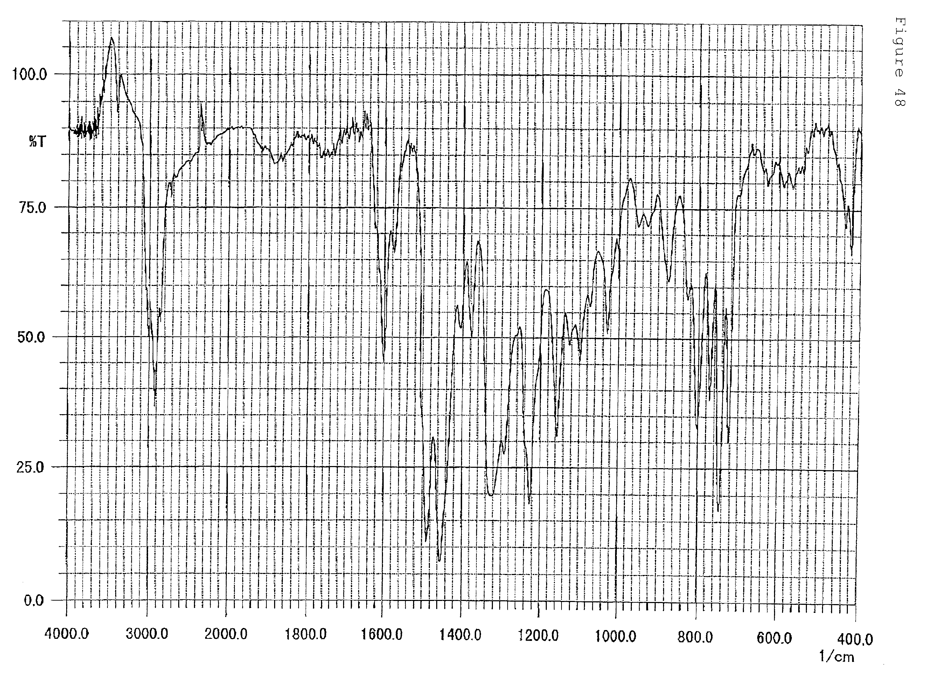

An NMR spectrum chart and an IR spectrum chart of the solid matter are shown in Figures 47 and 48 respectively. The obtained was identified as a blue light-emitting polymer having the repeating unit represented by formula (56).

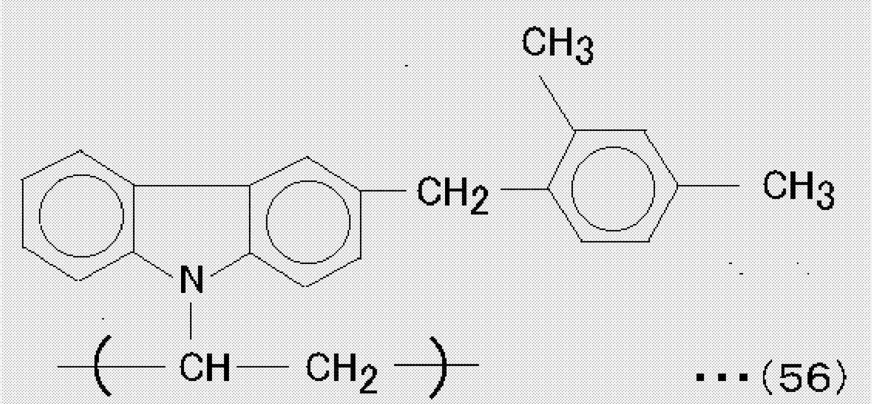

### (Example 8)

### - Preparation of Luminescent Element -

### <Luminescent Properties>

In a 5 ml graduated flask were placed 0.5 mg of the compound represented by formula (57) and 100 mg of powder of the polymer having the repeating unit of formula (15), which was prepared in Example 1, and dichloroethane was added to the placed so that the volume of the solution including the blue light- emitting polymer was 5 ml.

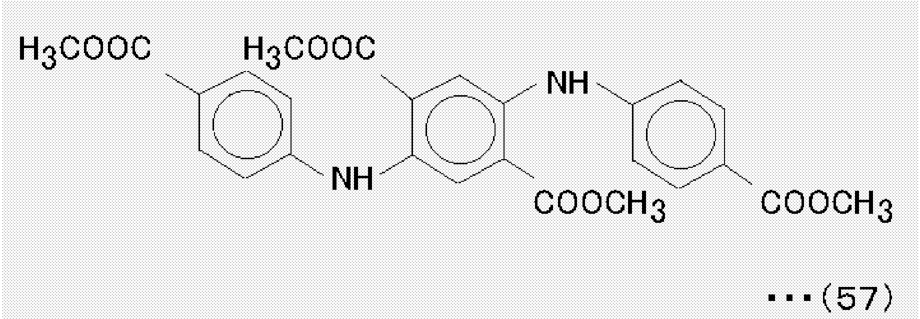

The solution was irradiated with an ultrasonic wave for 20 minutes using an ultrasonic cleaner (model: US-2, produced by NSD Co., Ltd.) and made sufficiently uniform. An ITO substrate (dimensions: 50 mm x 50 mm; the thickness of the ITO transparent electrode: 150 µm; produced by Sanyo Vacuum Industries Co., Ltd.) was ultrasonically cleaned in acetone for 10 minutes, then in 2-propanol for 10 minutes, and dried with nitrogen gas. In addition, the substrate was cleaned through the irradiation of UV rays for 5 minutes with a photo face processor/photo surface processor (produced by SEN Lights Corporation, wave length: 254 nm). The ITO substrate was spin-coated with the prepared solution including the blue light-emitting polymer by dripping the solution onto the substrate using a spin coater (produced by MIKASA Corporation, model: 1 H-D7) at 1,500 rpm for three seconds, so that a film of which thickness, when dried, would be 100 µm. The substrate with the film was dried in a thermostatic chamber at 50°C for 30 minutes. Then an aluminum alloy (Al:Li=99:1 (weight ratio), produced by Kojundo Chemical Laboratory Co., Ltd.) was vapor-deposited on the film at a thickness of about 150 nm under 4 x 10⁻⁶ torr with a vacuum metallizer (produced by DIAVAC Limited, model:
VDS-M2-46), so that an electrode of the aluminum alloy was formed on the film.
The luminescent element emitting blue light that had the structure shown in Figure 1 was prepared in this way.

The luminance and the chromaticity of the prepared element was measured with a Fast BM-7 measuring apparatus produced by TOPCON Corporation, with the voltage being raised gradually. The results were that, when the voltage was 30 V and the current was 12.55 mA, the luminance was 185.50 Cd/m², chromaticity X 0.4290, and chromaticity Y 0.5710.

### (Example 9)

### - Synthesis 8: Synthesis of a Blue Light-emitting Polymer -

### <Alkylation>

In a 300 ml three-necked flask were placed 10.0 g of 9-(2-chloro-1-ethyl)carbazole, which had been produced in the same method as in <Synthesis of Organic Halogen Compound> of Example 1, 4.27 g of zinc and 250 ml of carbon disulfide. The solution in the three-necked flask was heated to 45°C and allowed to react for 39 hours. During the early stage of the reaction, the solution prepared by dissolving 20.2 g of 2,4-dimethylbenzyl chloride in 50 ml of carbon disulfide was dripped in the solution. After the completion of the reaction, the solution was introduced into ice water. To the resulting mixture was added 300 ml of chloroform, and extraction was carried out. Then, moisture was removed by adding sodium sulfate and chloroform was distilled off. A paste was obtained. The paste was dissolved in methanol and reprecipitation was carried out. The precipitated was dried and 7.4 g of a paste was obtained.

The obtained was identified as a compound having the structure represented by formula (54), i.e. 3,6-di(3,4- dimethylbenzyl)-9-(2-chloroethyl)carbazole.

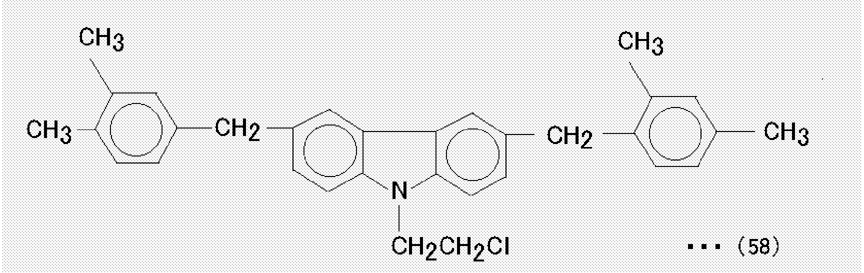

### <Dehydrohalogenation>

In a 300 ml pear-shaped flask were placed 4 g of the compound represented by the formula (58), 2.4 g of potassium hydroxide, 60 ml of ethanol, and 142.8 ml of 1,4-dioxane. The solution in the flask was heated in a silicone oil bath to 110°C and allowed to react for 19 hours. After the completion of the reaction, the resultant solution was introduced into ice water. Precipitates were formed. The precipitates were collected by filtration, dried and dissolved in toluene. The resultant solution was filtered and the filtrate was recrystallized in 2-propanol. 1.27 g of crystals having a melting point of 55°C was obtained.

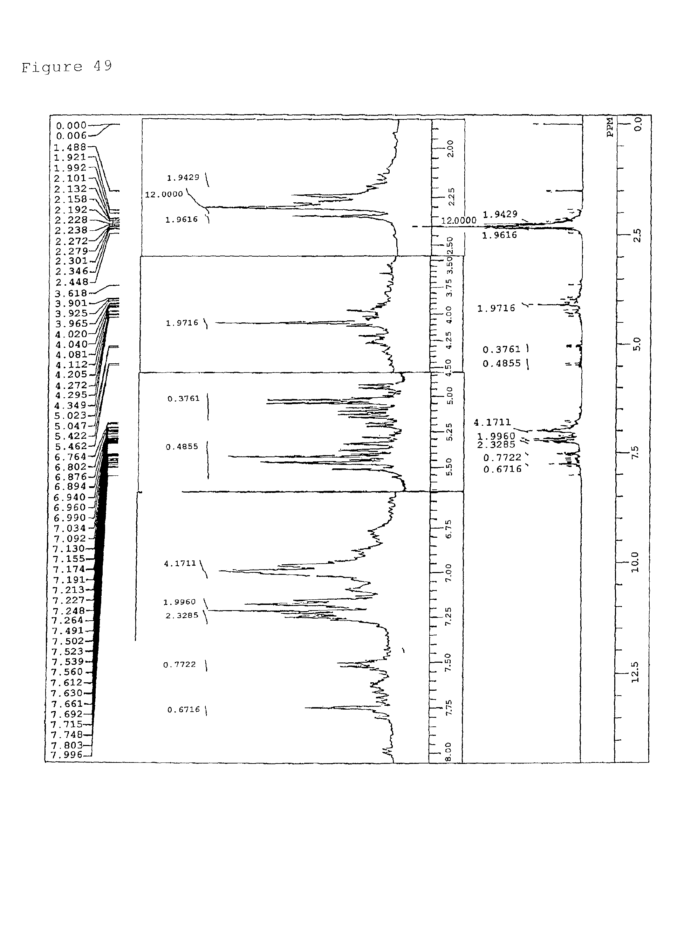

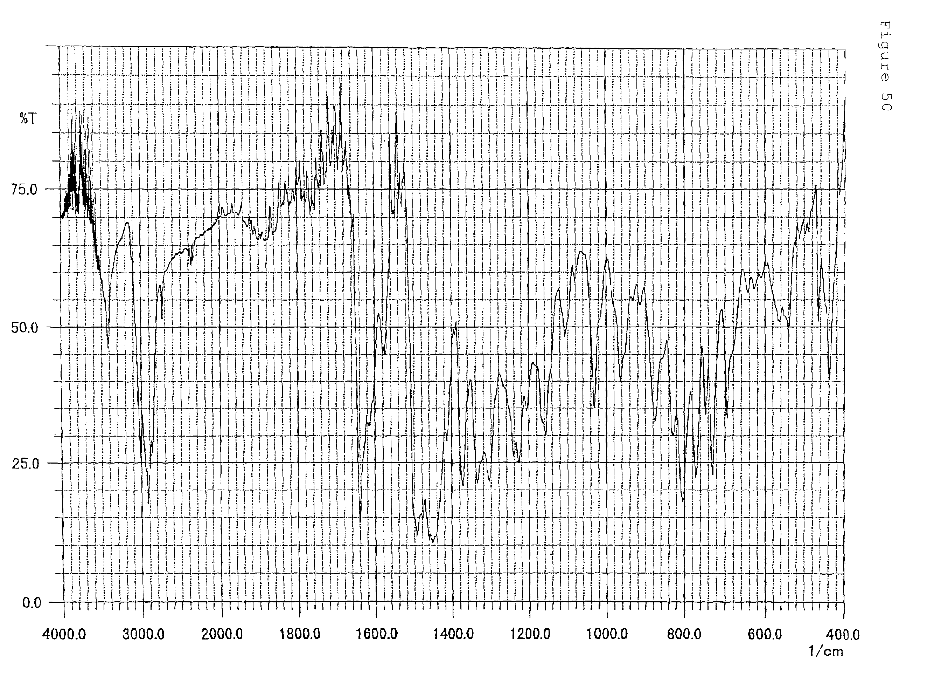

An NMR spectrum chart and an IR spectrum chart of the obtained crystals are shown in Figures 49 and 50 respectively. From these data the obtained was identified as a vinyl compound having the structure represented by formula (59).

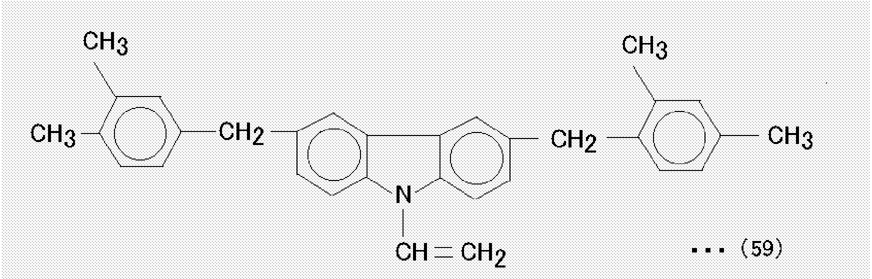

### <Polymerization>

0.988 g of the vinyl compound represented by the formula (59) and 30 ml of chlorobenzene were placed in a polymerizing tube. Then the polymerizing tube was filled with nitrogen gas. To the solution in the polymerizing tube was added 1 ml of the catalyst solution which had been prepared by dissolving 0.0264 g of tungsten hexachloride in 10 ml of chlorobenzene. After the addition the polymerizing tube was shaken for 64 hours. The polymerizing tube was opened and the solvent was let to evaporate from the produced liquid in the tube. As a result a solid matter was obtained. The solid matter was dissolved in a mixture of chloroform and methanol, and reprecipitated. Crystals were obtained.

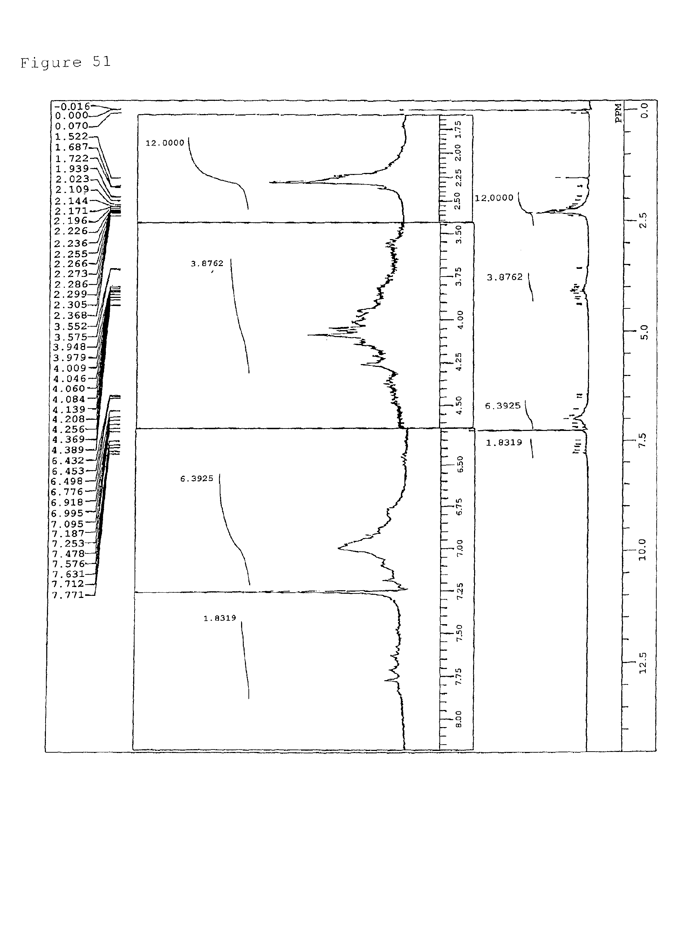

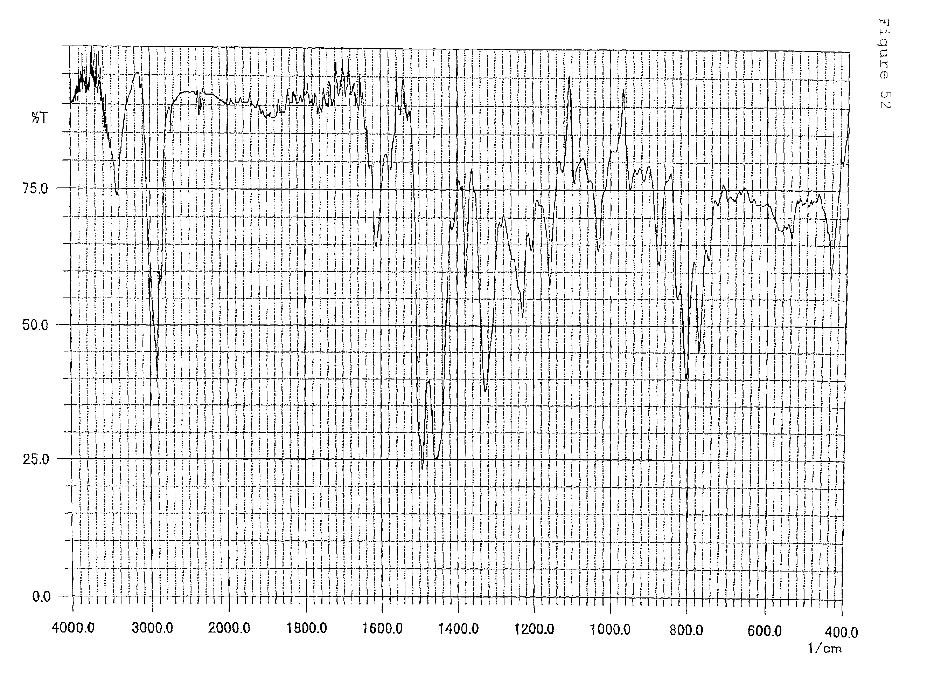

The crystals had a melting range between 85 and 160°C. An NMR spectrum chart and an IR spectrum chart of the crystals are shown in Figures 51 and 52 respectively. The obtained was identified as a blue light-emitting polymer having the repeating unit represented by formula (60).

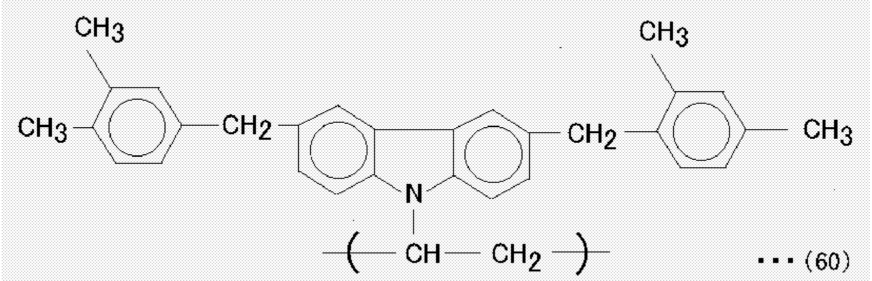

### (Example 10)

### - Luminescent Element -

A polymer solution was prepared by dissolving, in dichloromethan, dinaphthyl oxadiazole and the polymer represented by the formula (60), wherein the weight ratio of dinaphthyl oxadiazole to the polymer was 3:7, so that the concentration of the mixture of dinaphthyl oxadiazole and the polymerand dichloroethane in the solution was 15% by weight. A little amount of solvent was removed from the polymer solution and the solution was made to a concentrated solution.

An ITO substrate, a product by Sanyo Shinku Industries, Co., Ltd., of which dimensions were 50 x 50 mm, was ultrasonically cleaned in acetone for 10 minutes and then in 2-propanol for 10 minutes. The substrate was blow-dried with nitrogen gas. Then, the substrate was cleaned by ultraviolet-light irradiation for 30 seconds at a wavelength of 172 nm with a UV irradiator produced by M.D. Excimer Inc. An electron- injecting layer made of P dot, which is the name of a product produced by Bayer, having a thickness of 500□ was formed on the cleaned substrate with a model 1 H-D7 spin coater, a product by Mikasa Co., Ltd. The polymer solution was dripped onto the surface of the electron-injecting layer and a polymer layer as hole-transporting layer was formed on the surface of the substrate by spin-coating at 1,500 rpm for 3 seconds. The substrate with the layers was dried for 30 minutes in a constant temperature bath of 50°C. An aluminum alloy, a product by Kojundo Chemical Laboratory, Co., Ltd., in which the weight ratio of Al to Li was 99:1, was vapor-deposited on the polymer layer under 4 x 10⁻⁶ torr with a vacuum metallizer (Model VDS-M2-46, produced by DIAVAC Limited), so that an electrode of the aluminum alloy was formed on the polymer layer. The thickness of the electrode was 150 nm. Thus an EL element, which may be called blue light-emitting element hereinafter, was prepared.

The luminance and the chromaticity of the EL element were measured with a Fast BM-7 measuring apparatus produced by TOPCON Corporation, with the voltage being raised gradually. The results were that, when the voltage was in the range between 15 V and 30 V, the luminance was 200 Cd/m², chromaticity X 0.1877, and chromaticity Y 0.1520. The values on the X-Y coordinates confirmed that the light-emitting element employing the polymer emitted blue light.

### (Example 11)

### - Luminescent Element -

A blue light-emitting element was prepared in the same method as in Example 10, except that the blue light-emitting polymer prepared in Example 7, which has the repeating unit represented by the formula (56), was employed in place of the polymer having the repeating unit represented by the formula (60).

The luminance and the chromaticity of the blue light- emitting element were measured in the same method as in Example 10. The luminance was 200 Cd/m², chromaticity X 0.2896, and chromaticity Y 0.3333. These data confirmed that the light-emitting element prepared in this example emitted blue light.

### (Example 12)

### - Synthesis 9: Synthesis of a Blue Light-emitting Polymer -

### <Alkylation>

In a 300 ml three-necked flask were placed 20.26 g of 9-(2-chloro-1-ethyl)carbazole, which had been produced in the same method as in <Synthesis of Organic Halogen Compound> of Example 1 and represented by the formula (8), 5.77 g of zinc and 100 ml of carbon disulfide. The solution in the three-necked flask was heated to 45°C in a silicone oil bath and allowed to react for 2 hours, while the solution prepared by dissolving 20 g of 9-chloromethylanthracene in 400 ml of carbon disulfide was dripped in the solution. After the completion of the reaction, the resultant solution cooled with ice. Then, the cooled solution was introduced into ice water, to which sodium hydroxide was added, so that the mixture turned alkaline. Then, 200 ml of chloroform was added and extraction was carried out. Moisture was removed by adding sodium sulfate and chloroform was distilled off. The remaining was washed with hot ethanol, and the first component that was not dissolved in ethanol was separated from the ethanol solution including the second component that was dissolved in ethanol. The first component was recrystallized in benzene/cyclohexane and the precipitated crystals, which will be called the first crystals hereinafter, were collected by filtration. The melting point of the first crystals was 248°C.

The ethanol solution including the second component was concentrated and a solid matter was formed. The solid matter was recrystallized in benzene/cyclohexane, and the precipitated crystals, which will be called the second crystals hereinafter, were collected by filtration. The melting point of the second crystals was 169°C.

From an NMR spectrum chart and an IR chart of the first crystals, they were identified as a first organic halogen compound having the structure represented by formula (61). Also, from an NMR spectrum chart and an IR chart of the second crystals, they were identified as a second organic halogen compound having the structure represented by formula (62).

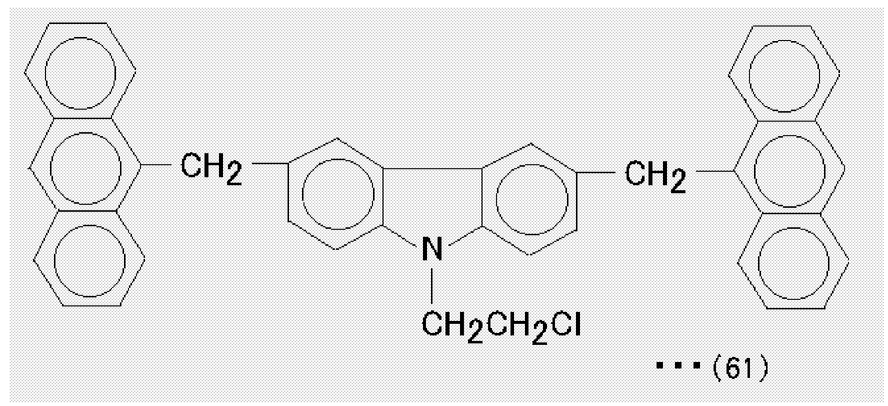

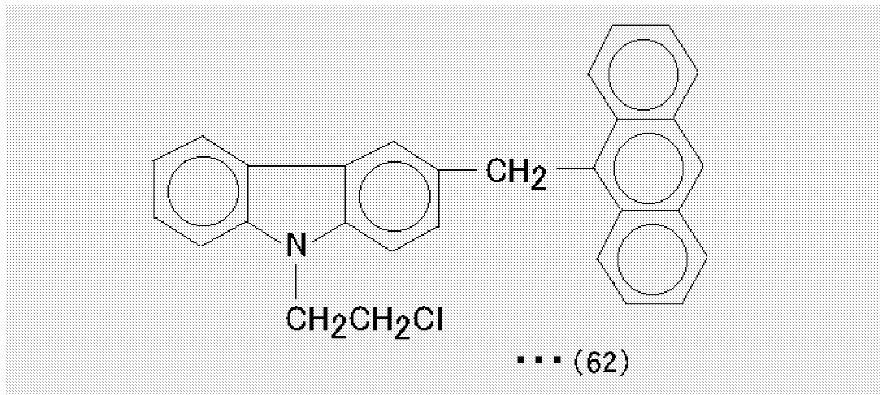

### <Dehydrohalogenation>

In a one-liter pear-shaped flask were placed 5.44 g of the first organic halogen compound represented by the formula (61), 4.35 g of potassium hydroxide, 120 ml of ethanol and 240 ml of 1,4-dioxane. The solution in the flask was boiled in a silicone oil bath and allowed to react for one night and day. After the completion of the reaction, the resultant solution was concentrated, and the obtained concentrate was introduced into ice water. The concentrate in ice water was extracted with 200 ml of chloroform. The extract was washed with water and dried. A paste was obtained. A column, which had been filled with silica gel, was charged with the paste, and the paste was purified with toluene as a developer. Then, the purified was recrystallized in benzene/cyclohexane. 2.30 g of crystals having a melting point of 241 °C were obtained.

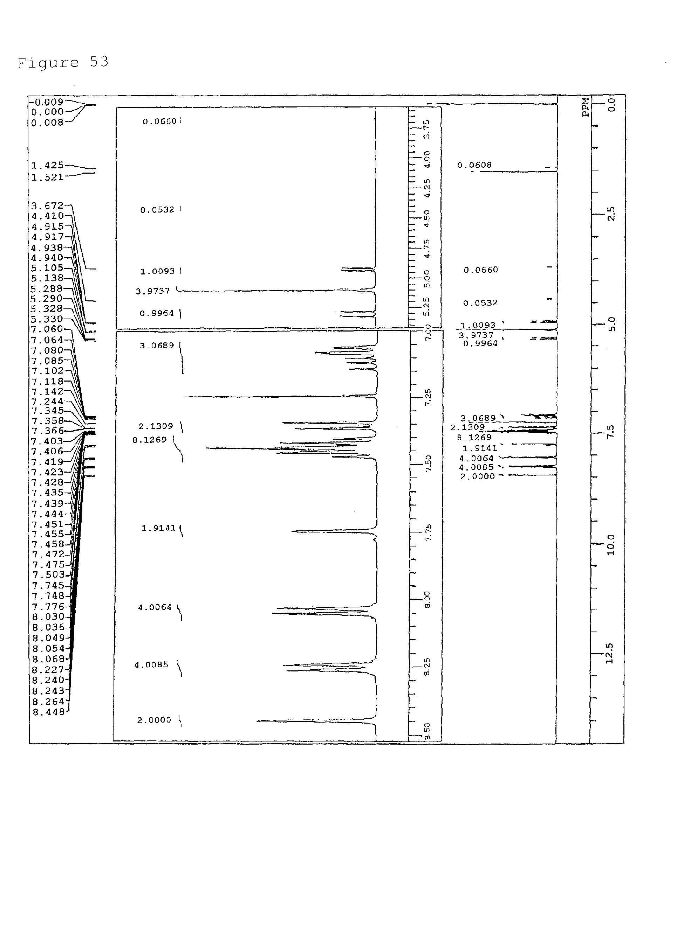

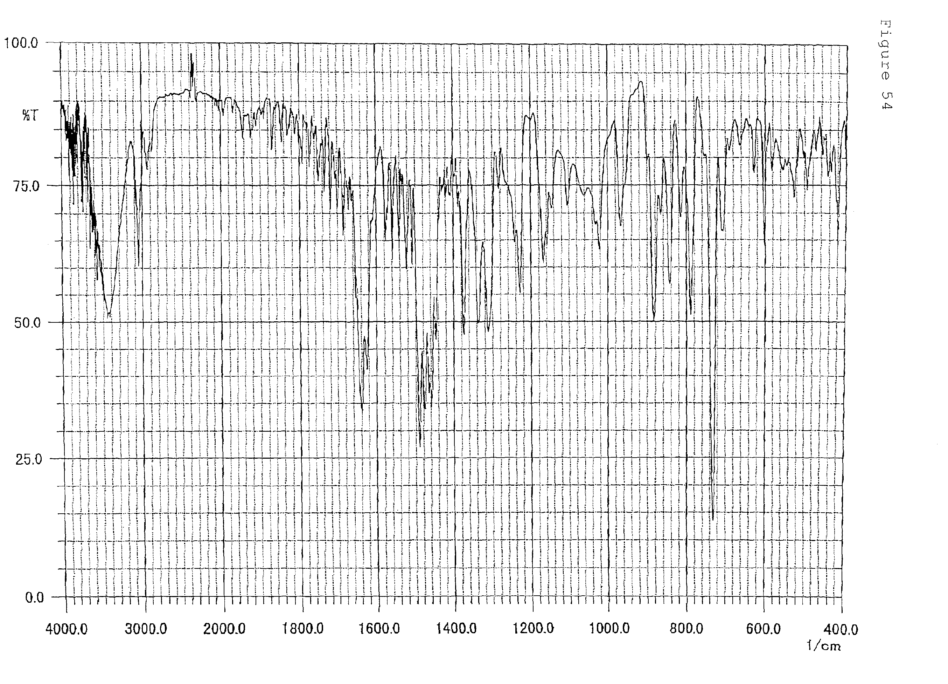

An NMR spectrum chart and an IR spectrum chart of the obtained crystals are shown in Figures 53 and 54 respectively. From these data the obtained was identified as a vinyl compound having the structure represented by formula (63), which will be called the first vinyl compound herein after.

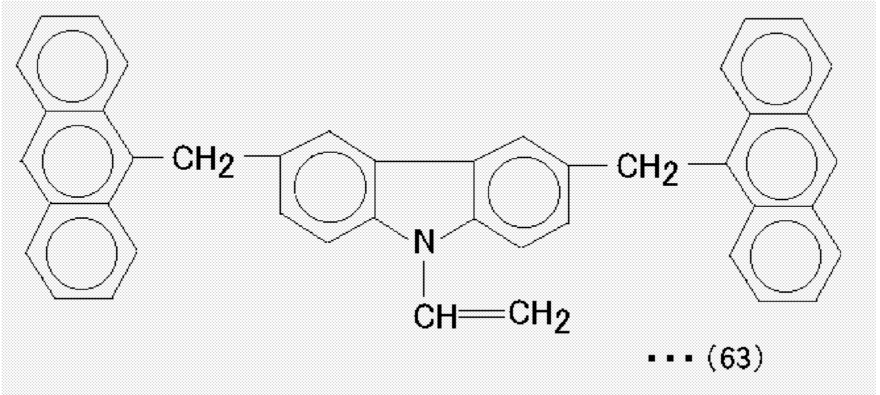

The preceding steps were repeated, except that 1.47 g of the second organic halogen compound represented by the formula (62), 1.18 g of potassium hydroxide, 50 ml of ethanol and 50 ml of 1,4-dioxane were used in place of 5.44 g of the first organic halogen compound represented by the formula (61), 4.35 g of potassium hydroxide, 120 ml of ethanol and 240 ml of 1,4-dioxane.

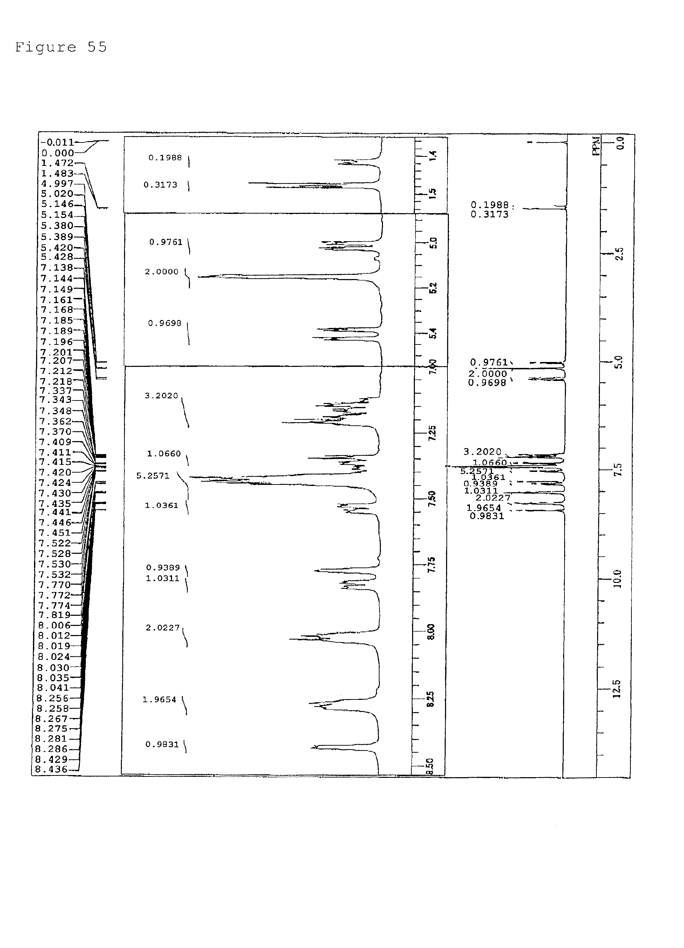

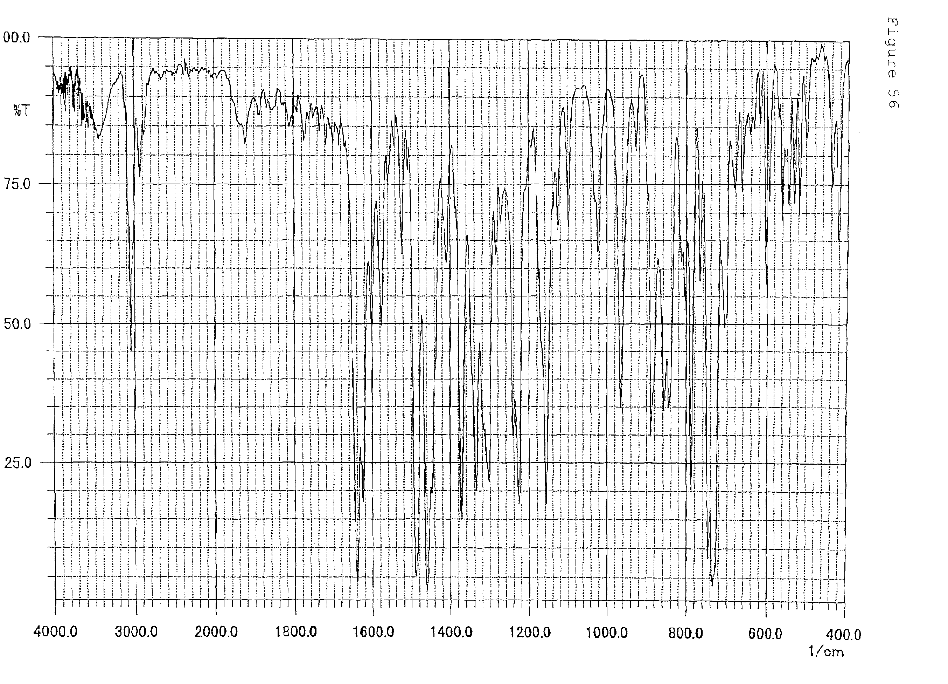

As a result, a vinyl compound having melting range between 167°C and 169°C and represented by formula (64), which will be called the second vinyl compound hereinafter, was obtained. An NMR spectrum chart and an IR spectrum chart of the second vinyl compound are shown in Figures 55 and 56 respectively.

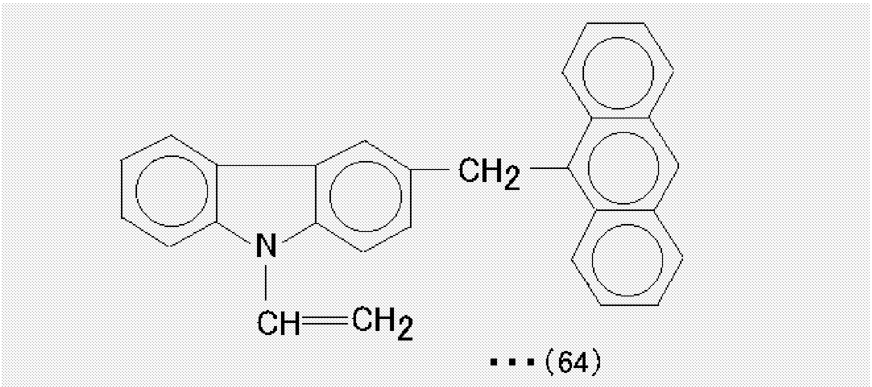

### <Polymerization>

1.50 g of the first vinyl compound represented by the formula (63) and 100 mg of chlorobenzene were placed in a polymerizing tube. Then the polymerizing tube was filled with nitrogen gas. To the solution in the polymerizing tube was added 1 ml of the catalyst solution which had been prepared by dissolving 0.0029 g of tungsten hexachloride in 10 ml of chlorobenzene. After the addition the polymerizing tube was shaken for 64 hours.

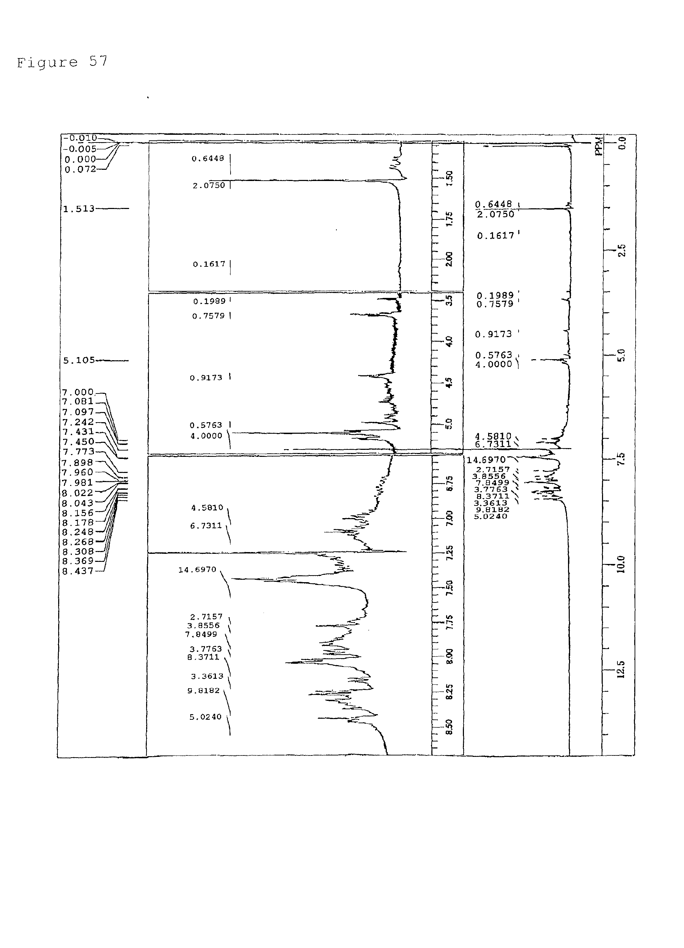

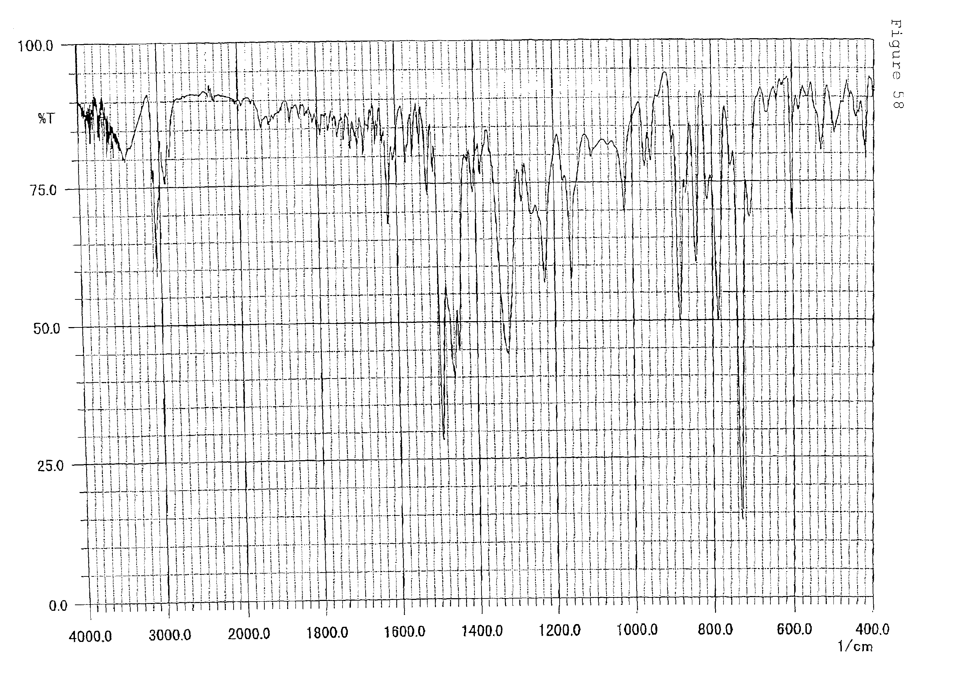

The steps that followed the 64-hour shaking were the same as those in Example 9. A solid matter was obtained. An NMR spectrum chart and an IR spectrum chart of the solid matter are shown in Figures 57 and 58 respectively. The obtained was identified as a first blue light-emitting polymer having the repeating unit represented by formula (65).

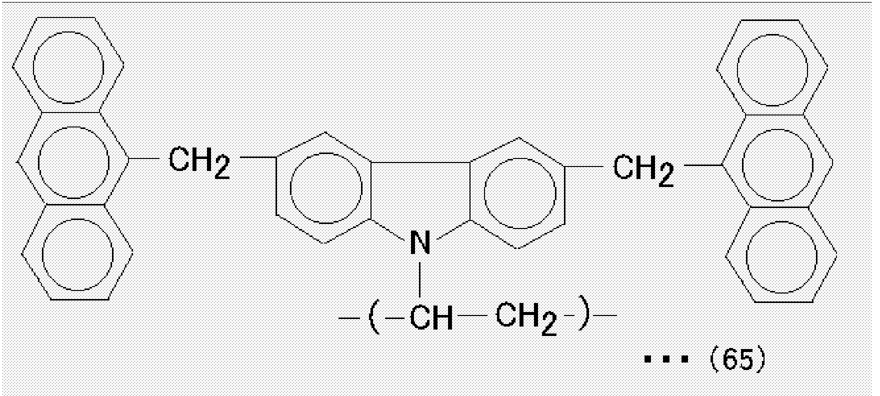

1.30 g of the second vinyl compound represented by the formula (64) and 60 ml of chlorobenzene were placed in a polymerizing tube. Then the polymerizing tube was filled with nitrogen gas. To the solution in the polymerizing tube was added 1 ml of the catalyst solution which had been prepared by dissolving 0.0040 g of tungsten hexachloride in 10 ml of chlorobenzene. After the addition the polymerizing tube was shaken for 64 hours.

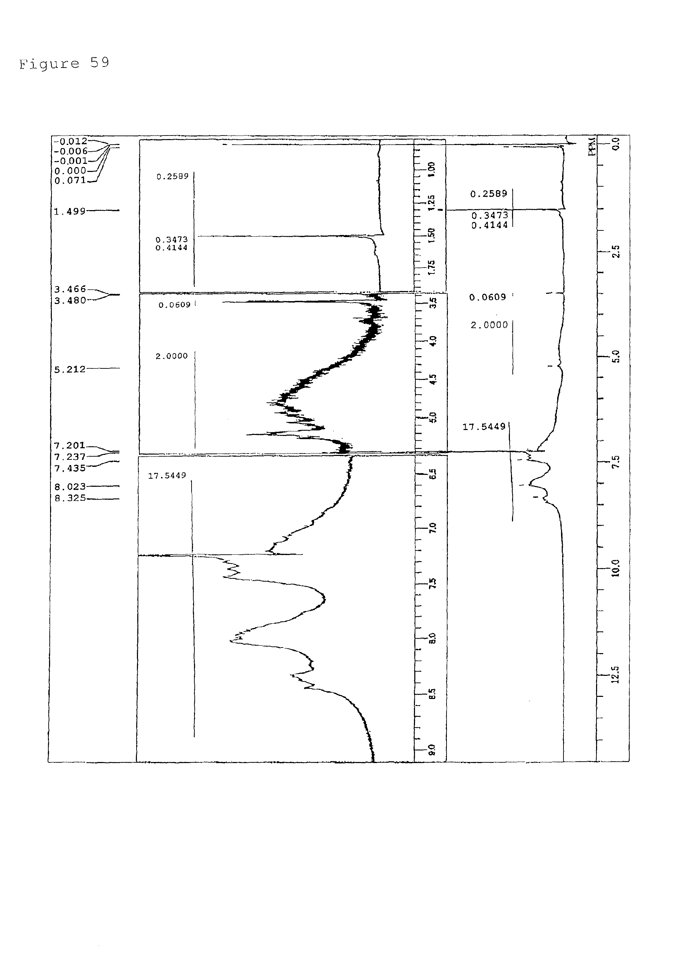

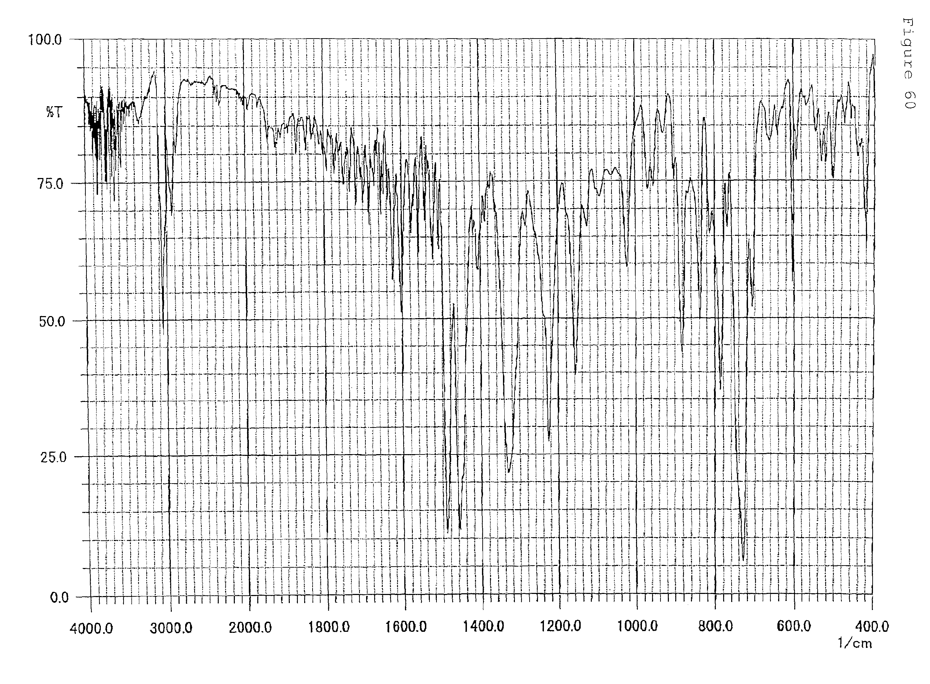

The steps that followed the 64-hour shaking were the same as those in Example 9. Another solid matter was obtained. An NMR spectrum chart and an IR spectrum chart of this solid matter are shown in Figures 59 and 60 respectively. The obtained was identified as a second blue light-emitting polymer having the repeating unit represented by formula (66).

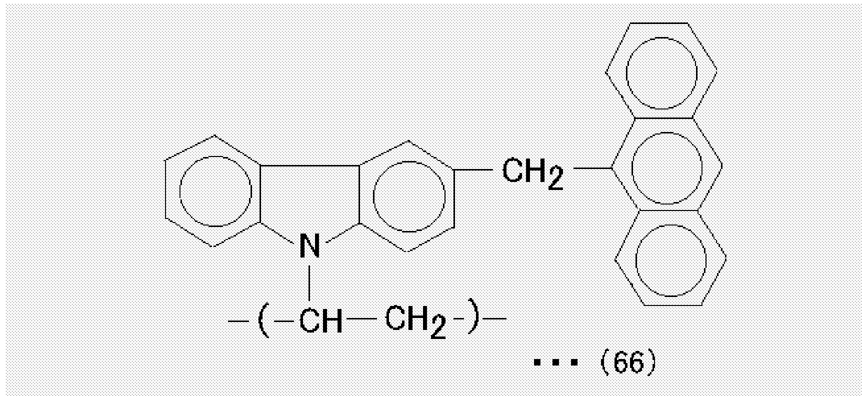

## Claims

1. A blue light-emitting compound having a chemical structure represented by formula (1): 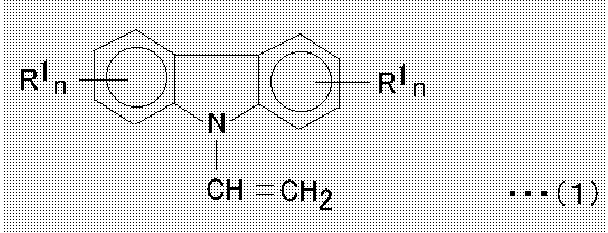 wherein R¹ is a hydrogen atom, an alkyl group having 1 to 10 carbon atoms, or a substituent represented by formula (2), two R¹s may be the same or different from each other, and n denotes an integer of 1 to 4, wherein the formula (2) is: 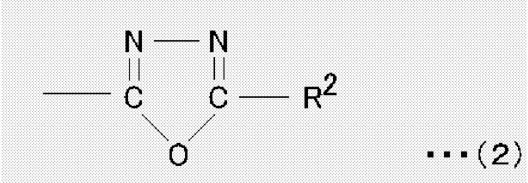 wherein R² is an aryl group represented by formulas (2-1) to (2-8): 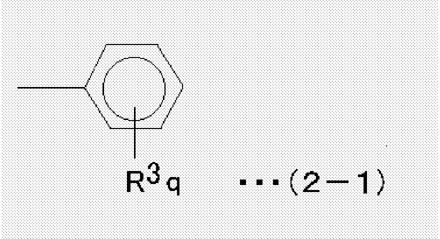 wherein R³ is a hydrogen atom or an alkyl group having 1 to 10 carbon atoms, and q denotes an integer of 1 to 5; 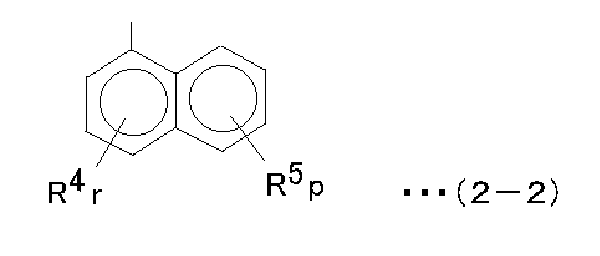 wherein each of R⁴ and R⁵ is a hydrogen atom, an alkyl group having 1 to 10 carbon atoms or an alkoxyl group having an alkyl group that has 1 to 5 carbon atoms, R⁴ and R⁵ may be the same or different from each other, p denotes an integer of 1 to 4, and r that of 1 to 3; 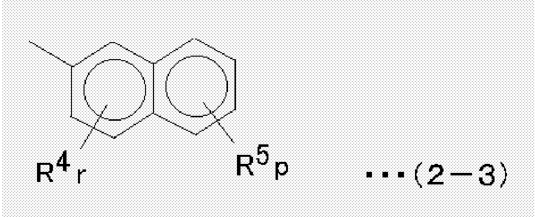 wherein R⁴, R⁵, p and r mean the same as those defined above, and R⁴ and R⁵ may be the same or different from each other; 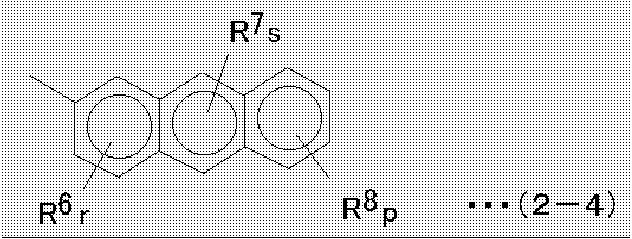 wherein each of R⁶, R⁷ and R⁸ is a hydrogen atom or an alkyl group having 1 to 5 carbon atoms, R⁶, R⁷ and R⁸ may be the same or different from each other, p and r denote the same as those defined above, and s denotes an integer of 1 or 2; 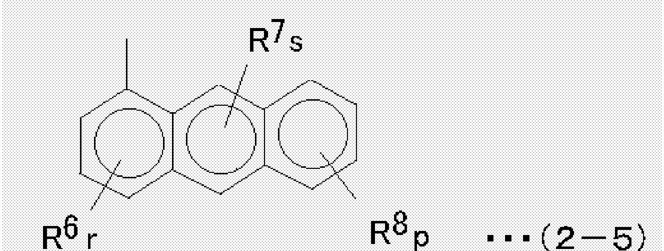 wherein R⁶, R⁷, R⁸, p, r and s mean the same as those defined above, and R⁶, R⁷ and R⁸ may be the same or different from each other; 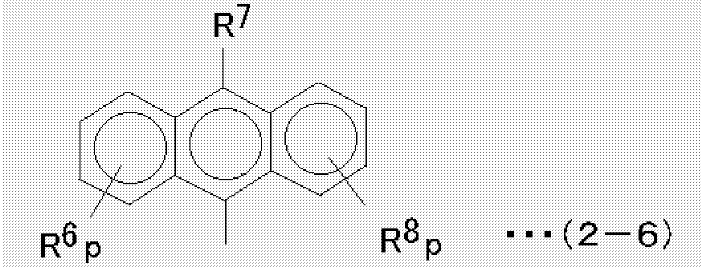 wherein R⁶, R⁷, R⁸, p, r and s mean the same as those defined above, and R⁶, R⁷ and R⁸ may be the same or different from each other; 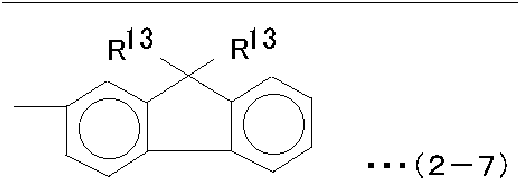 wherein R¹³ is a hydrogen atom or an alkyl group having 1 to 10 carbon atoms; and 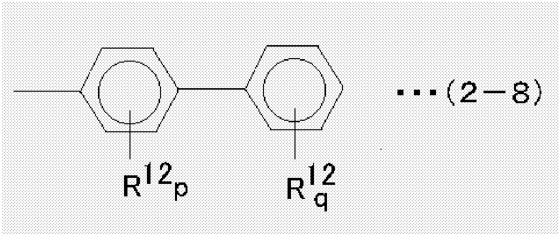 wherein R¹² is a hydrogen atom or an alkyl group having 1 to 10 carbon atoms, two R¹²S may be the same or different from each other, and p and q mean the same as those defined above.

2. A blue light-emitting compound having a structure represented by formula (1'): 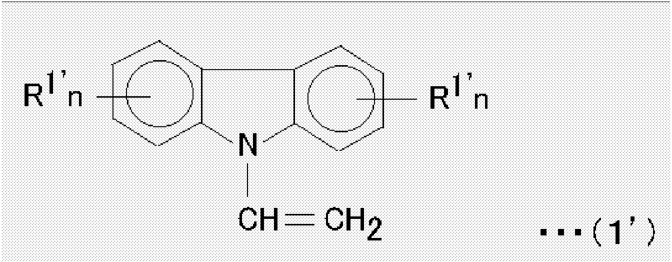 wherein R^{1'} is a hydrogen atom, an alkyl group having 1 to 10 carbon atoms, or a substituent represented by formula (2'), two R^{1'}s may be the same or different from each other, and n denotes an integer of 1 to 4, wherein the formula (2') is: 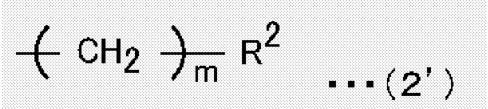 wherein R² is an aryl group represented by the formulas (2-1) to (2-8) according to claim 1, or a substituent represented by formula (2'-1), and m denotes an integer of 0 to 3; wherein the substituent represented by the formula (2'-1) is: 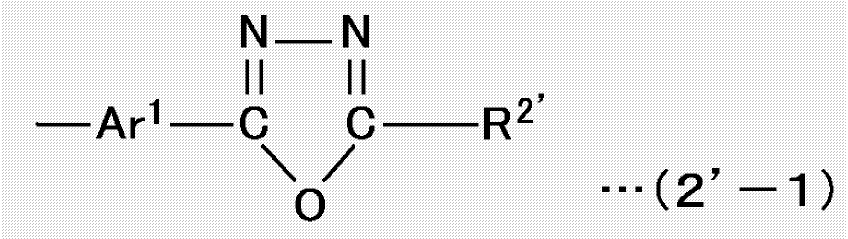 wherein Ar¹ is a phenylene group, a naphthylene group, or an anthrylene group, and R²' means an aryl group represented by the formulas (2-1) to (2-8) according to claim 1.

3. A process of producing the blue light-emitting compound comprising:
reacting a halogen compound represented by formula (4) with a hydrazide to produce an intermediate represented by formula (5), the formula (4) being: 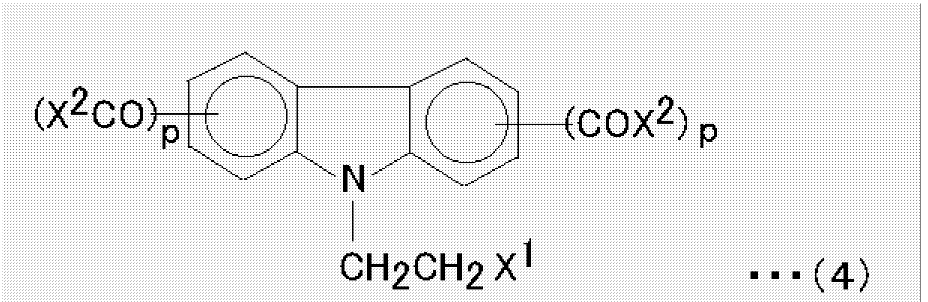 wherein X¹ and two X²s are a halogen atom, X¹ and two X²s may be the same or different from each other, p denotes an integer of 1 to 4, and q denotes an integer of 1 to 5, the formula (5) being: 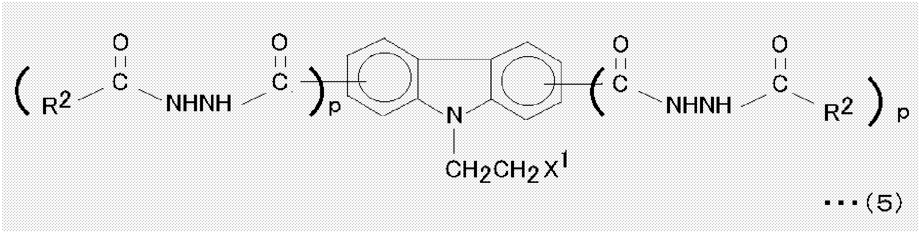 wherein R² means an aryl group represented by the formulas (2-1) to (2-8) according to claim 1, X¹ and p are the same as those defined above, and two R²s may be the same or different from each other; and
dehydrating the intermediate; and
dehydrohalogenating the dehydrated intermediate.

4. A process of producing the blue light-emitting compound that has the structure represented by the formula (1') comprising:
carrying out a Friedel-Crafts reaction with a halogen compound represented by formula (4'): 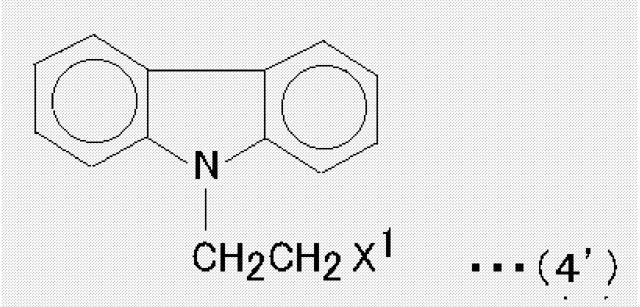
wherein X¹ is a halogen atom; to produce an organic halogen compound represented by formula (5'): 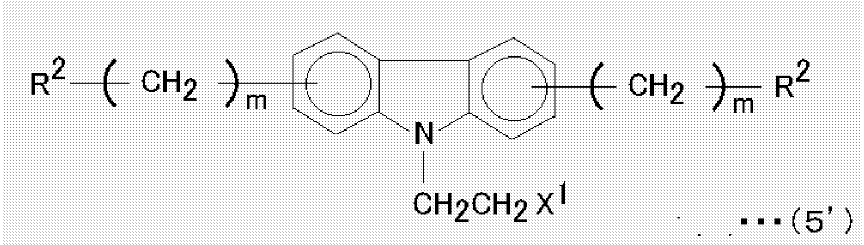 wherein R² means an aryl group represented by the formulas (2-1) to (2-8) according to claim 1, X¹ is a halogen atom and m denotes an integer of 1 to 3; and
dehydrohalogenating the organic halogen compound.

5. A blue light-emitting polymer having a repeating unit represented by formula (1''): 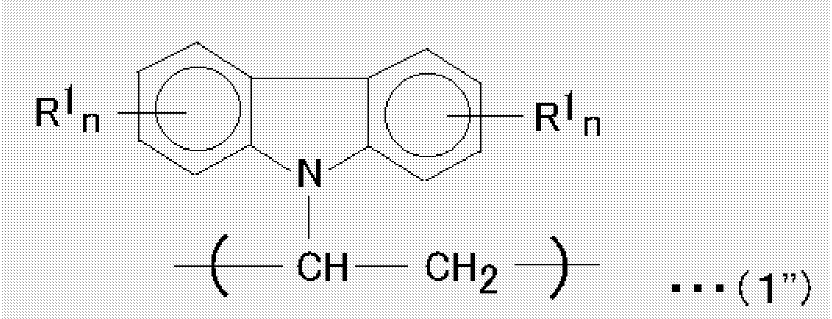 wherein R¹ and n mean the same as those as defined in claim 1.

6. A blue light-emitting polymer having a repeating unit represented by formula (1'''): 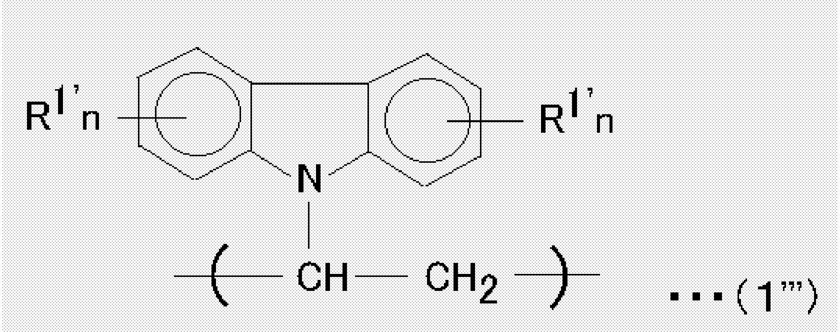 wherein R^{1'} and n mean the same as those defined in claim 2.

7. A luminescent element which includes a light-emitting layer comprising a blue light-emitting polymer as defined in claim 5 or claim 6.
